(19) Europäisches Patentamt
European Patent Office
Office européen des brevets

(11) Publication number: **0 073 143**
**B1**

(12) **EUROPEAN PATENT SPECIFICATION**

(45) Date of publication of patent specification: **21.11.85**

(51) Int. Cl.$^4$: **C 07 K 5/06,** C 07 D 207/16, A 61 K 31/02

(21) Application number: **82304377.3**

(22) Date of filing: **19.08.82**

(54) **Novel complex amido and imido derivatives of carboxyalkyl peptides and thioethers and ethers of peptides.**

(30) Priority: **21.08.81 US 295137**

(43) Date of publication of application:
**02.03.83 Bulletin 83/09**

(45) Publication of the grant of the patent:
**21.11.85 Bulletin 85/47**

(84) Designated Contracting States:
**AT BE CH DE FR GB IT LI LU NL SE**

(56) Reference cited:
**EP-A-0 012 401**

(73) Proprietor: **Ryan, James Walter**
**3420 Poinciana Avenue**
**Miami Florida 33133 (US)**

(72) Inventor: **Ryan, James Walter**
**3420 Poinciana Avenue**
**Miami Florida 33133 (US)**
Inventor: **Chung, Alfred**
**8781 Southwest 87th. Street**
**Miami Florida 33183 (US)**

(74) Representative: **De Minvielle-Devaux, Ian Benedict Peter et al**
**CARPMAELS & RANSFORD**
**43 Bloomsbury Square**
**London WC1A 2RA (GB)**

**EP 0 073 143 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

*Background of the Invention*

Angiotensin converting enzyme (peptidyldipeptide hydrolase, hereinafter referred to as ACE) occupies a central role in the physiology of hypertension. The enzyme is capable of converting the decapeptide angiotensin I, having the sequence.

AspArgValTyrIleHisProPheHisLeu

to an octapeptide, angiotensin II, by removal of the carboxy-terminal HisLeu. The symbols for the foregoing chemical moieties and others used throughout this application are explained in the following table:

Arg = arginine
Asp = aspartic acid
Boc = t-butyloxycarbonyl
Cbo = carbobenzyloxy
<Glu = pyro-L-glutamic acid
Gly = glycine
Hip = Hippuric acid (Benzoyl-glycine)
His = histidine
Ile = isoleucine
Leu = leucine
Phe = phenylalanine
Pro = proline
ΔPro = 3,4-dehydroproline
Ser = serine
Tos = tosyl
Trp = tryptophan
Tyr = tyrosine
Val = valine
Pht = phthaloyl
ACE = angiotensin converting enzyme
Hepes = N-2-hydroxyethylpiperazine-N′-2-ethanesulfonic acid

In each instance the symbol for any amino acid is also used herein at times to refer to a mono-or-divalent radical of such acid and those of ordinary skill in the art will readily understand the context of each specific use.

Angiotensin I is formed by the action of the enzyme renin, an endopeptidase found in kidney, other tissues and plasma, on a serum α-2 globulin.

Blood pressure is affected by certain peptides found in the blood. One of these, angiotensin II, is a powerful pressor (blood pressure elevating) agent. Another, bradykinin, a nonapeptide with the sequence ArgProProGlyPheSerProPheArg is a powerful depressor (blood pressure lowering) agent. In addition to a direct pressor effect, angiotensin II stimulates release of aldosterone which tend to elevate blood pressure by causing retention of extracellular salt and fluids. Angiotensin II is found in measurable amount in the blood of normal humans. However, it is found at elevated concentrations in the blood of patients with renal hypertension.

The level of ACE activity is ordinarily in excess, in both normal and hypertensive humans, of the amount needed to maintain observed levels of angiotension II. However, it has been found that significant blood pressure lowering is achieved in hypertensive patients by treatment with ACE inhibitors [Gavras, I. et al., *New Eng. J. Med. 291*, 817 (1974)].

ACE is a peptidyldipeptide hydrolase. It catalyzes the hydrolysis of the penultimate peptide bond at the C-terminal end of a variety of acylated tripeptides and larger polypeptides having an unblocked α-carboxyl group. The action of ACE results in hydrolytic cleavage of the penultimate peptide bond from the carboxyl-terminal end yielding as reaction products a dipeptide and a remnant.

The reactivity of the enzyme varies markedly depending on the substrate. At least one type of peptide bond, having the nitrogen supplied by proline, is not hydrolyzed at all. The apparent Michaelis constant (Km) varies from substrate to substrate over several orders of magnitude. For general discussion of the kinetic parameters of enzyme catalyzed reactions, see Lehninger, A., *Biochemistry,* 2nd. Ed., Worth Publishers, Inc., New York, 1975, pp. 189—195. Many peptides which are called inhibitors of the enzymatic conversion of angiotensin I to angiotensin II are in fact substrates having a lower Km than angiotensin I. Such peptides are more properly termed competitive substrates. Examples of competitive substrates include bradykinin, and the peptride $BPP_{5a}$ (also called SQ20475) from snake venom, whose sequence is <GluLysTrpAlaPro.

Numerous synthetic peptide derivatives have been shown to be ACE inhibitors by Ondetti, et al. in U.S. patent 3,832,337 issued August 27, 1974.

The role of ACE in the pathogenesis of hypertension has prompted a search for inhibitors of the enzyme that could act as antihypertensive drugs. See for example U.S. patents 3,891,616, 3.947,575, 4,052,511 and 4,053,651. A highly effective inhibitor, with high biological activity when orally administered, is D-3-mercapto-2-methylpropanoyl-L-proline, designated SQ14225, or "captopril" disclosed in U.S. patent 4,046,889 to Ondetti et al., issued September 6, 1977, and in scientific articles by Cushman, D.W. et al., *Biochemistry 16*, 5484 (1977), and by Ondetti, M, et al., *Science 196*, 441 (1977). The inhibitor SQ14225 reportedly has an $I_{50}$ value of $2.3 \times 10^{-8}$M. The $I_{50}$ value reported by Cushman, et al., *supra* is the concentration of inhibitor required to produce 50% inhibition of the enzyme under a standard assay system containing substrate at a level substantially above $K_m$. It will be understood that $I_{50}$ values are directly comparable when all potential factors affecting the reaction are kept constant. These factors include the source of enzyme, its purity, the substrate used and its concentration, and the composition of the assay buffer. All $I_{50}$ data reported herein have been performed with the same assay system and same enzyme (human urinary ACE) and with the same level of substrate and are therefore internally consistent.

The mode of action of SQ14225 has been based upon a model of the active site of ACE developed by analogy with the better known related enzyme, carboxypeptidase A. The active site was postulated to have a cationic site for binding the carboxyl end group of the substrate and a pocket or cleft capable of binding the side chain of the C-terminal amino acid and providing especially tight binding for the heterocyclic ring of a terminal proline residue. A similar pocket for the penultimate amino acid residue was postulated, and the published data suggested a rather stringent steric requirement, since the D-form of the inhibitor was substantially more potent that its stereoisomer or the 3-methyl and unsubstituted analogs. The sulfhydryl group on the inhibitor, postulated to be bound at the active site near the catalytic center, was believed to play a central role in inactivation of the enzyme by combining with the zinc moiety known to be essential for catalytic activity. Substituents on the sulfhydryl, such as a methyl group, and a S-acetyl derivative, substantially reduced potency of the inhibitor. See Cushman, D.W., et al., *Biochemistry, supra.*

*In vitro* study of the mechanism by which SQ14225 and its analogs act to inhibit ACE has been somewhat hampered by the instability of these molecules under ambient conditions. For example, it has been observed that a fresh aqueous solution of concentration, e.g., 1 mg per ml of SQ14225 at a pH of about 8 becomes substantially less active upon standing for as little as 30 minutes, and that activity continues to decrease as the solution stands for longer periods. It is believed that this loss in activity is mainly the result of dimerization of SQ14225 occurring at the sulfhydryl end groups, whereby a disulfide is formed which is largely inactive as an inhibitor. Since the free sulfhydryl group is highly reactive and may be readily oxidized to polar acidic moietes such as sulfone and sulfoxide groups, it may also be that the observed *in vitro* loss of activity of aqueous solutions of SQ14225 on standing is in the same part a consequence of one or more such oxidation reactions, with formation of a sulfone or sulfoxide which does not function effectively as an inhibitor for ACE.

Such reports of SQ14225 clinical testing as are currently available, some of which refer to the compound under the name "Captopril" or "Capoten", suggest that the product is sufficiently stable in the normal gastric and intestinal environments of most patients to be an effective inhibitor of ACE when administered orally. It is not yet clear, however, whether there may be a group of patients for which SQ14225 is substantially ineffective. Because of the high reactivity of the free sulfhydryl group, SQ14225 could readily form mixed disulfides with serum, cellular proteins, peptides or other free sulfhydryl group-containing substances in the gastric or intestinal environments, in addition to the possibility for dimer formation or oxidative degradation reactions. A mixed disulfide with protein may be antigenic and, indeed, occasional allergic reactions have been clinically observed. See Gavras, et al., *New England J. Med. 298*, 991 (1978). Disulfides and oxidative degradation products of SQ14225, if formed, may at best be expected to be largely ineffective as inhibitors. It may be postulated accordingly that dose response to SQ14225 may vary with conditions of administration and among individual patients. Moreoever, in at least some patients, unwanted side effects may occur and maintenance of an effective concentration of the inhibitor in the body may be difficult to control.

Adverse effects of SQ14225 in man include fevers and rashes. (Gabras et al, *supra*). Hoorntje et al., *The Lancet,* i.e., 1212—1214 (1980) describe the performance of renal biopsies on 13 patients treated with SQ14225. All biopsies showed evidence of immune complex deposition along the glomerular basement membranes, although 9 of 13 patients were asymptomatic at the time of the biopsy. These authors also discussed similarities of their findings with those induced by another drug with a free mercapto group, D-penicillamine.

In an effort to devise better inhibitors of angiotensin converting enzyme that are more stable than captopril and less likely to induce D-penicillamine-like adverse effects, applicants have prepared a series of compounds having side chain structure analogous to an effective substrate for the enzyme, benzoyl-Phe-Ala-Pro and disclosed them in copending U.S. application Ser. No. 187992 filed September 17, 1980. Also relevant are the class of carboxyalkyldipeptides derivatives disclosed in European published application n°0012401 of Patchett et al. published on or about June 25, 1980. The present application defines compounds such as N-[L-1-carboxy-3-(carboanilide)propyl]D,L-Ala-L-Pro, N-[L-1-carboxy-3-(carbo-4-iodoanilide)propyl]-D, L-Ala-L-Pro, and analogs i.e., amides and imides of N-(lower alkylene)Ala-Pro. These two named compounds were found to be unexpectedly effective in inhibiting angiotensin converting enzyme *in vitro*, that is they have a very low $I_{50}$, in the order of $10^{-9}$M. In contrast another closely related

analog of the two named compounds, i.e., N-[L-1-carboxy-2-(carbopyrrolide)ethyl]-D,L-Ala-Pro, was found to have a much higher $I_{50}$, in the order of $10^{-7}$M, a potency of inhibitor likely to be too low for anti-hypertensive effectiveness. It is believed, therefore, that amides and imides of N-(lower alkylene)-Ala-Pro and related compounds have unpredictable effects on angiotensin converting enzyme.

In addition, the removal of iodine from N-[L-1-carboxy-3-(carbo-4-iodoanilide)propyl]-D,L-Ala-L-Pro increases intravenous effectiveness three-fold, an unexpectedly large difference in the *in vivo* effect of the anti-hypertensive compounds of this invention. Hence, amides and imides of N-(lower alkylene)-D,L-Ala-Pro and related compounds are new agents with surprising effectiveness in lowering blood pressure *in vivo*.

Moreover, since the compounds of this invention do not have the free sulfhydryl group of SQ14225, they are most likely to be stable and have durations of action much longer than that of SQ14225. Thus, inhibitors of this invention may be used for treating hypertension with less frequent dosage schedules than required for SQ14225 and may be capable of administration under less rigorously controlled conditions.

Brief Description of the Invention

Novel inhibitors of ACE are disclosed which base the general formula

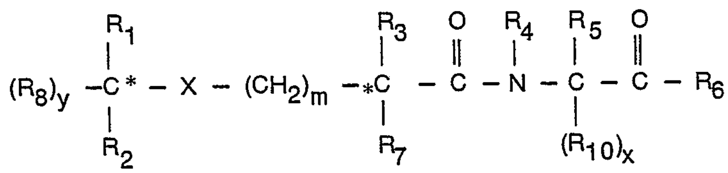

wherein

x and y are 0 or 1, X may be S, O or N—$R_q$ and $R_q$ may be —H or —$CH_3$;

$R_{10}$ is H, $CH_3$, F Cl or Br;

m is 0 or 1,

$R_2$ is COOH, $CH_2COOH$, COSH, $CH_2COSH$, $CH_2SH$, $CH_2$, $CH_2SH$, a physiologically acceptable nontoxic salt of any of them; COOY, $CH_2$ COOY, COSY, $CH_2SY$, or $CH_2CH_2SY$ wherein Y is phenyl, benzyl or a 1—5 carbon alkyl group, or

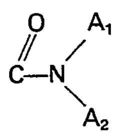

wherein either of $A_1$ and $A_2$ may be H, phenyl, benzyl or a 1—5 carbon alkyl group;

$R_4$ and $R_5$ together form a ring with the nitrogen and carbon atoms to which they are respectively attached, which ring is one of the structures:

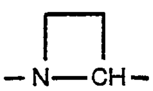 , 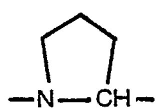 , 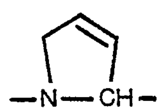 , 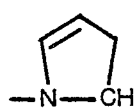 , 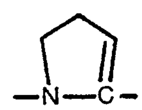 ,

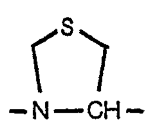 , 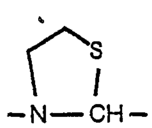 , 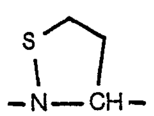 , 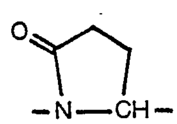 , 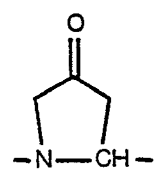 ,

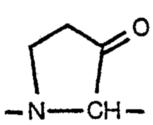 , 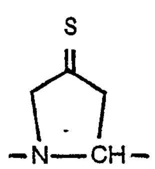 , 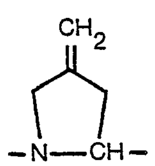 , 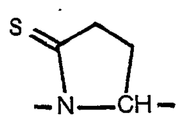 , 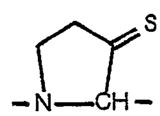 ,

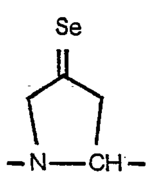 , 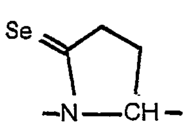 , 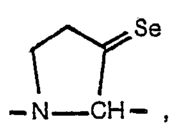 , 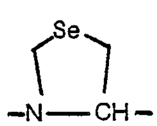 , 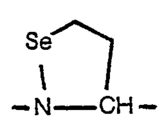 ,

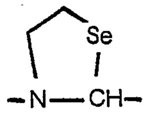 , 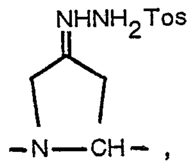 , 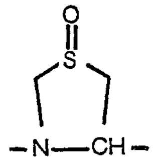 , 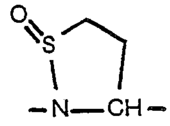 , 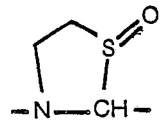 ,

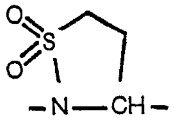 , 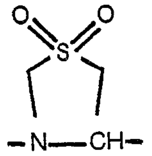 , 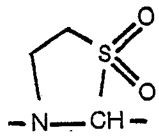 , 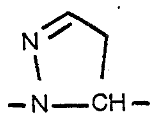 , 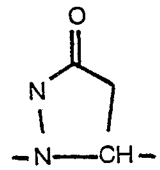 ,

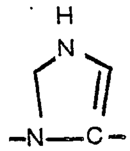 , 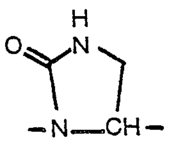 , 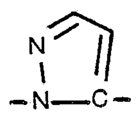 , 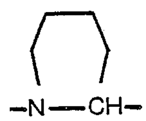 , 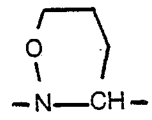 ,

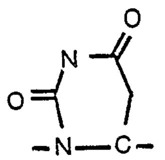 , 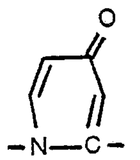 , 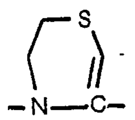 , 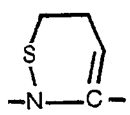 , or 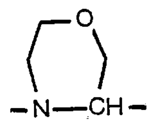 ,

it being understood tht any of these structures may be monosubstituted with

$-OH$, $-OCH_3$, F, $-O$ 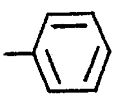 , $OCH_2$ 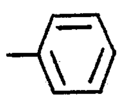 ,

Cl, Br, I, phenyl, hydroxyphenyl, $-SH$, $-SCH_3$, $-S$ 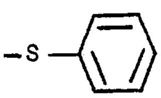 ,

$-SCH_2$ 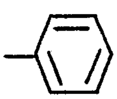 , $-NHCH_3$, $-CH_2NH_2$, $-CH_3$, $-CH_2OH$, propyl,

guanidino, nitroguanidino or thioguanidino and that any of the 5- or 6-membered rings may be disubstituted with —OH, F, Cl, Br, I, $OCH_3$ or any combination of two of this group of substituents;

$R_6$ is —OM or —SM, wherein M may be H, an alkyl group of 1—3 carbon atoms or any other ester moiety hydrolyzable under mammalian *in vivo* conditions to —OH, or an ionically bonded anion of a physiologically acceptable nontoxic salt;

$R_7$ is H—, $CH_3$—, halomethyl, hydroxymethyl, aminomethyl or mercaptomethyl;

$R_8$ is H—, $CH_3$—, amino, halomethyl, hydroxymethyl, aminomethyl, dihalomethyl, trihalomethyl, mercaptomethyl, methoxymethyl, methylthiomethyl, methoxycarbonylmethyl, cyanomethyl, benzyl, acetoxymethyl, $CH_2$=CH—$CH_2$—, isobutyl, mercaptoalkyl of 2—3 carbon atoms, hydroxyalkyl of 2—3 carbon atoms, acetylthioethyl, benzamido, acetamido, phthaloylaminoalkylene wherein the alkylene group has 1—4 carbon atoms, α-alkoxycarbonyl isoalkylene wherein the alkyl group contains 1—5 carbons the isoalkylene group contains 3—5 carbons, benzoylamine, alkanoylamine of 1—5 carbons, alkylamide of 1—5 carbons, phenylamine, alkylamine of 1—5 carbons, or ethyl; and

A. $R_1$ and $R_3$ may each be of the general formula

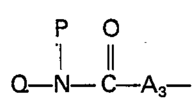

wherein $A_3$ is

(i) alkylene of 1—6 carbons, branched chain alkyl of 1—6 carbons, cycloalkyl alkylene, alkylcycloalkylalkylene, or alkylcycloalkylene;

(ii) aralkylene wherein the alkyl group is 1—6 carbons or alkylaryl;

(iii) phenyl;

(iv) alkylaralkylene wherein the alkyl groups may be the same or different and are 1—6 carbons in length;

(v) substituted alkylene, substituted branched chain alkyl, substituted cycloalkylalkylene, substituted alkylcycloalkylalkylene, substituted alkylcycloalkylene, substituted alkylaryl, substituted aralkylene, substituted phenyl or substituted alkylaralkylene wherein the substituent or substituents may be the same or different, may be included in an alkylene chain or pendant thereto, and are selected from amino, halo, hydroxy, mercapto, $NO_2$, carboxy, $CONH_2$, lower alkyl, halomethyl, hydroxymethyl, aminomethyl, dihalomethyl, trihalomethyl, cyano, mercaptomethyl, methoxymethyl, methylthiomethyl, methoxycarbonylmethyl, cyanomethyl, benzyl, acetoxymethyl, $CH_2=CH—CH_2—$, isobutyl, mercaptoalkyl of 2—3 carbon atoms, hydroxyalkyl of 2—3 carbon atoms, acetylthioethyl, benzamido, acetamido, phthaloylaminoalkylene wherein the alkylene group has 1—4 carbon atoms, -alkoxycarbonyl isoalkylene wherein the alkyl group contains 1—5 carbons and the isoalkylene group contains 3—5 carbons, benzoylamino, alkanoylamino of 1—5 carbons, alkylamide of 1—5 carbons, phenylamine, alkylamine of 1—5 carbons, lower alkoxy, aryloxy, lower alkylamino, diloweralkylamino, acylamino, arylamino, guanidino, imidazolyl, indolyl, lower alkylthio, arylthio, carboxy amido and carbolower alkoxy;

(vi) alkylenethio- or alkylenethioalkylene of 1—6 carbons, alkylthioalkylene of 1—6 carbons;

(vii) alkyleneoxy or alkyleneoxyalkylene wherein the alkyl groups may be the same of different and are 1—6 carbons;

(viii) alkoxyphenyl or alkoxybenzyl in which the alkoxy group has 1—3 carbons, phenoxyphenyl, phenoxybenzyl, benzyloxybenzyl or benzyloxyphenyl or a thioether analog of any of them;

(ix) 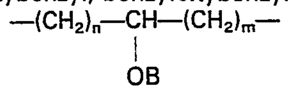

wherein n = 0—4, m = 0—4 and B = H or a 1—5 carbon alkyl group; or an —SB analog thereof;

(x) 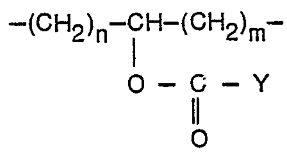 or 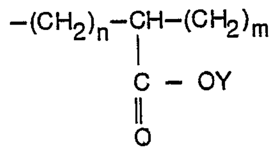

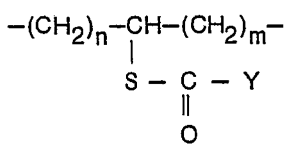 or 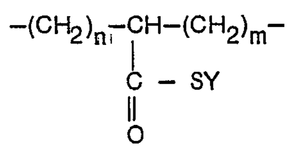

wherein n and m have the same significance as above, Y is phenyl, benzyl or a 1—5 carbon alkyl group;

(xi) 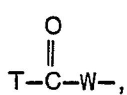, 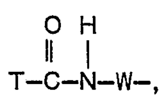, 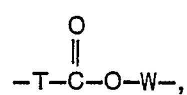, 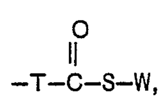,

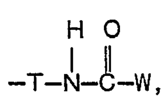, 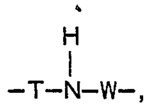, 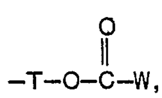, 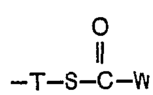

wherein T and W may be the same or different and are alkylene, aryl, benzyl or cycloalkyl; and P and Q may be the same, or one of them may be H or they may combine to form a ring with the nitrogen to which they are attached.

Either or both of P and Q may be selected from any of the following:

(a) $C_1$—$C_6$ straight or branched chain alkyl groups or $C_1$—$C_6$ straight or branched chain alkenyl groups, any one of which may be substituted with any of halo, hydroxy, alkoxy, aryloxy, amino, alkylamino, dialkylamino, alkylacylamino, arylamino, guanidino, thioguanidino, nitroguanidino, hydrazino, ureido, nitro, mercaptocarbonyl, hydroxyamino, histidinyl, cyano, imidazolyl, indolyl, mercapto, alkylthio, arylthio, carboxy amido or carboalkoxy, wherein the alkyl groups contain 1—6 carbon atoms;

(b) cycloalkyl or cycloalkyl alkylene wherein cycloalkyl has 4—12 carbons, and alkylene 1—5 carbons,

which may be substituted with any of —OH, —SH, halo, COOH, COSH, $CONH_2$, $NO_2NH_2$, $NO_2$, $CH_3$, —$OCH_3$,

$-SCH_3$, $-\overset{O}{\overset{\|}{C}}-OCH_3$,

hydrazino, ureido, hydroxyamino, cyano, guanidino, thioguanidino or nitroguanidino groups;

(c) aralkyl or alkaryl groups which may be ring substituted with one or more of the following: SH, halo, $CH_2COOH$, $CH_2CONH_2$, $CH_2CONH$-alkyl, COSH, COOH, $CONH_2$, CONH-alkyl, $CH_2COSH$, $CH_2SH$, $CH_2OH$, OH, $NO_2$, amino, alkyl, alkoxy, aralkyloxy, alkylthio and aralkylthio groups, wherein the alkyl groups contain 1—6 carbons and may also or alternatively be chain substituted with —$CH_3$—, —OH,

$-OCH_3$, halo, $-SCH_3$, $\overset{O}{\overset{\|}{C}}-CH_3$, $-NH_2$, $-NO_2$, —CN, —SH, —NHOH, —$NHNH_2$, $NH-\overset{O}{\overset{\|}{C}}-NH_2$

or a thio or nitro derivative thereof, —COOH or COSH;

(d) an aryl, heterocyclic or adamantanyl group which may be ring-substituted with at least one group selected from halo, —OH, —O-alkyl, —O-aryl, $NH_2$, NH-alkyl, N-$(alkyl)_2$, $alkyl-\overset{O}{\overset{\|}{C}}-NH_2$,

aryl-$NH_2$, guanidino, thioguanidino, nitro-guanidino, hydrazino, ureido, nitro, mercaptocarbonyl, hydroxyamino, cyanoimidazolyl, indanyl, histidinyl,

—SH, —S—alkyl, S—aryl, $-\overset{O}{\overset{\|}{C}}-NH$ , $-\overset{O}{\overset{\|}{C}}-O-alkyl$, $-\overset{O}{\overset{\|}{C}}-alkyl$, $-\overset{O}{\overset{\|}{C}}-O-aryl$, $-\overset{O}{\overset{\|}{C}}-aryl$,

$-\overset{O}{\overset{\|}{C}}-SH$, $\overset{O}{\overset{\|}{C}}-S-alkyl$, $-\overset{O}{\overset{\|}{C}}-S-aryl$, and $-NO_2$

when P and Q join with N to form a ring, the ring may be any 4—10 membered hererocyclic ring which contains a nitrogen with only two of its valences attached to other ring members.

B. Alternatively $R_1$ may be $Q-\underset{}{\overset{P}{\overset{|}{N}}}-\overset{O}{\overset{\|}{C}}-A_3-$

and $R_3$ may be

(i) mono-N substituted alkylene of 2—4 carbons wherein the N substituent is benzoyl, Boc, CbO, Tos, formyl or acetyl;

(ii) hydroxyphenyl or hydroxyphenyl-(1—6C)-alkylene or a thiol analog of either;

(iii) mercaptoalkylene of 1—6 carbons;

(iv) phenylalkylene wherein the alkylene group has 1—6 carbons

(v) phenylthioalkylene or benzylthioalkylene wherein the alkylene group has 1—6 carbons;

(vi) alkylthioalkylene wherein the alkyl and alkylene groups have 1—3 carbons;

(vii) alkoxyphenyl or alkoxybenzyl in which the alkoxy group has 1—3 carbons, phenoxyphenyl, phenoxybenzyl, benzyloxybenzyl or benzyloxyphenyl or a thioether analog of any of them;

(viii) $-(CH_2)_n-\underset{\underset{OB}{|}}{CH}-CH_3$

wherein n = 0—4 and B = H or a 1—6 carbon alkyl group; or an —SB analog thereof;

(ix) $(CH_2)_pCOOZ$ or $(CH_2)_pCOSZ$ wherein p = 0—3 and Z is H, phenyl, benzyl, a 1—5 carbon alkyl group, or an anion of a physiologically acceptable salt;

(x) 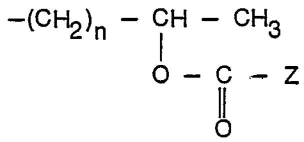 or 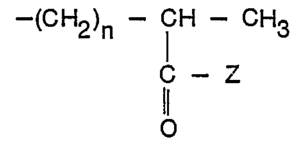

or 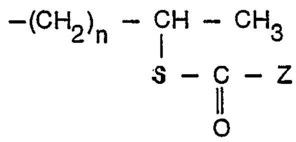 or 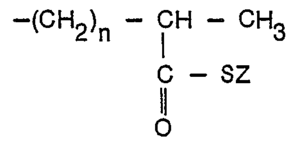

wherein n is 0 to 4 and Z each have the same significance as above;

(xi) 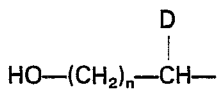 or 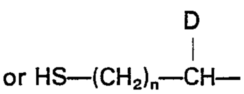

wherein n = 0—4, D is phenyl, thienyl or a 1—3 carbon alkyl group;

(xii) $HO—(CH_2)_n—C(CH_3)_2—$, $HS—(CH_2)_n—C(CH_3)_2—$, p-hydroxyphenyl — $(CH_2)_n—C(CH_3)_2—$ or p-mercaptophenyl-$(CH_2)_n—C(CH_3)_2—$ wherein n has the same significance as above;

(xiii) p-mercaptophenyl-$(CH_2)_n—CH_2—$ or p-hydroxyphenyl-$(CH_2)_n—CH_2—$ wherein the phenyl ring has one or two nitro or amino substituents and n has the same significance as above;

(xiv) 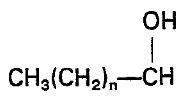 or 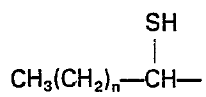

wherein n has the same significance as above;

(xv) $NH_2$-Alkylene or $NO_2$-Alkylene containing one hydroxy or mercapto substituant and having 1—6 carbon atoms;

(xvi) hydroxy- or mercapto-phenoxybenzyl;

(xvii) 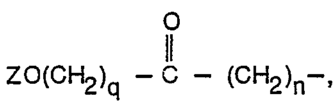, $ZS-(CH_2)_q-O-(CH_2)_n-$,

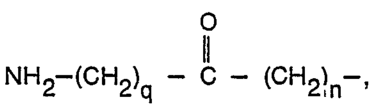, 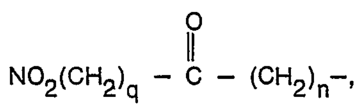, 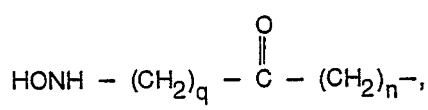,

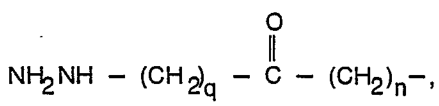, 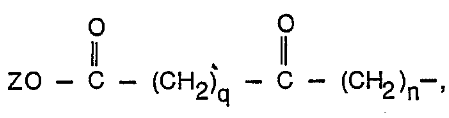,

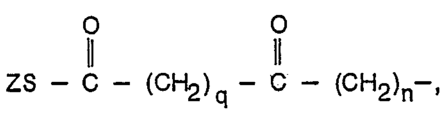, or 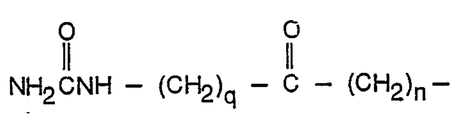

wherein q = 1 — 5 and n is from 0 to 4 and Z has the same significance as above;

(xviii) 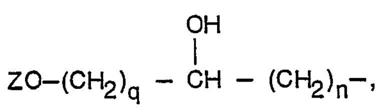, 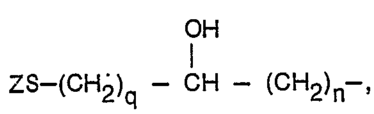,

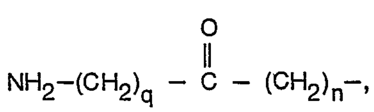, 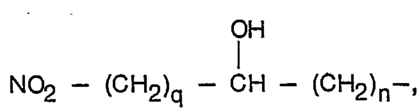,

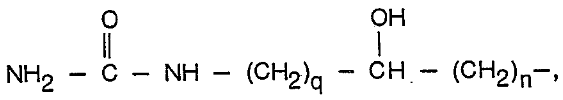 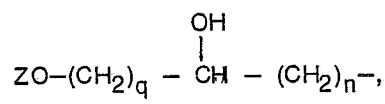

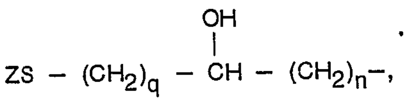

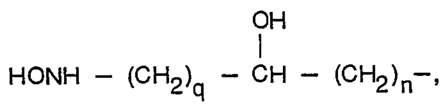 or 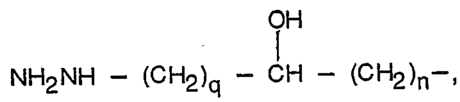

wherein q and n all have the same significance as above;

(xix) 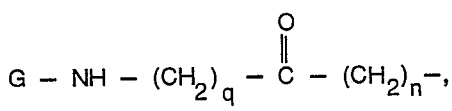 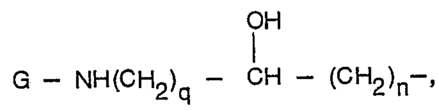

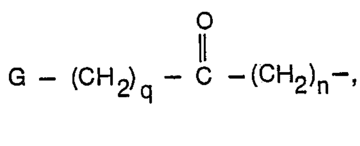 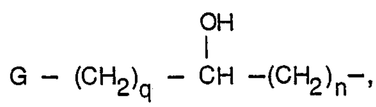 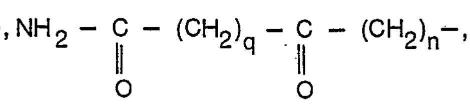

or 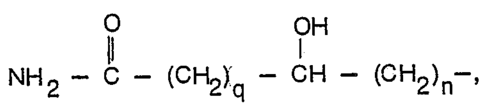

wherein G is an alkacyl or alkacyloxy group of 1—6 carbons, a benzoyl or benzoyloxy group, or a phenylalkacyl or phenylalkacyloxy group wherein the alkacyl or alkacyloxy group contains 2—6 carbons and q and n have the same significance as set forth above;

(xx) 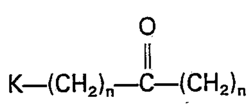 or 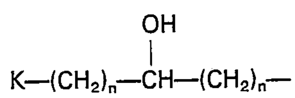

wherein n has the significance stated above and K is selected from carboxyphenyl, aminophenyl, nitrophenyl, halophenyl, hydroxyphenyl, alkylthiophenyl, alkylphenyl, mercaptophenyl, cyanophenyl, mercapto-carbonylphenyl, alkylcarbonylphenyl, alkylcarbonyloxyphenyl, hydrazinophenyl, ureidophenyl, alkylcarbonylaminophenyl, alkylcarbonylthiophenyl, alkoxyphenyl and hydroxy-aminophenyl, wherein all alkyl groups contain 1—6 carbon atoms;

(xxi) 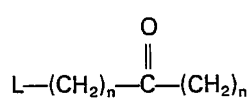 or 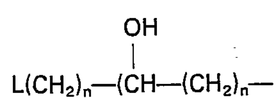

wherein n has the significance stated above and L is selected from cycloalkyl groups of 3—7 carbons which may be unsubstituted or substituted with up to two groups selected from among carboxy, amino, nitro, halo, hydroxy, mercapto, mercaptocarbonyl, hydroxyamino, alkyl, alkylcarbonyl, alkylcarbonyloxy, alkylthio, alkylcarbonylamino, alkylcarbonylthio, cyanohydrazino, ureido and alkyloxy, wherein all alkyl groups contain 1—6 carbon atoms;

(xxii) guanidino alkylene, thioguanidinoalkylene or nitroguanidino alkylene in which the alkylene groups contain 1—6 carbon atoms;

(xxiii) ring substituted aryl groups in which the ring substituents may be the same or different and may comprise up to five per ring of the following: $—NH_2$, —OZ, —SZ, halogen, —CN, $—NO_2$, —COOZ, —COSZ, $CONH_2$, $—NHNH_2$, alkyl alkylcarbonyl, alkylcarbonyloxy, alkylcarbonylamino, haloalkyl, dihaloalkyl, trihalomethyl, hydroxyamino, alkylcarbonylthio, phenoxy, and benzyloxy wherein the alkyl groups contain 1—6 carbon atoms and Z has the same significance as above;

(xxiv) amidoalkylene or alkylcarbonyl-aminoalkylene wherein the alkyl and alkylene groups contain 1—6 carbon atoms;

(xxv) hydroxyaminoalkylene of 1—6 carbons;

(xxvi) vinyl and substituted vinyl groups in which the substituents may be alkyl, aryl, cycloalkyl or heterocyclic groups;

(xxvii) unsubstituted heretocyclic groups from among phenothiazinyl, pyrrolidinyl, pyrrolyl, quinolinyl, imidazolyl, pyridyl, thyminyl, benzothiazinyl, indolyl, thienyl, purinyl, piperidinyl, morpholinyl, azaindolyl, pyrazinyl, pyrimidyl, piperonyl, piperazinyl, furanyl, thiazolyl and thiazolidinyl, cytosinyl;

(xxviii) alkylene or alkenyl groups of 1—6 carbons substituted with one of the heterocyclic rings from (xxvii) above;

(xxix) groups from (xxvii) or (xxviii) above containing up to four ring substituents on the heterocyclic ring selected from among —OZ, —SZ, —COOZ, $-NO_2$, $-NH_2$, —COSZ, halogen, haloalkyl, dihaloalkyl, trihalomethyl, cyano, $CONH_2$, alkyl, alkylcarbonyl, alkylcarbonyloxy, alkylcarbonylamino, alkylcarbonylthio, phenoxy, benzyloxy, 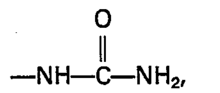

$-NHNH_2$ and HONH—, wherein Z has the same significance as above;

(xxx) groups from (xxvii), (xxviii) or (xxix) attached to one valence of an etheric —O— or —S—;

(xxxi) mono-, di- or tri-alkyl, alkenyl- or phenyl-silyl or -selenyl wherein the alkyl or alkenyl groups contain 1—6 carbons;

(xxxii) any of H, 1—5 carbons straight or branched chain alkyl, phenyl, —OH, alkoxy of 1—6 carbons, benzyloxy, benzyloxyalkylene or phenoxyalkylene wherein the alkylene has 1—5 carbons, alkoxyalkylene having 1—5 carbons in the alkoxy and alkylene groups, aminoalkylene of 1—6 carbons, alkenyl of 1—6 carbons, benzyl, hydroxyalkyl of 1—6 carbons, mercaptoalkyl of 1—6 carbons, histidinyl, haloalkyl of 1—6 carbons, 4-aminomethyl-benzyl, acetamidoalkyl of 1—5 carbons, benzylthiomethylene, or dimethylaminoalkyl of 1—5 carbons.

C. Alternatively, $R_3$ may be 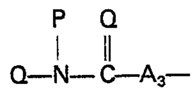 and

$R_1$ may be any of groups (i) — (xxxii) above or any of H, $C_1$—$C_8$ straight or branched chain alkyl, phenyl, benzyl, unsubstituted aminoalkylene of 2—6 carbons, hydroxyalkylene of 1—6 carbons, hydroxyphenyl, phenoxyalkylene or benzyloxyalkylene wherein the alkylene group has 1—6 carbons, cycloalkyl of 3—6 carbons, cycloalkyl methyl, 3 indolyl-, phenylethyl, methylthioethyl, 3 indolyl alkyl wherein the alkyl group contains 1—5 carbons, imidazolyl, imidazolylalkyl wherein the alkyl group contains 1—5 carbons, phenoxymethyl, phenylthiomethyl, 4-aminomethyl benzyl, 2-aminophenethyl, naphthylethyl, 4-halophenethyl, 3,4-dihalophenethyl or phenoxyphenethyl, or $R_1$ and $R_2$ together may form with —CH a lactone ring of the formula:

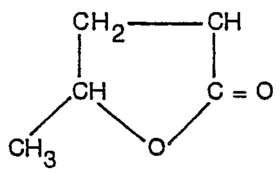

or

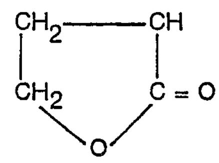

or an analogous six-membered ring.

In the general formula above, asterisks indicate possible asymmetric centers. These centers may be racemized or in any optically active form. However, the S-form is preferred.

The inhibitors are useful as orally effective anti-hypertensive agents.

*Detailed Description of the Invention*

The present invention in its broad aspects relates to thioether, ether and secondary amino compounds containing at least one amino acid or related structure containing the sequence

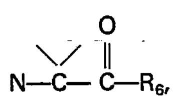 preferably 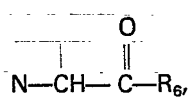

and includes at least one group of the general formula 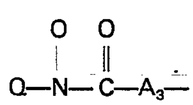

in the $R_1$ or $R_3$ position.

The compounds of this invention wherein X = $NR_9$ may be made in a variety of ways. For example, an alpha keto carboxylic acid of the general formula

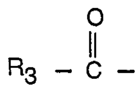 may be coupled to 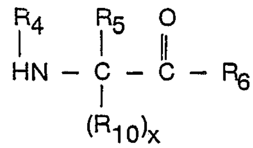

to give a product 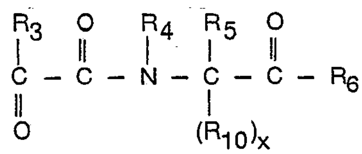

using a conventional coupling agent such as dicyclohexylcarboiimide ("DCC") or diphenylphosphorylazide ("DPPA"). This product in turn may be coupled, in the presence of a reducing agent such as sodium cyanoborohydride to a comound of the general formula

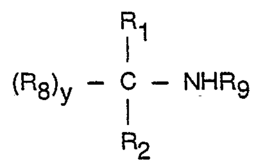

to give the desired compound.

In such an instance 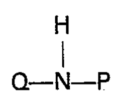

may, e.g. be first reacted with an appropriate ω - carboxylated compound, e.g.,

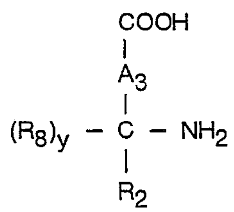 to yield 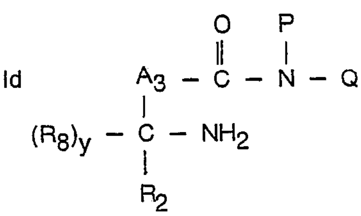

In this particular scheme, e.g.,

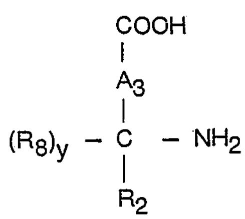

may alternatively first be coupled with 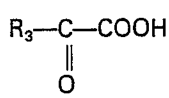

and the product then coupled with 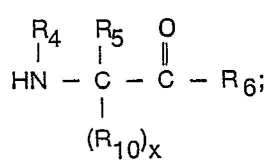;

in such instances Q—N—P may be reacted with the —COOH attached to $A_3$ after the first or second coupling step. Similarly,

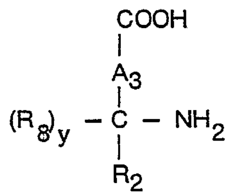 may be coupled to 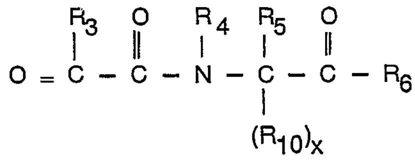

and Q—N—P may then be reacted with the COOH adjacent to $A_3$. As those of ordinary skill in the art will readily understand, conventional blocking groups such as Boc, Cbo etc. may be introduced at appropriate stages to protect reactive groups and may be removed when protection is no longer needed or wanted.

It is within ordinary skill, e.g., to use in lieu of 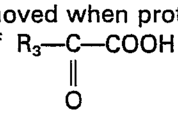 a compound 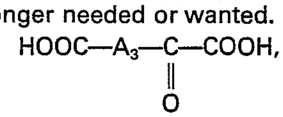 protect either of the COOH groups as desired in the particular reaction scheme preferred and prepare,

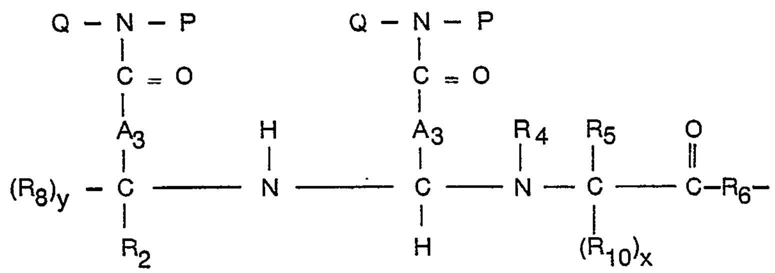 ;

Similarly if 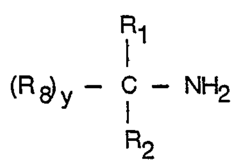

$R_1$ is other than 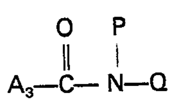 is chosen and 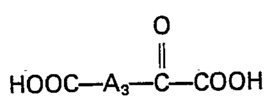 is used in lieu of 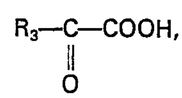, the reactions may be manipulated with appropriate blocking and coupling steps to yield a product

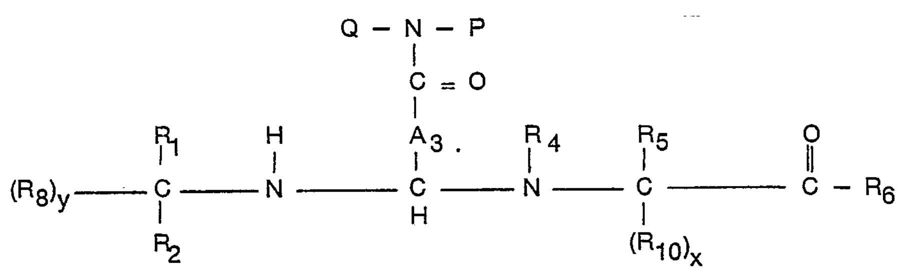

Among suitable ω-carboxylic acids of the general formula

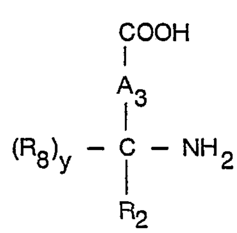 are

glutamic acid α-benzyl ester
glutamic acid α-ethyl ester
glutamic acid α-methyl ester
glutamic acid α-t-butyl ester
aspartic-α-benzyl ester
2-amino-5-carboxy-indan-2-carboxylic acid para-carboxy phenyl alanine α-methyl ester
ortho-carboxy tyrosine α-methyl-ester
2-amino malonic acid monoethyl ester
2-amino adipic acid 1-ethyl ester
2-amino pimelic acid 1-ethyl ester
2-amino suberic acid 1-ethyl ester
2-amino azelaic acid 1-ethyl ester
2-amino sebacic acid 1-ethyl ester and others

which will readily occur to those of ordinary skill in the art.

These acids may be purchased in many instances from, e.g. Aldrich Chemical Co. or Chemical Dynamics Co. It is also well known that α-amino acids of the formula

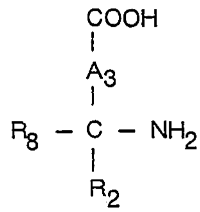

wherein $R_2$ is COOH or another carboxyl function may be obtained from α-keto dicarboxylic acids using methods described by Waters, K. L., Chem. Rev. *41*, 585-98 (1947).

Among suitable compounds of the general formula 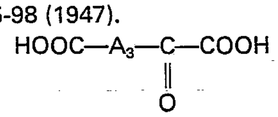 referred to above are:

α-keto glutaric acid
oxalacetic acid
ketomalonic acid
4-keto pimelic acid
para-carboxy phenyl pyruvic acid
indole-1-carboxy-3-pyruvic acid
β-carboxy-DL-lactic acid
2-ketoadipic acid

It is to be understood that when $R_3$ = 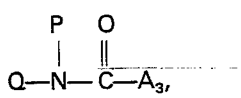, the compound of the general formula 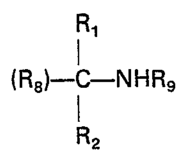 can be selected from a very wide group.

Suitable $R_1$ compounds of the general formula 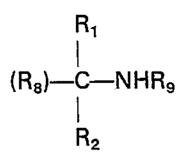 for use in making the compounds of the invention include, but are not limited to *tert.*leucine, 2-methylglutamic acid, α-amino-γ-guanidino butyric acid, α-amino-β-guanidinopropionic acid, β-fluorophenylalanine, β-hydroxyvaline, α-oxalysine, 3-hydroxy ornithine, $N^6$-hydroxylysine, $N^8$-methyl arginine, $N^8$-hydroxyarginine, canvanin, 5,5$^1$-dihydroxyleucine, β-carboxyaspartic acid, β-fluoroaspartic acid, β-methylaspartic acid, β-methylene aspartic acid, p-amido phenylalanine, p-guanidinophenylalanine, p-methyl-phenylalanine, 2-ethoxy-5-nitrophenyl-alanine, 2-hydroxy-5-nitrophenylalanine, 4-mercaptophenylalanine, 2-amino-2-indoleacetic acid, 2-amino-3-adamantylpropionic acid, β-methylene norvaline, 3-chloroglutamic acid, α-amino-γ-nitrovaleric acid, 4-azalysine, β-(2,4,5-trihydroxyphenyl)-alanine, β-(3-bromo-5-methoxyphenyl) alanine, β-(3,5 dimethyl-4 methoxyphenyl) alanine, 3,5-di (ethylthio)-4-(4′ hydroxyphenoxy)-phenylalanine, 3,5-di (ethylthio)-4(3′-isopropyl-4′-methoxyphenoxy)-phenylalanine, β-pyrrolyl-alanine, 2-amino-4-pyrrolyl-butyric acid, 2-amino-5-pyrrolyl-valeric acid, β-(2 pyridyl) alanine, β-(3 pyridyl) alanine, β(6-aminopurin-9yl) alanine, β-(4-amino-2-hydroxypyrimidin-1-yl) alanine, β-(2,4 dihydroxy-5 methyl-pyrimidin-1-yl) alanine, β-(6-hydroxy-purin-9-yl) alanine, β(6-dimethylamino-purin-9-yl) alanine, β-(6-mercaptopurin-9-yl) alanine, β-(6-methylthiopurin-9-yl) alanine, 4-azatryptophan, 4-methyl-6-chloro-7-azatryptophan, $N^{\varepsilon}$-(1,4-dehydro-6 methyl-3-hydroxy-4-oxo-1 pyridyl) lysine, S-(2-hydroxy-2-carboxyethanethiomethyl)-cysteine, 2-amino-3-(6-thieno[3,2-*b*]pyrrolyl) propionic acid, 3,3,',5,5' tetramethyl thyronine, 3-hydroxy-L-lysine, 2-aminohex-4-ynoic acid, N-hydroxyornithine, 4-piperazinobut-2-ynoic acid, 4-piperidinobut-2-ynoic acid, 4-pyrrolidinobut-2-ynoic acid, α-amino-$N^{\gamma}$-nitro-guanidinobutyric acid, α-amino β(1-imidazolyl) propionic acid, 4-nitrohistidine, 2-methyl-3 (2',4'-diiodo 5'-hydroxyphenyl) alanine, 4-(3'amino-2',4',6'-triiodophenyl)-isovaline, 4-(3' acetamido-2', 4',6'-triiodophenyl)-isovaline, 4-(3'-hydroxy-2', 4', 6'-triiodophenyl)isovaline, 2-amino-4-thiosulfobutyric acid, S-(3-aminopropyl) homecysteine, S-(cyclopentyl methyl) homocysteine, 5'-guanosyl homocysteine, β-(cytosin-1-yl)-alanine,

S-[(diphenyl-α-naphthyl)methyl]-L-cysteine,
S-[(diphenyl-β-naphthyl)methyl]-L-cysteine,
2-amino-6-(methylthio)caproic acid,
$N^{G}N^{G}$-dimethyl-L-arginine,
$N^{G}N'^{G}$-dimethyl-L-arginine,
$N^{\varepsilon}N^{\varepsilon}N^{\varepsilon}$-trimethyl-δ-hydroxy-L-lysine,
$N^{e}$-(5-amino-5-carboxypentyl)-5-hydroxy-L-lysine,
δε-dihydroxy-L-norleucine,
cis-1-amino-1,3-dicarboxycyclohexane,
trans-1-amino-1,3-dicarboxycyclohexane,
3,3,4,4,4,-pentafluoro-2-aminobutyric acid,
3,3,4,4,5,5,5-heptafluoro-2-aminovaleric acid,
ω-fluoro-DL-and L-allo-isoleucine,
2,6-diamino-4-hexynoic acid,
O-(α-D-glucopyranosyl)-L-serine,
2-amino-5,6-dihydroxyindan-2-carboxylic acid,
3-(m-fluorophenyl)-2-methylalanine,
3-(m-bromophenyl)-2-methylalanine,
3-(m-iodophenyl)-2-methylalanine,
2-[(m-iodophenyl)methyl]glycine,
4-(m-iodophenyl)-2-methyl-2-aminobutyric acid
3,5,3'-tri-isopropyl-DL-thyronine,
3,5-dimethyl-3'-isopropyl-thyronine
3,5-di-isopropyl-thyronine,
3,5-di-isopropyl-4'-amino-thyronine,
3,5-di-isopropyl-3'-bromo-thyronine,
3,5-di-isopropyl-3'-methyl-thyronine,
3,5-di-s-butyl-thyronine,
3,5-di-s-butyl-4'-amino-thyronine,
3,5-di-s-butyl-3'-bromo-thyronine,
3,5-di-s-butyl-3'-iodo-thyronine,
4-fluoro-tryptophan,
5-fluoro-tryptophan,
6-fluoro-tryptophan,
β-5(-hydroxy-6-iodo-2-pyridyl)-alanine,
β-(benzimidazol-5-yl)-alanine,
β-(2-amino-6-hydroxypurin-9-yl)-alanine,
β-(2-amino-6-mercaptopurin-9-yl)-alanine,
$N^{\varepsilon}$-(5-Amino-6-chloro-4-pyrimidyl)lysine,
α-Amino-ε-(6-chloro-9-purinyl)caproic acid,
4-Fluoro-DL-histidine,
S-Methyl-2-methyl-cysteine,
S-Ethyl-2-methyl-cysteine,
S-propyl-2-methyl-cysteine,
S-Isopropyl-2-methyl-cysteine,
S-Butyl-2-methyl-cysteine,
S-Isobutyl-2-methyl-cysteine,
S-t-Butyl-2-methyl-cysteine,
S-Amyl-2-methyl-cysteine,
S-Isoamyl-2-methyl-cysteine,
S-Allyl-2-methyl-cysteine,
S-(β-Aminoethyl)homocysteine,

γ,δ,δ'-trihydroxy-leucine,
$N^{\varepsilon}$-(indole-3-acetyl)-lysine,
p-hydroxymethylphenylalanine,
O-ethylhomoserine,
5-methyl-2-aminohex-4-enoic acid,
α-(3-hydroxyphenyl)glycine,
α-(3,5-dihydroxyphenyl)glycine,
β-(cyclohexa-1,4-dienyl)alanine,
β-(cyclohex-1-enyl)-alanine,
β-(1-hydroxycyclohexyl)-alanine,
4-bromoacetyl-phenylalanine,
4-bromoacetamido-phenylalanine,
3-chloroacetamido-phenylalanine,
4-fluoro-3-chloroacetamido-phenylalanine,
3,4,5-tri-iodo-phenylalanine,
3,5-di-isopropyl-3'-iodo-thyronine,
β-(4-methoxy-1-naphthyl)-α-methylalanine,
β-(4-hydroxy-1-naphthyl)-α-methylalanine,
α-(2-indanyl)glycine,
β-trimethylsilyl-alanine,
α-amino-β-(methylamino)propionic acid,
$N^{\varepsilon}N^{\varepsilon}$-bis(2-cyanoethyl)-lysine,
α,γ-dimethylnorleucine,
α-methyl-$N^{\varepsilon}N^{\varepsilon}$-diethylornithine,
α-ethyl-3,4-dimethoxy-phenylalanine,
α-methyl-4-morpholino-phenylalanine,
β-(2-amino-4-pyrimidinyl)alanine,
3-(2-Methyl-4,5-dihydroxyphenyl)-alanine,
3-(2-Ethyl-4,5-dihydroxyphenyl)-alanine,
3-(2-Isopropyl-4,5-dihydroxyphenyl)-alanine,
3-(2-t-Butyl-4,5-dihydroxyphenyl)-alanine,
3-(2,5-Dimethoxy-4-methylphenyl)-alanine,
3-Ethyl-α-methyl-tyrosine,
2-amino-3,3-dimethylhex-5-enoic acid,
2-aminohexa-4,5-dienoic acid,
2-amino-3,3-dimethylhexa-4,5-dienoic acid,
2-aminohepta-4-5-dienoic acid,
2-amino-3,3-dimethylhepta-4,5-dienoic acid,
2-amino-3,3-dimethylnona-4,5-dienoic acid,
2-aminohepta-5,6-dienoic acid,
2-amino-3-methylhepta-5,6-dienoic acid,
2-amino-5-t-butyl-6,6-dimethylhepta-3,4-dienoic acid,
2-amino-5-methylhepta-3,4-dienoic acid,
2-aminohept-4-en-6-ynoic acid,
ε-hydroxy-β-carboxy-norleucine,
β-carboxy-lysine,
β-(3,4-dihydroxyphenyl)-α-methyl-serine,
S-benzyl-β,γ-dimethyl-homocysteine,
S-benzyl-α,γ,γ-trimethyl-homocysteine,
β-methyl-methionine,
α-methyl-selenomethionine,
β-methyl-L-selenomethionine,
γ-methyl-selenomethionine,
γ,γ'-difluoro-valine,
δ,δ'-difluoro-leucine,
γ-fluoro-allothreonine,
β-hydroxy-asparagine,
β-hydroxy-isoleucine,
β-methoxy-isoleucine,
a-amino-γ-(methylamino)butyric acid,
α-amino-β-(ethylamino)propionic acid,
3-Isopropyl-α-methyl-tyrosine,
3-t-Butyl-α-methyl-tyrosine,
2-Amino-5-hydroxy-indan-2-carboxylic acid,
2-Amino-5-methoxy-indan-2-carboxylic acid,

2-Amino-5-carboxy-indan-2-carboxylic acid,
2-Amino-5-chloro-indan-2-carboxylic acid,
2-Amino-5-bromo-indan-2-carboxylic acid,
2-Amino-5-iodo-indan-2-carboxylic acid,
3-(2,4-Difluorophenyl)-alanine,
3-(3,4-Difluorophenyl)-alanine,
3-(3,5-Difluorophenyl)-alanine,
3-(2,5-Difluorophenyl)-alanine,
3-(2,6-Difluorophenyl)-alanine,
3-(2,3,5,6-Tetrafluorophenyl)-alanine,
3-(3,5-Dichloro-2,4,6-trifluorophenyl)-alanine,
3-(2,3,4,5,6-Pentafluorophenyl)-alanine,
β-(1,2-Dihydro-2-oxo-3-pyridyl)-alanine,
β-(1,2-Dihydro-2-oxo-4-pyridyl)-alanine,
β-(1,2-Dihydro-2-oxo-5-pyridyl)-alanine,
β-(1,2-Dihydro-2-oxo-6-pyridyl)-alanine,
β-(2-Fluoro-3-pyridyl)-alanine,
β-(2-Fluoro-5-pyridyl)-alanine,
β-(2-Fluoro-6-pyridyl)-alanine,
β-(2-Bromo-3-pyridyl)-alanine,
β-(2-Bromo-4-pyridyl)-alanine,
β-(2-Bromo-5-pyridyl)-alanine,
β-(2-Bromo-6-pyridyl)-alanine,
β-(2-Chloro-3-pyridyl)-alanine,
β-(2-Chloro-4-pyridyl)-alanine,
β-(2-Chloro-5-pyridyl)-alanine,
β-(2-Chloro-6-pyridyl)-alanine,
β-(Thymin-1-yl)-alanine,

It is further contemplated that 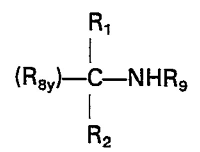

may be selected from among any of the known amino acids or esters or from amides thereof in which, when $R_1$ is any of $CH_3$, $NH_2—(CH_2)_3$, $(NH_2(CH_2)_4—$, $CH_3S(CH_2)_2—$, benzyl-, p-hydroxybenzyl, 3,4-dimethoxy-benzyl,

$CH_3O\overset{O}{\overset{\|}{C}}—(CH_2)_2—$, or 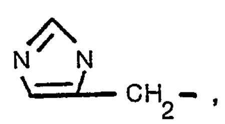

$R_8$ is selected from among $CH_3—$, $(CH_3)_2CH—CH_2—$, Pht $N(CH_2)_3—$, $CH_2=CH—CH_2—$, benzyl-, nitrilomethylene-, ethyl-, $CH_3O—\overset{O}{\overset{\|}{C}}—CH_2—$, $CH_3OCH_2—$, $CH_3SCH_2—$, $—CH_2F$, $—CHF_2$, $—CF_3$, $—CH_2Cl$, $—CF_2Br$, PhtN $(CH_2)_2$)τ $CH_3\overset{O}{\overset{\|}{C}}—S(CH_2)_3—$, $HS(CH_2)_3—$, or 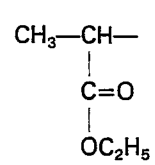

There are also known aminoacids, and esters or primary amides thereof in which, when $R_8$ is hydroxymethyl, $R_1$ may be methyl, ethyl, isopropyl, isobutyl, phenyl, benzyl or methylthioethyl.

It is also contemplated that reactants of the general formula

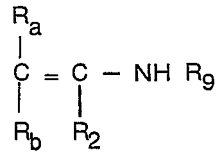

wherein $R_2$ is COOH may be utilized in lieu of 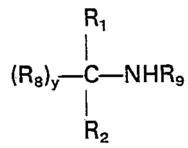 in the coupling reaction with 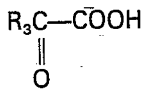 or its coupling product already described.

In such case, 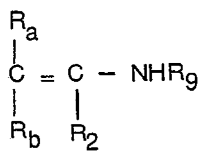 may be, e.g. dehydroalanine, α,β-dehydrophenylalanine, vinyl glycine or a known compound in which $R_a$ and $R_b$ are both methyl or ethyl or $R_a$ is phenyl or a substituted phenyl group such as 3,4-dimethoxyphenyl and $R_b$ is methyl. In this instance various functional groups such as halo, hydroxy or mercapto groups and their methylene analogs, may later be added to one or both carbons of the unsaturated bond via well known and conventional organic chemical procedures.

Many suitable variations in 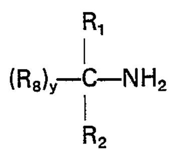

will readily occur to those of ordinary skill in the art.

Another general method for synthesizing compounds of this invention is to couple a suitable α keto carboxylic acid with a suitable dipeptide derivative. A suitable α keto acid can be formed in the reaction,

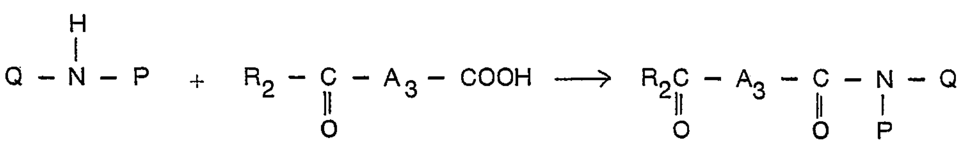

I

in the presence of a conventional coupling agent. An appropriate dipeptide derivative can be formed in the reaction

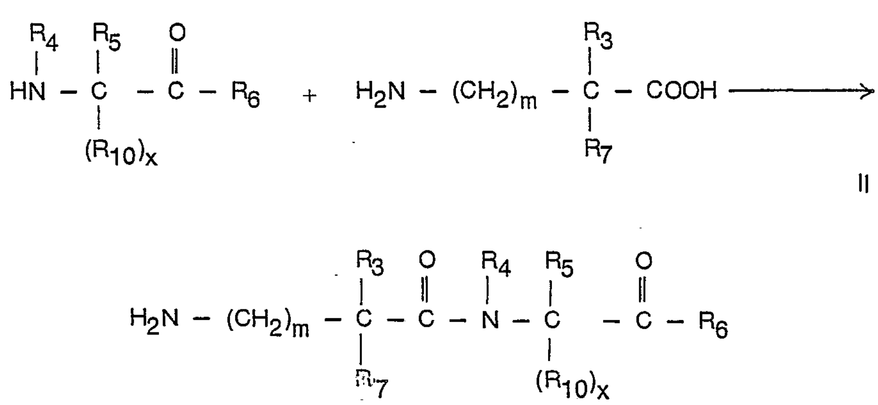

II

Compounds of this invention are then obtained by reacting I and II. Alternative schemes are readily apparent, for example,

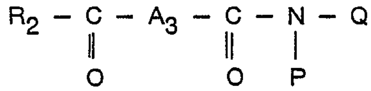

may be reacted with 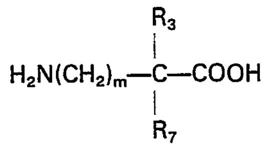

and the product then reacted with 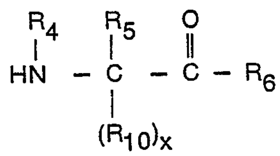.

Suitable compounds of the formula 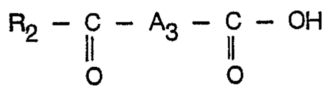

for use in this synthesis method include, but are in no sense limited to, acetoacetic acid, 5-aminolevulinic acid, acetobutyric acid, acetyl cyclopentanecarboxylic acid, chloromethylketocyclopentane carboxylic acid, dibromomethylketocyclohexanecarboxylic acid, 1-acetyl-4-piperidinecarboxylic acid, N-acetyltryptophan, p-carboxyphenoxyacetic acid, 2-benzoylbenzoic acid, 4-benzoylbenzoic acid, 4-benzoylbutyric acid, 3-benzoylpropionic acid, mercaptoacetophenone-4-carboxylic acid, hydroxyethylbenzoyl benzoic acid, the pyruvoyl alkyl carboxylic acids, and others which will readily occur to those of ordinary skill in the art.

Suitable compounds of the formula, 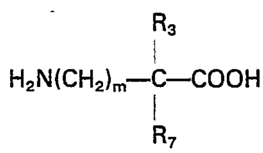

include, but are by no means limited to 2-methylalanine, histidine, N-acetyl-lysine, tryptophan, α-methyltryptophan, albizziin, 2-amino-adipic acid, p-aminophenylalanine, phenylalanine, arginine, aspartic acid, asparagine, 2-methylglutamic acid, N-hydroxylysine, 2-amino-3-adamantyl propionic acid, α-hydroxymethylalanine, α-methyl methionine, α-methyl-N,N-diethylornithine, α-methyl-4 morpholino-phenylalanine, β-(4-methoxy-1-naphthoyl) α-methylalanine, and β-(4-hydroxy-1-naphthoyl)α-methyl-alanine, α-ethyl-3,4-dimethoxy phenylalanine and others which will readily occur to those of ordinary skill in the art.

So long as $R_1$ is of the formula 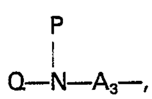,

any compound of the general formula 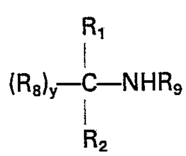

given above, wherein $R_9$=H, and $R_2$=COOH may be used as 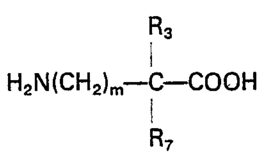

In these cases $R_1$ becomes $R_3$, $R_8$ becomes $R_7$ and m = O.

Useful compounds of the type 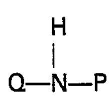

include aminoacenaphthene, para-morpholinoaniline, piperidine, phenylpiperidine, hydantoin, alloxazine, rhodanine, morpholine, aminophenanthrene, adenosine, adamantanamine, adenine, C-aminoacridine, C-aminopyrimidine, aminoanthracene, aminoanthraquinone, aminoantipyrine, aminophenol, amino-naphthalene, aminobenzophenone, C-aminobenzothiadiazole, C-aminobenzothiazole, benzothiazole, aminobiphenyl, C-aminopyridine, C-aminothiazole, pyrazole, C-aminopyrazole, C-aminobenzoxazole, C-aminopurine, aminochrysene, aminocyclopentane, aminocyclopropane, aminocyclobutane, amino-cyclohexane, aminocycloheptane, aminocyclooctane, aminocyclononane, aminocyclodecane, C-aminobenzimidazole, C-aminopteridine, N-aminopiperidine, C-amino-1,2,4-triazine, C-aminouracil, uracil, C-amino,N,N-dimethyluracil, aminodiphenylmethane, N-aminoethylimidazoline, N-aminoethylmorpholine, C-aminomorpholine, N-aminoethylpiperazine, C-aminopiperazine, N-aminoethylpiperidine, 3-amino-N-ethylpiperidine, 2-aminoethylpyridine, N(aminoethyl)-pyrrolidine, pyrrolidine, aminofluoroanthene, 1-, 2-, or 4-aminofluorenone, aminohexane, aminopentane, N-aminohomopiperidine, homopiperidine, 1-amino, 4-(βhydroxyethyl) piperazine, amino-9-hydroxyfluorene, 2-amino-4-hydroxy-6-methylpyrimidine, 4-amino-6-hydroxypyrazole, 4-aminoimidazole, aminoindan, C-aminoindazole, C-aminoindole, 1- or 5-aminoiso-quinoline, 3-amino-mercapto-1, 2, 4-triazole, 4-aminobutanol-1, 5-aminopentanol-1, 2-aminomethyl-1-ethylpyrrolidine, 5-aminoisothiazole, 2-amino-6-methylmercaptopurine, 6-aminohexanol-1, 1-amino-4-methylpiperazine, 4-aminomethylpiperidine, 2-amino-1, 3, 4-thiadiazole, 2-amino-4-methyl thiazole, N-aminomorpholine, 2-amino-4-morpholine-s-triazine, 4-amino-1,8-naphthalimide, 6-aminonicotinamide, 5-amino-6-nitroquinoline, 2-amino-5-nitrothiazole, 6-aminopenicillanic acid, 4-aminophenyl ether, 2(p-aminophenyl)-6-methylbenzothiazole, 3-amino-1-phenyl-2-pyrazoline-5-one, 3-aminophthalhydrazide, N-aminophthalimide, 2-aminopecoline, N-aminopiperidine, 3-aminopropanol-1, N-(3-aminopropyl) morpholine, N-(3-aminopropyl), ethanolamine, N(3-aminopropyl) pyrrolidinone, 2-amino-6-purinethiol, aminopyrazine, 3-aminopyrazole, 4-aminopyrazolopyrimidine, aminopyrene, 4-aminoquinaldine, N-aminorhodanine, 4- or 5-aminosalicylic acid, 5-aminotetrazole, tetrazole, 2-aminothiazoline, aminovaleric acid, aniline, 3,4-dimethoxyaniline, aminoxylene, benzisooxazole, o- or p-aminobenzamide, o- or p-aminobenzoic acid, o- or p-aminobenzonitrile, 8-aza-6-aminopurine, 2-azacyclooctanone, 3-azabicyclononane, 2-azacytidine, 5-azacytosine, cytosine, 6-azacytosine, 5- or 6-azauracid, azetidine, aminoazulene, barbituric acid, aminobenzofluorene, C-aminobenzofuran, benzothiazinone, benzylpiperazine, bis(2-ethoxyethyl)-amine, bromoguanine, bromoisatin, ε-caprolactam, carbazole, tryptophan, glycine, glycinamide, glycinanilide, oxazolidine, oxazolidinone, 8-chlorotheophylline, chlorzoxazone, creatinine, aminocyclo-heptadiene, aminocyclooctatriene, aminocyclooctratetraene, cycloserine, cytidine, cytosinecarboxylic acid, dehydroabietylamine, 4,5-diaminoacenaphthene, aminobenzidine, aminothiophene, dimethylhydantoin, aminofuran, N,N-diethylethylenediamine, aminotoluene, aminoidenone, ethyl-4-amino-5-imidazole carboxylate, α-methyltryptamine, glutamine, glutathione, glutarimide, guanine, guanosine histamine, dodecamethyleneimine, homocarnosine, dithiouracil, 2,2′-dipyridylamine, 2,5-dimethyl-3-pyrroline, 2,6-dimethylpiperazine, isoamarine, glycouril, leucinol, leucenol, myrtanylamine, nicotinamide, homopiper-azine, isonicotinamide, 6-β-hydroxyethylamino pureine, aminonorbornane, aminonorbornene, orotic acid, oxindole, phenoxazine, proline, phthalimide, pyrimidone, pyrole, <Glu, thiazolidine, triacanthine, and 1,2,4-triazole. The various compounds named can be substituted with, e.g., —OH, halo, dihalomethyl, trihalomethyl, —SH, O-alkyl, S-alkyl, phenyl, O-phenyl, S-phenyl, COOY, alkylcarbonyloxy, ureido, cyano, hydroxylamino, alkyl, alkoxyalkyl, alkoxyphenyl, phenoxyphenyl and the like. These compounds are illustrative, rather than limiting, as to suitable

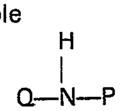

compounds.

It will be understood that

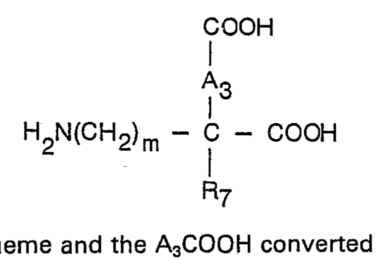

may be utilized in this particular scheme and the $A_3COOH$ converted to

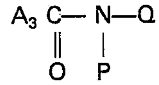

at any desired stage of the synthesis process.

A variety of known methods can be employed to esterify or block any carboxyl group of a multi-carboxyl amino acid or an α-keto carboxylic acid. See, for example, Schroder E. et al, The Peptides Vol. 1, Academic Press (1965) pp. 181—207, and Merrifield, R. B., *Adv. Enzym. 32,* 221 (1969). Furthermore, many of these precursors can be obtained commercially, e.g., from Chemical Dynamics, South Plainfield, N.J., or from Bachem Chemical Co., Torrance, California.

Another method for synthesizing compounds of Formula I involves the use of a diazomethyl intermediate. See, for example, Boyer, J. H. et al *Chem. Rev. 54,* 1—57 (1954); *Aldrichimica Acta,* 3(4) 9

(1970) an article available from Aldrich Chemical Co., Milwaukee, Wisconsin; Lieber, E. et al, *Chem. Rev. 65,* 377—384 (1965); L'Abbe, G. *Chem. Rev. 69.* 345—363 (1969). This method is especially useful for synthesizing compounds of the invention wherein $A_3$= $CH_2$—. Typically a carboxylic acid is reacted with diazomethane via a mixed anhydride reaction, e.g.,

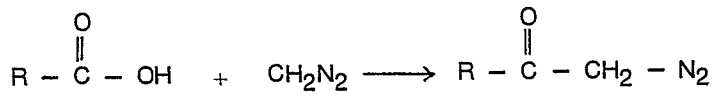

the product is then reacted with an acid such as HBr or HCl, in a solvent such as ethyl acetate, to form an α-haloketone as follows:

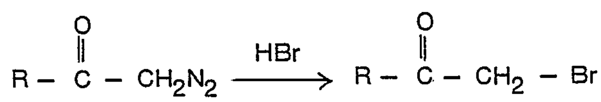


The α-haloketone can then be reacted with an equivalent of diethylformamidomalonate, then decarboxylated in aqueous HCl to form derivatives of 2-amino-4-keto carboxylic acid, that is compounds of the formula

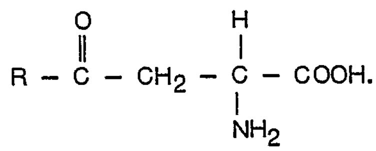

Compounds of this general formula can then be coupled with compounds of general formula

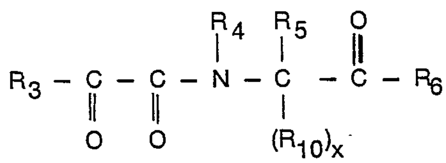

in the presence of a reducing agent such as sodium cyanoborohydride in aqueous solution with an organic solvent (for example $CH_2Cl_2$ or $CHCl_3$) to form compounds of the invention. Alternatively,

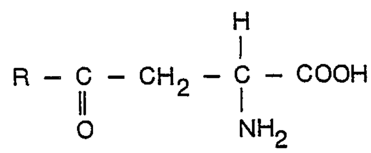

can be coupled with

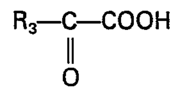

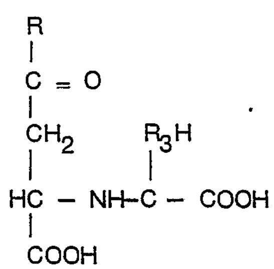
which in turn is coupled with
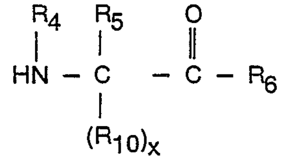

in the presence of DCC or DPPA to form a compound of this invention.

The diazomethyl intermediate can be formed with virtually any carboxylated organic compound. Thus,

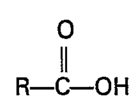

can be a difunctional or trifunctional amino acid, any dicarboxylic acid or any carboxylic acid. Appropriate protecting groups may also be necessary.

The thioether compounds of this invention can be produced by several methods of synthesis. In the examples of synthesis which follow for the thioether as well as the ether and secondary amine compounds, proline will be utilized as prototype amino acid moiety. It is to be understood that this is done for illustration purposes only and that the other ring structures

$$\overset{R_4}{\overset{|}{N}} - \overset{R_5}{\overset{|}{C}} - (R_{10})_x$$

can be substituted for proline in these methods unless noted otherwise. According to one preferred method the compound

$R_1\overset{R_8}{\overset{|}{C}}(OH)COOC_2H_5$ is reacted with $P_2S_5$ to form the compound $R_1\overset{R_8}{\overset{|}{C}}(SH)COOC_2H_5$.

The compound $CH_2{=}C(R_3)COCl$ is reacted with Pro to form $CH_2{=}C(R_3)\overset{O}{\overset{\|}{C}}{-}Pro$

The two products of these reactions are reacted together to yield the compounds

$$R_1\overset{R_8}{\overset{|}{C}}\ (COOC_2H_5)S - CH_2 - \overset{H}{\overset{|}{C}}(R_3) - \overset{O}{\overset{\|}{C}} - Pro.$$

Saponification removes the ethyl alcohol radical and forms the corresponding salt. The free acid can be formed therefrom by acidification. In this method, △ Pro cannot be substituted for Pro.

In a second preferred method, the compound $R_1\overset{R_8}{\overset{|}{C}}(Br)COOH$

is reacted with isobutylene in the presence of sulfuric acid to form the t-butyl ester of $R_1\overset{R_8}{\overset{|}{C}}(Br)COOH$.

This compound is then reacted with $R_3\underset{(CH_2)_m-SH}{\underset{|}{\overset{R_7}{\overset{|}{C}}}}\ COOH$ to form the product:

$$R_1\overset{R_8}{\overset{|}{C}}\ (COOC(CH_3)_3) - S - (CH_2)_m - \overset{R_7}{\overset{|}{C}}(R_3) - \overset{O}{\overset{\|}{C}}OH.$$

Next, the t-butyl ester of Pro is coupled to this product using conventional coupling methods, such as the dicyclohexylcarbodiimide (DCC) method. Other useful coupling methods include the mixed anhydride, symmetrical anhydride, acid chloride, active ester, Woodward reagent K, or the like, methods. For a review of the coupling methods, see *Methoden der Organischen Chemie* (Houben-Weyl), Vol. XV, part II, page 1 et seq. (1974).

The product formed,

$$R_1\overset{R_8}{\overset{|}{C}}(COOC(CH_3)_3) - S - CH_2)_m - \underset{R_7}{\underset{|}{C}}\ (R_3) - \overset{O}{\overset{\|}{C}} - Pro - t - butyl$$

is deprotected, i.e. the t-butyl ester groups are removed by conventional means such as treatment with trifluoroacetic acid (TFA) and anisole to produce the desired product. For a review of other deprotecting methods, see *Methoden der Organischen Chemie* (Houben-Weyl), Vol. XV, part I, page 376 et seq. (1974).

An alternative method is the reaction of 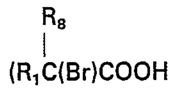 with 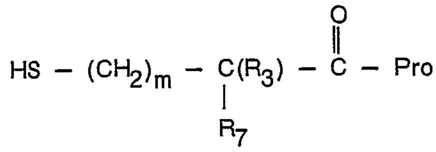

to form the desired product. This alternative method will not work for △ Pro although the original method will.

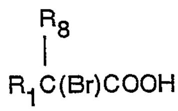 can be prepared by reacting 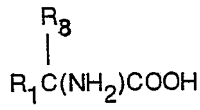

with HBr in the presence of $NaNO_2$ or with $KB_r$ in 2.5 N $H_2SO_4$ in the presence of $NaNO_2$ to yield the desired product.

The ether compounds of formula I can also be prepared by several other methods. According to one preferred method, the compound $R_1CH_2Cl$ is reacted with the diethyl ester of malonic acid to yield

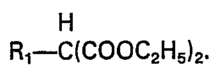

This product is then reacted with bromine to produce $R_1{-}C(Br)(COOC_2H_5)_2$. The compound

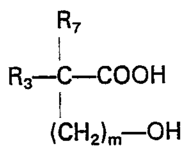

is coupled to an ester of Pro using conventional coupling means. These two products are then reacted to form the compound

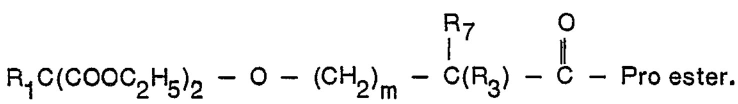

The various ester groups are removed by conventional means and one carboxyl group is removed by acidification and heat produce the desired product. By substituting

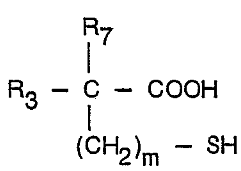 for the 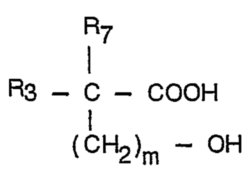

and following this procedure, the thioether compounds can be formed but this will not work with △ Pro.

In still another method, the compound 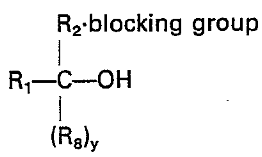

is reacted with 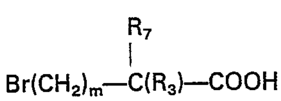 t-butyl ester to produce 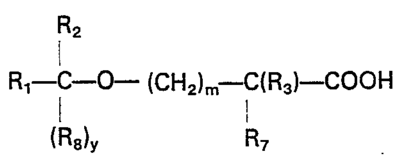

t-butyl ester. The ester is removed, the product reacted with Pro, and the blocking group is removed to yield a final product according to this invention.

The secondary amine compounds of this invention wherein $R_8$ is H and X is NH can also be synthesized by the following method. The compound

$$R_1\overset{\overset{O}{\|}}{C}—COOH$$

is coupled with thiophenol using the mixed anhydride method to produce $R_1—\overset{\overset{O}{\|}}{C}—\overset{\overset{O}{\|}}{C}—S—C_6H_5$.

This product is then reacted with $H_2N(CH_2)_m—\overset{\overset{R_7}{|}}{C}(R_3)—\overset{\overset{O}{\|}}{C}—Pro$, to yield

$$R_1\underset{\underset{H}{|}}{C}(\overset{\overset{O}{\|}}{C} - S - C_6H_5) - NH - (CH_2)_m - \overset{\overset{R_7}{|}}{C}(R_3) - \overset{\overset{O}{\|}}{C} - Pro.$$

This compound is reacted with NaSH to form

$$R_1 - CH(\overset{\overset{O}{\|}}{C} - SH) - NH - (CH_2)_m - \overset{\overset{R_7}{|}}{C}(R_3) - \overset{\overset{O}{\|}}{C} - Pro.$$

Compounds of this invention in which $R_1$ and $R_2$ are bridged to form a lactone ring can be prepared using 2-halolactones, e.g., α —Br-γ-valerolactone and α —Br-γ-butyrolactone. The α-bromo group is reactive with

$$HS - (CH_2)_m - \overset{\overset{R_3}{|}}{CH} - \overset{\overset{O}{\|}}{C} - \overset{\overset{R_4}{|}}{N} - \overset{\overset{R_5}{|}}{CH} - COR_6$$

or those analogs in which an $NH_2$— or OH-group is substituted for the HS-group to form compounds in which X is —S—, —O— or —NH— in the general formula for compounds of this invention. △ Pro cannot be used in this procedure unless added as

$$—\overset{\overset{R_4}{|}}{N}—\overset{\overset{R_5}{|}}{C}—COR \text{ as a final step, i.e. after the } \underset{\underset{R_2}{|}}{\overset{\overset{R_1}{|}}{CH}}—X$$

bond has been formed. The lactone ring can be opened, e.g., with a base such as $Ba(OH)_2$ to form the corresponding γ-OH—1-carboxymethyl compounds. The hydroxy-group can be converted to a salt with sodium, potassium or an organic cation such as that from arginine, or can be converted to an ethyl or methyl ester.

Compounds $R_1—\overset{\overset{O}{|}}{C}—COOH$ or $R_3—\overset{\overset{O}{\|}}{C}—COOH$

used in any of the procedures disclosed herein may be selected from known ketocarboxylic acids, including, but not limited to,
pyruvic acid,
phenylpyruvic acid,
3-cyclohexyl-2-oxopropionic acid,
6-methyl-2-oxoheptanoic acid,
4-methyl-2-oxopentanoic acid,
2-oxobutyric acid,

3-methyl-2-oxobutyric acid,
2-oxoglutaric acid,
2-oxoadipic acid,
2-oxo-4-phenylbutyric acid,
4-(3-indolyl)-2-oxobutyric acid,
N-acetylaminoethyl-2-oxo-4-phenylbutyrate,
dimethylaminoethyl-2-oxo-4-phenylbutyrate,
2-oxo-5-methylhexanate,
phenoxypyruvic acid,
phenylthiopyruvic acid,
4-p-chlorophenyl-2-oxobutyrate,
indole-3-pyruvic acid,
2-oxo-3-p-cyanophenylpropionate,
4-α-naphthyl-2-oxobutyrate,
4-(3,4-dichlorophenyl)-2-oxo-butyrate), or
2-oxo-4-p-phenoxyphenylbutyric acid.

The compounds of this invention have one or more asymmetric carbons as indicated by the asterisks in the general formula. The compounds accordingly exist in stereoisomeric forms or in racemic mixtures thereof. All of these are within the scope of the invention. The above described syntheses can utilize a racemate or one of the enantiomers as starting material. When the racemic starting material is used in the synthetic procedure or a racemic mixture results from the synthesis, the stereoisomers obtained in the product can be separated by conventional chromatographic or fractional crystallization methods. In general the *S*-isomer with respect to the carbon bearing $R_1$ constitutes the preferred isomeric form. Also the *S*-isomer of the carbon bearing $R_5$ is preferred.

The compounds of this invention form basic salts with various inorganic and organic bases which are also within the scope of the invention. Such salts include ammonium salts, alkali metal salts like sodium and potassium salts (which are preferred), alkaline earth metal salts like the calcium and magnesium salts, salts with organic basis, e.g., dicyclohexylamine, benzathine, N-methyl-D-glucamine, procaine salts, salts with amino acids like arginine, lysine, and the like. The non-toxic, physiologically acceptable salts are preferred.

The salts are formed in conventional manner by reacting the free acid form of the product with one or more equivalents of the appropriate base providing the desired cation in a solvent or medium in which the salt is insoluble, or in water and removing the water by freeze drying. By neutralizing the salt with an insoluble acid like a cation exchange resin in the hydrogen form (e.g., polystyrene sulfonic acid resin like Dowex 50) or with aqueous acid and extraction with an organic solvent, e.g., ethyl acetate, dichloromethane or the like, the free acid form can be obtained, and, if desired, another salt formed.

Additional experimental details are found in the examples which are preferred embodiments and also serve as models for the preparation of other members of the group.

The compounds of this invention inhibit the conversion of the decapeptide angiotensin I to angiotensin II and therefore are useful in reducing or relieving angiotensin related hypertension. The action of the enzyme renin on angiotensinogen, a pseudoglobulin in blood plasma, produces angiotensin I. Angiotensin I is converted by angiotensin converting enzyme (ACE) to angiotensin II. The latter is an active pressor substance which has been implicated as the causative agent in various forms of hypertension in various mammalian species, e.g., rats and dogs. The compounds of this invention intervene in the angiotensin $\xrightarrow{(renin)}$ angiotensin I $\xrightarrow{(ACE)}$ angiotensin II sequence by inhibiting angiotensin converting enzyme and reducing or eliminating the formation of the pressor substance angiotensin II. Thus the adminstration of a composition containing one or a combination of compounds of formula I including their physiologically acceptable salts, angiotensin-dependent hypertension in the species of mammal suffering therefrom is alleviated. A single dose, or in some cases up to two to four divided daily doses, provided on a basis of about 0.03 to 20 mg per kilogram per day, is appropriate to reduce blood pressure. The substance is preferably administered orally, but parenteral routes such as subcutaneous, intra-muscular, intravenous or intraperitoneal can also be employed.

The compounds of this invention can be utilized to achieve the reduction of blood pressure by formulating them in compositions such as tablets, capsules or elixirs for oral administration or in sterile solutions or suspensions for parenteral administration. About 10 to 500 mg of a compound or mixture of compounds of formula I, including the physiologically acceptable salts thereof, is compounded with a physiologically acceptable vehicle, carrier, excipient, binder preservative stabilizer, flavor, etc., in a unit dosage form as called for by acepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, gum acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil.

Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor. Anti-oxidants may also be added. Suitable antioxidants are α-tocopherol nicotinate, vitamin A, C, E and analogs of vitamin E known in the art, retinal palmitate and other antioxidants known in the art as food additives such as the gallates.

Sterile compositions for injection can be formulated according to conventional pharmaceutical practice by dissolving or suspending the active substance in a vehicle such as water, a naturally occurring vegetable oil like sesame oil, coconut oil, peanut oil, cottonseed oil, etc., or a synthetic fatty vehicle like ethyl oleate or the like. Buffers, preservatives, and the like can be incorporated as required.

The present invention will be further described by the following examples. All temperatures are in degrees Celsius unless otherwise indicated. Molar equivalents of the reactants as usually utilized.

Example 1

Synthesis of 3-N-[1-carboxy-2-(para-ethylaminocarbonyl phenyl)ethyl]-aminopropanoyl-L-5-ketoproline

A. 20 mmoles of 3-N-(benzyloxycarbonyl)-aminopropanoic acid is dissolved in $CH_2Cl_2$ at 0°C. 20 mmoles of N-hydroxysuccinimide is added and then 20 mmoles of DCC is added dropwise to this mixture. The reaction mixture is stirred for 30 minutes at 0°C and then overnight at 4°C. Crystalline dicyclohexylurea is removed by filtration. The solvent from the filtrate is removed under reduced pressure. The resulting product is dissolved in cold THF and then the solution is added to a cold solution of 20 mmoles of L-glutamic acid and 40 mmoles of $NaHCO_3$ in THF/water. The reaction mixture is stirred overnight at room temperature and then the THF is removed with a rotary evaporator. The glutamyl residue is cyclized to give the L-5-keto-proline residue according to Gibian H. and Klieger E., *Justus Liebig's Ann. Chemie 640*, 145 (1961). The benzyloxycarbonyl group is removed by treatment with hydrogenolysis.

B. A solution of 10 mmoles of this product and 50 mmoles of 2-keto-3-(4-ethylaminocarbonyl-phenyl) propionic tert butyl ester in ethanol is stirred with powdered molecular sieves at room temperature for 1/2 hour. A solution of 40 mmoles of sodium cyanoborohydride in ethanol is slowly added over the course of six hours. The reaction mixture is filtered. The t-butyl ester is removed by treatment with trifluoroacetic acid in anisole. The named product is obtained after removal of the solvent with a rotary evaporator.

Example 2

Synthesis of Np[L-1-carboxyl (2-propylaminocarbonylethyl)-1-carboxyethyl]-D,L-Ala-L-Pro

A. 200 mmoles of propylamine and 150 mmoles of the α-ethyl ester of N -Boc aspartic acid are dissolved in 600 ml of cold dimethyl formamide and 125 mmoles of DPPA. A volume of 25 ml of triethylamine in DMF is added drop-wise, holding the temperature at about −10°C for two hours. The reaction is stored overnight at room temperature and rotary evaporated to remove DMF. The Boc group is removed with TFA. The product is 3-(propylaminocarbonyl)-1-aminopropanoic acid ethyl ester.

B. A solution of 60 mmoles of pyruvic acid plus 60 mmoles of L-proline ethyl ester in redistilled chloroform is cooled to −50°C in an acetone-dry ice bath. To this solution is added 60 mmoles of a precooled solution of dicyclohexylcarbodiimide (DCC) is chloroform and the mixture is stirred at −5°C for 1 hour. The reaction mixture is slowly warmed to room temperature and stirred for an additional 2 hours and then stirred at 4°C overnight. The mixture is filtered to remove dicyclohexylurea, then cooled in an ice bath. The organic phase is washed with cold water, cold 1N $NaHCO_3$ and finally with cold saturated NaCl. The organic phase is dried over anhydrous $MgSO_4$ and filtered. The solvent is removed with a rotary evaporator yielding N-pyruvoyl-L-proline ethyl ester.

C. 40 mmoles of the product of Step A is reacted with 200 mmoles of the product of Step B in ethanol with stirring in the presence of molecular sieves at room temperature. A solution of 40 mmoles of sodium cyanoborohydride in ethanol is then slowly added over the course of 6 hours. The reaction mixture is filtered and the solvent removed by a rotary evaporator. The product is purified by partition chromatography (Sephadex G—25), developed with butanol/acetic acid/$H_2O$ (4:1:5). The ethyl esters are removed by saponification to yield the named product.

Example 3—16

By substituting any one of the reactants for propylamine of Example 2, and following the procedures of Example 2, compounds are obtained with $R_1$ groups as shown in the Table.

TABLE

| Example | Reactant | $R_1$ |
|---|---|---|
| 3 | butylamine | 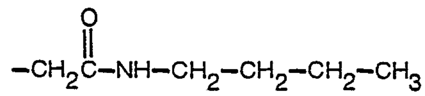 |
| 4 | cyclopentylamine | 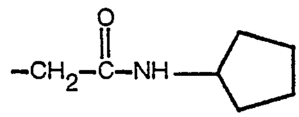 |
| 5 | L-proline | 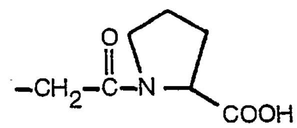 |
| 6 | adenine | 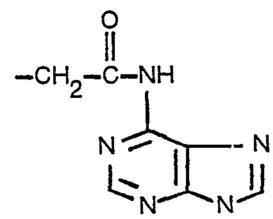 |
| 7 | indoline | 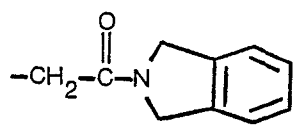 |
| 8 | 2-azacyclooctanone | 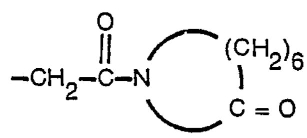 |
| 9 | aniline | 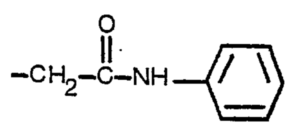 |
| 10 | 2-amino-5-bromo-pyridine | 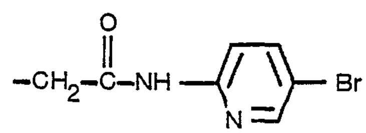 |
| 11 | 2-amino-5-chlorothiazole | 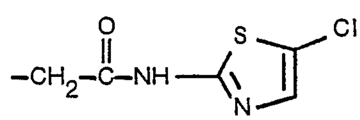 |
| 12 | N-amino piperidine | 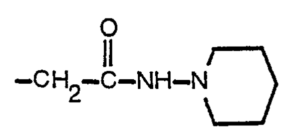 |
| 13 | isobutyramide | 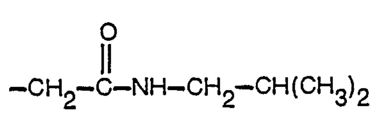 |

TABLE (Continued)

| Example | Reactant | $R_1$ |
|---|---|---|
| 14 | maleimide | 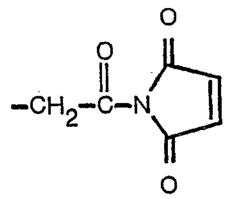 |
| 15 | diacetamide | 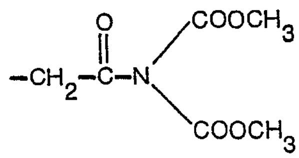 |
| 16 | diallylamine | $-CH_2-\overset{O}{\overset{\|}{C}}-N-(CH_2CH=CH_2)_2$ |

Example 17

Synthesis of N-[3-(2-butylaminocarbonyl-1-carboxy-ethyl thio)-2-D-methylpropanoyl]-L-proline

A. 0.25 mmoles of 3-mercapto-2-D-methylpropanoyl-L-proline ethyl ester, 0.28 mmoles of 2-bromo-succinic acid monoethylester (esterified at Cl) and 0.16 mmole of $K_2CO_3$ were added to 0.6 ml of a 50:50 mixture of absolute ethanol and water. The suspension was stirred overnight at room temperature. 0.25 mmoles of $K_2CO_3$ in 0.15 ml of water was then added and the reaction mixture was stirred for an additional 24 hours. This mixture was then acidified to pH 2.0 with HCl and the product was extracted with ethyl acetate. The organic phase was washed with saturated NaCl. The product appeared to be pure and behaved as a single substance on thin layer chromatography in two separate solvent systems. The ethyl acetate phase was dried over anhydrous $MgSO_4$ and the solvent was removed with a rotary evaporator to yield the N-[3-(1-carboxy-2-ethoxycarbonyl)-2-D-methylpropanoyl]-L-proline ethyl ester as a colorless oil.

B. A quantity of 0.15 mmoles of butylamine and 0.15 mmoles of the product of Synthesis A are dissolved in 0.6 ml of cold DMF and 0.05 ml DPPA. A volume of 0.025 ml of triethylamine in DMF is added, holding the temperature at about −10°C for 2 hours. The reaction is stored overnight at room temperature, rotary evaporated to remove DMF, then the residue is partitioned between water and ethyl acetate. The organic layer is chromatographed to obtain the named product.

Example 18

Synthesis of N-[1-carbethoxy-3-(methylaminocarbonyl) propyl]-L-Ala-L-Pro

A solution of 50 mmoles of 4-methylaminocarbonyl-2-oxo-butyric acid ethyl ester and 10 mmoles of L-Ala-L-Pro butyl ester in ethanol is stirred with powdered molecular sieves at room temperature for 30 minutes. A solution of sodium cyanoborohydride, 10 mmoles, in ethanol is added slowly over the next 5 hours. The mixture is filtered, and the solvent of the filtrate is removed with a rotary evaporator and deprotected by treatment with TFA. The product, N-[1-carbethoxy-3-methylaminocarbonyl-propyl]-alanyl-proline, is obtained after partition column chromatography [butanol/acetic acid/$H_2O$ (4:1:5 by vol.)].

Example 19

Synthesis of N-[(1-carboxy-3-carboanilide)propyl]-δ-propylamino-L-glutamyl-L-proline

A. 175 mmoles of propylamine and 150 mmoles of the α-ethyl ester of Boc-glutamic acid are dissolved in 600 ml of DMF and 125 mmoles of DPPA. A volume of 25 ml of triethylamine is added drop-wise, holding the temperature at about −10°C for 2.5 hours. The reaction is stored overnight at room temperature, rotary evaporated to remove DMF, then the product, δ-propylamido-L-Boc-glutamic acid ethyl ester, is saponified and then purified by chromatography on silica gel.

B. 100 mmoles of the product is then reacted with 100 mmoles of L-proline-t-butyl ester in redistilled dichloromethane, precooled to −5°C. To this solution is added 100 mmoles of a precooled solution of dicyclohexylcarbodiimide in dichloromethane and the mixture is stirred in an ice bath for 2 hours. The reaction mixture is slowly warmed to room temperature and then stirred at 4°C overnight. The mixture is filtered to remove dicyclohexylurea, then cooled in an ice bath. The organic phase is washed with cold 1N HCl, cold 1N $NaHCO_3$ and finally with cold saturated NaCl. The organic phase is dried over an anhydrous

$MgSO_4$ and filtered. The solvent is removed with a rotary evaporator yielding δ-propylamino-Boc-L-glutamyl-L-proline-t-butyl ester. The t-butyl ester and Boc groups are removed with TFA.

C. 50 mmoles of the product of Synthesis A of Example 19 and 250 mmoles of the product of Synthesis B of Example 19 are condensed in the presence of 50 mmoles sodium cyanoborohydride by the method described in Synthesis B of Example 1 plus 300 mmoles of $NaHCO_3$ in water to yield an esterified compound.

Examples 20—26

By substituting propylamine and the 1-ethyl ester of Boc-glutamic acid in Synthesis A of Example 19 with each pair of reactant compounds in the Table, then reacting the product with L-proline-tert-butyl ester according to Synthesis B of Example 19, and finally reacting the resulting compound with 4-carboanilide-2-ketobutyric acid ethyl ester according to Synthesis B of Example 1, a series of analogs of the product of Example 19 are obtained, which products have $R_3$ and $R_7$ groups given in the Table.

TABLE

| Example | Pair of Reactants | $R_7$ | $R_3$ |
|---|---|---|---|
| 20 | methylamine, 1-ethyl ester of Boc-glutamic acid | H | 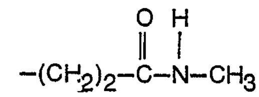 |
| 21 | methylamine, 1-ethyl ester of Boc-2-amino-malonic acid | H | 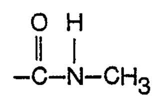 |
| 22 | methylamine, 1-ethyl ester of Boc-aspartic acid | H | 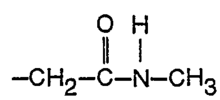 |
| 23 | aniline, 1-ethyl ester of Boc-2-amino adipic acid | H | 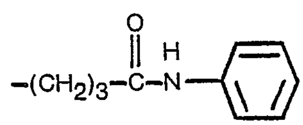 |
| 24 | aniline 1-ethyl ester of p-carboxy-Boc-phenylglycine | H | 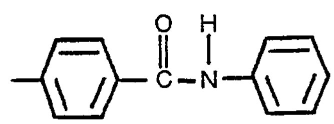 |
| 25 | propylamine, 1-ethyl ester of p-carboxy-Boc-phenylalanine | H | 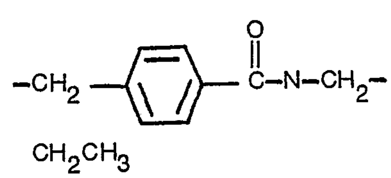 |
| 26 | aniline, 1-ethyl ester of methyl-Boc-aspartic acid | | $-CH_3$, 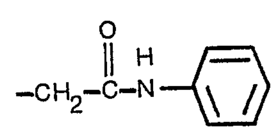 |

Example 27

Preparation of N-[L-1-benzyloxycarbonyl-3-(carbo-4-iodoanilide)propyl]-Alanyl-L-Proline

*A. Synthesis of L-glutamic acid-α-benzyl ester-δ-4-iodoanilide*

A solution of 4 mmoles of $N^{\alpha}$-Boc-L-Glu-δ-2-$NO_2$-phenyl ester-α-benzyl ester in 3 ml of $CH_2Cl_2$ was added to a solution of 4.1 mmoles of 4-iodo-aniline in 3 ml of $CH_2Cl_2$ and the resulting solution was stirred at room temperature overnight. (The reaction was judged to be complete by thin layer chromatography). An oily residue was obtained after work-up. The product was dissolved in 4 ml of anhydrous trifluoroacetic acid. After 45 minutes at room temperature, the solvent was removed by rotary evaporation at 40°C. White crystals were formed after the addition of 4.5 ml M HCl in ethyl acetate. The mixture was left at 0°C for one hour and was filtered. The precipitate was washed with cold ethyl acetate in ether and then dried over $P_2O_5$ and NaOH in a vacuum desiccator. Yield 0.79 g; d.p. 119—120°C; second crop yield 1.11 g; d.p. 119—120.5°C. The material was recrystallized from $CHCl_3$/isopropyl ether; d.p. 119.5—120.5°C. Elemental analysis for $C_{18}H_{10}N_2IClO_3$: Calculated C 45.54; H 4.24; N 5.90; I 26.73 Cl 7.47; O 10.11. Found: C 45.55; H 4.19; N 5.92; I 26.53; Cl 7.34.

*B. Synthesis of N-L-1-benzyloxycarbonyl-3-(carbo-4-iodoanilide)propyl]-Ala-L-Pro-t-butyl ester*

A solution of the product of A (1 mmole in 1 ml of ethanol) is added with stirring to 1 mmole of $NaHCO_3$ in 0.2 ml of $H_2O$. To the resulting solution was added 5 mmoles of N-pyruvoyl-L-proline-t-butyl ester in 2 ml of ethanol plus 1.6 g of molecular sieves. The mixture was stirred for 30 minutes at room temperature. Sodium cyanoborohydride, 65 mg in 1.5 ml of ethanol, was added, drop-wise, over a period of 4 hours. The reaction mixture was left at room temperature overnight. The mixture was filtered, the filtrate saved, and the precipitate was washed several times with ethanol. Solvent of the combined filtrates was removed by rotary evaporation at 30°C to yield a yellow oil. The crude product was purified on Sephadex LH—20 (2.22 × 99 cm column) developed with THF/isopropanol (3:7 by vol.); 250 drops (5.8 ml/fraction). Fractions 33—35 contained the desired product.

C. The named compound of this Example was obtained by dissolving the desired product of B in 2 ml of anhydrous TFA. The solution was allowed to stand at room temperature for 30 minutes and then the trifluoroacetic acid was removed by rotary evaporation at 30°C. The residue was dissolved in a small amount of ethanol, and the solution was applied to a column (1.2 × 43 cm) of AG1—X2 ($OH^-$ form) in $H_2O$. The column was developed with $H_2O$, 62 ml, and then a linear gradient was developed between $H_2O$ and 0.5M ammonium acetate (2 liters total). The column was washed with 0.5M ammonium acetate (1 liter), 1.0M ammonium acetate (200 ml) and then with 1.0M ammonium acetate/ethanol (1:2 by vol). The desired product was eluted with the last-named solution. Solvent volume was reduced by rotary evaporation and then ammonium acetate was removed by lyophilization and sublimation.

Example 28

Preparation of N-[L-1-carboxy-3-(carbo-anilide) propyl]-Alanyl-L-Proline

The product of Example 27, 40 mg, in 3 ml of methanol, was reacted with 20 mg of 10% palladium on carbon and $H_2$ at 1 atmosphere for 3 hours at room temperature. The precipitate was removed by filtration, and the solvent of the filtrate was removed by rotary evaporation. The desired product was obtained by chromatography on Sephadex G—10 (1.2 × 96 cm column) developed and eluted with 2% pyridine in water (yield 12.1 mg).

Example 29

Preparation of N-[L-1-carboxy-3-(carbo-4-iodo-anilide) propyl] Alanyl-L-Proline

The product of Example 27, 60 mg, was treated with 3 ml of anhydrous HF in the presence of anisole for 1 hr. The desired product was obtained by the chromatographic system of Example 28. Yield 22.15 mg.

Example 30

Preparation of N-[L-1-carboxy-2-(carbopyrrolide)ethyl]-Alanyl-L-Proline

*A. Synthesis of L-aspartic acid-β-pyrrolide-α-ethyl ester*

$N^{\alpha}$-Cbo-L-aspartic acid-α-ethyl ester, 8 mmoles, in 5 ml of $CH_2Cl_2$ was cooled to −5°C. A cold solution of DCC, 8 mmoles in 3 ml of $CH_2Cl_2$ was added with stirring. To this solution was added 0.67 ml of pyrrolidine. Stirring was continued at −5°C for 30 minutes and at 4°C overnight. The mixture was filtered, and the precipitate was washed with ethyl acetate. The combined filtrate was washed until neutral. The organic phase was dried over $MgSO_4$ and then filtered. The solvent of the filtrate was removed under vacuum to yield 1.85 of a yellow oil. The oil, 1.5 g, was dissolved in 20 ml of methanol and the Cbo-protecting group was removed by hydrogenolysis (150 mg of 10% palladium on carbon with $H_2$ at 10 pounds per square inch for 90 minutes). The mixture was filtered, and solvent was removed under vacuum to yield white crystals. Recrystallization was effected from methanol/isopropyl ether. The desired product remained in the mother liquid and was converted to its HCl salt by adding HCl in ethyl acetate. Solvent was removed and the residue was dried over $P_2O_5$ and KOH in a vacuum desiccator to yield a hydroscopic foam. Crystals, 0.47 g, were obtained from $CHCl_3$/ethyl acetate.

*B. Alkylation of pyruvoyl-L-proline with the product of A (Example 30)*

Molecular sieves (1.312 g) were added with stirring to a mixture of 0.206 g of HCl·L-Asp-$^{\beta}$-pyrrolide-α-ethyl ester, 0.073 g of $NaHCO_3$ and 0.986 g of N-pyruvoyl-L-proline-t-butyl ester in 0.1 ml of $H_2O$ and 2.0 ml of ethanol at room temperature. The mixture was stirred for 30 minutes and then 0.054 g of sodium cyanoborohydride in 1.0 ml of ethanol was added drop-wise over a period of 4 hours. Stirring was continued for another 18 hours. The mixture was filtered and the precipitate was washed with ethanol. Solvent of the combined filtrates was removed by rotary evaporation to yield a yellow oil. The material was chromatographed on LH—20 (2.2 × 99 cm) developed with THF/isopropanol (3:7 by vol). The residue obtained by rotary evaporation was dissolved in 1.2 ml of TFA. After 45 minutes at room temperature, TFA was removed and the material was purified by chromatography on AG1—X2 (1.2 × 38 cm) developed first with $H_2O$ and then with a linear gradient between $H_2O$ and 0.5M ammonium acetate. Apparently pure product, 31.5 mg, was obtained by chromatography on Sephadex G—10 (1.2 c 97 cm column) developed with 2% pyridine. The ethoxy group was removed by saponification.

Example 31

In vitro assays of the potency of selected compounds as inhibitors of angiotensin converting enzyme

Compounds of this invention were assayed through the following protocol: 25 microliters of buffer (0.05M Hepes buffer, pH 8.0, plus 0.1M NaCl and 0.75M $Na_2SO_4$) or 25 microliters of an inhibitor in buffer was added to the bottom of a 7 ml liquid scintillation vial. To this was added 100 microliters of buffered substrate [S], [$^3$H]benzoyl-Gly-His-Leu, 80 nM (25 Ci/mmole). The reaction was started by adding 100 microliters of partially-purified human plasma angiotensin converting enzyme, or 100 microliters of buffer alone. The concentration of enzyme [E] used was that required to hydrolyze 8—12% of substrate when incubated at 37°C for 15 minutes. The scintillation vials and their contents were incubated at 37°C for 15 minutes, and the reactions were stopped by adding 200 microliters of 0.5M HCl to each vial. The radioactive reaction product, [$^3$H]benzoyl-Gly(hippuric acid), was separated from unhydrolyzed substrate by adding and mixing (by inversion) 3 ml of Ventrex Cocktail No. 1 (Ventrex Laboratories, Inc., Portland, Maine), a fluid disclosed in copending U.S. patent application No. 184,653, filed September 6, 1980. Extractable $^3$H was quantified by liquid scintillation counting. Substrate, in c.p.m., was quantified by scintillation counting of a vial containing 100 μl of buffered substrate in 5 ml of RIAfluor (New England Nuclear). The reaction mixture containing all constituents except for inhibitor was termed the control (C). The reaction mixture lacking enzyme and inhibitor was called the blank (B). Reaction mixtures containing inhibitor (varied over the range of $10^{-4}$—$10^{-12}$M) were called the test (T) reactions. Under the conditions of this assay, the reaction obeys first order enzyme kinetics, thus the concentration of inhibitor required to inhibit the rate of hydrolysis by half ($I_{50}$) approximate the Ki value. The results were estimated by use of the formula:

$$[E] = \frac{C - B}{[S]} \times 100 \times \frac{1}{15 \text{ min}}$$

where C = control c.p.m.; B = blank c.p.m.; [S] = substrate c.p.m. The factor 100 converts fractional substrate utilization into percentage utilization, and 1/15 minute connects to percentage substrate utilization/minute. Thus, [E] is enzyme activity in percentage substrate utilization/minute. By substituting T for C, hydrolysis rates are computed for the test reaction mixtures. By comparing a given test rate against the control rate, the degree of inhibition can be computed.

| Compound Product of Example No. | $I_{50}$ |
|---|---|
| 28 | $5.2 \times 10^{-9}$M |
| 29 | $2.3 \times 10^{-10}$M |
| 30 | $1.4 \times 10^{-7}$M |

Example 32

Intravenous effectiveness of N-[L-1-carboxy-3-(carbo-4-iodoanilide)propyl]-D,L-Ala-L-Pro

Rats (190—290 g body weight) were fasted overnight and then anesthetized with intraperitoneal pentobarbital, 50—60 mg/kg. Tracheostomy was performed and the animals were ventilated mechanically. A cannula was inserted into a femoral vein for injection of angiotensin I, and a second cannula was inserted into a common carotid artery for direct measurement of arterial blood pressure. Heparin, 1,000 units, was injected via the femoral vein to prevent coagulation. Blood pressure was measured with a pressure transducer connected to a polygraph. The rats were injected with 400 mg/kg of angiotensin I in 20 μl of 0.9 g% NaCl, an amount of angiotensin I sufficient to raise mean arterial blood pressure by approximately 48 mm Hg.

After the responsiveness of a given rat to angiotensin I was established, the named compound at 0.5 micromole/kg (drug dissolved in 15 microliters of 0.9% NaCl) was given intravenously. At timed intervals, the effects of 400 ng/kg of angiotensin I on mean arterial blood pressure were tested. Results are shown below:

| Time after IV Administration (minutes) | Blood Pressure Reponse to 400 ng/kg of Angiotensin I (% of Control) |
|---|---|
| −5 | 100% (48 mm Hg.) |
| +1 | 33% |
| 5 | 42% |
| 10 | 46% |
| 15 | 52% |
| 20 | 60% |
| 25 | 67% |
| 30 | 71% |
| 35 | 71% |
| 50 | 83% |
| 60 | 92% |
| 70 | 100% |
| 80 min. | 104% |

Example 33

"Intravenous Effectiveness of N-[L-1-carboxy-3-(carboanilide)propyl]-D,L-Ala-L-Pro"

Experiments were carried out using rats according to Example 32. The results are shown below:

| Time After IV Administration (minutes) | Blood Pressure Reponse to 400 ng/kg of Angiotensin I (% of Control) |
|---|---|
| −5 | 100% (48 mm Hg.) |
| +1 | 42% |
| 5 | 37% |
| 10 | 37% |
| 15 | 40% |
| 20 | 40% |
| 25 | 40% |
| 30 | 35% |
| 40 | 35% |
| 50 | 46% |
| 60 | 46% |
| 70 | 48% |
| 80 | 52% |
| 90 | 54% |
| 102 min. | 62.5% |

Examples 34—52

By substituting pyruvic acid in Synthesis B of Example 2 with any of the α-keto carboxylic acids (appropriately protected) in the Table, and reacting the product with 3-(propyl amino-carbonyl)-2-amino-propanoic acid ethyl ester as in Synthesis A of Example 2, products with $R_3$ groups in the Table are formed.

TABLE

| Example | α-keto carboxylic acid | $R_3$ |
|---|---|---|
| 34 | pyruvic acid | $CH_3-$ |
| 35 | phenylpyruvic acid | 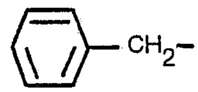 |
| 36 | 3-cyclohexyl-2-oxo-propionic acid | 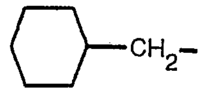 |
| 37 | 6-methyl-2-oxo-heptanoic acid | 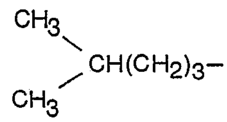 |
| 38 | 4-methyl-2-oxo-pentanoic acid | 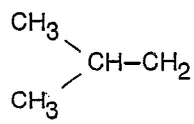 |
| 39 | 2-oxo-butyric acid | $CH_3CH_2$ |
| 40 | 3-methyl-2-oxo-butyric acid | 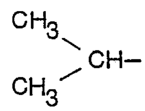 |
| 41 | 2-oxo-glutaric acid ethyl ester | $EtOOC-CH_2-CH_2-$ |
| 42 | 2-oxo-adipic acid ethyl ester | $EtOOC-CH_2CH_2CH_2-$ |
| 43 | 2-oxo-4-phenyl butyric acid | 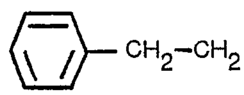 |
| 44 | 4-(3-indolyl)-2-oxo-butyric acid | 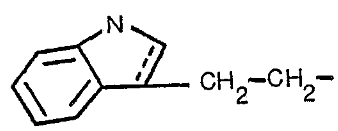 |
| 45 | phenoxypyruvic acid | 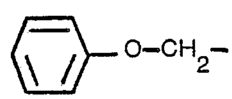 |
| 46 | phenylthio pyruvic acid | 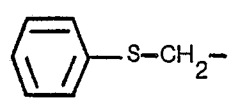 |
| 47 | 4-p-chlorophenyl-2-oxobutyric acid | 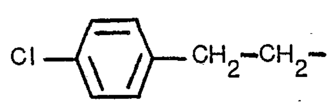 |

TABLE (Continued)

| Example | α-keto carboxylic acid | $R_3$ |
|---|---|---|
| 48 | indole-3-pyruvic acid | 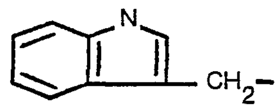 |
| 49 | 2-oxo-3-p-cyanophenyl-propionic acid | 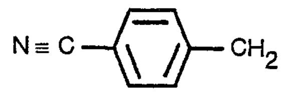 |
| 50 | 4-alpha-naphthyl-2-oxo-butyric acid | 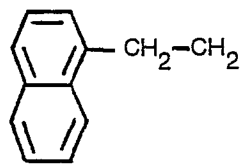 |
| 51 | 4-(3,4-dichlorophenyl)-2-oxo-butyric acid | 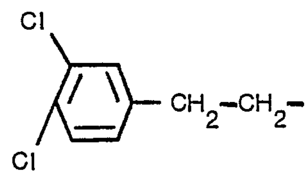 |
| 52 | 2-oxo-4-p-phenoxy-phenyl butyric acid | 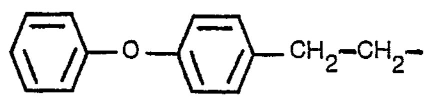 |

Example 53

Synthesis of 2-keto-butyryl-L-<Glu

L-pyroglutamic acid (35 mmoles) is suspended in a mixture of 35 ml of propylene oxide and 210 ml of dry acetonitrile at room temperature. Bis-trimethylsilyltri-fluoro acetamide (77 mmole) is added and the stopped reaction is stirred at room temperature for 15 minutes. 2-keto-butyric acid mixed carbonic anhydride (prepared by 2-keto-butyric acid, 36.8 mmole, triethylamine in isobutyl chloro formate) is added and the reaction is stirred at room temperature overnight. Acetonitrile is then removed *in vacuo* and the resulting residue is dissolved in ethyl acetate. The organic phase is washed with $H_2O$, then saturated NaCl, dried over anhydrous $Na_2SO_4$ and filtered, and the solvent removed with a rotary evaporator.

*B. Synthesis of N-2-(1-anilinocarbonyl-2-benzylthioethyl)butyryl-L-pyroglutamic acid*

Nα-Boc-S-benzyl-D-cysteine 100 mmoles is reacted with an equivalent of aniline in the presence of a slight excess of one equivalent of DCC (105 mmoles) by the method described in Synthesis A of Example 27. The Boc group is then removed with anhydrous TFA. 50 mmoles of the product is then coupled to 10 mmoles 2-keto-butyryl-L-<Glu(Synthesis A) with 10 mmoles of cyanoborohydride according to Example 18 to yield the named compound.

Example 54

Synthesis of N-2-(1-anilinocarbonyl-2-benzylthioethyl)propanoyl-L-pro

By substituting pyruvoyl-L-pro (Example 2) for 2-keto-butyryl-L-<Glu (Synthesis A of Example 53), and following the procedure of Synthesis B of Example 53, the named product is obtained.

Example 55

Synthesis of N-[-1-(N-acetylaminoethoxy carbonyl)-3-(carboanilide) propyl]-D,L-Ala-L-Pro-ethyl ester

A solution of 50 mmoles of 2-Boc-amino-4-carboxy butyric acid N-acetyl aminoethyl ester is coupled to

50 mmoles of aniline in the presence of an equivalent of DCC according to Synthesis A of Example 27. The Boc group is then removed with anhydrous TFA. 40 mmoles of the product is then reacted with 200 mmoles of N-pyruvoyl-L-Pro-ethyl ester, then 42 mmoles of sodium cyanoborohydride in ethanol is slowly added over the course of 6 hours. The reaction mixture is filtered and the solvent removed by a rotary evaporator, yielding the named compound.

Example 56

Synthesis of N-[L-1-(dimethylaminoethoxycarbonyl)-3-(carbonylmethylamino)propyl]-D.L-Ala-L-Pro ethylester

By substituting 2-amino-4-carboxy butyric acid dimethyl aminoethyl ester for 2-amino-4-carboxy butyric acid N-acetylaminoethyl ester of Example 55, the named compound is synthesized.

Example 57

A. By substituting L-proline-tert butyl ester for L-proline ethylester in Synthesis B of Example 2, the procedure of Synthesis B of Example 2 yields the pyruvoyl-L-Pro-tertbutyl ester.

The tert butyl ester can be removed by treatment with TFA in anisole.

B. A solution of 10 mmoles of $N^{\alpha}$-Boc-S-benzyl-D-cysteine-α-$NO_2$-phenyl ester in 3 ml of $CH_2Cl_2$ is added to a solution of 10.5 mmoles of any of the amine or imine compounds (listed in Table I, below) in 3 ml of $CH_2Cl_2$, and the resulting solution is stirred overnight at room temperature. The reaction is judged to be complete by thin layer chromatography. The resulting mixture is dissolved in 4 ml of TFA to remove the Boc blocking group, rotary evaporated and crystallized, to yield α-amides and α-imides of S-benzyl-D-cysteine as the product.

TABLE I: AMINE AND IMINE COMPOUNDS

aniline
benzylamine
methylamine
ethylamine
1-aminopropane
2-aminopropane
2-aminobutane
1-amino-2-butanone
t-butylamine
cyclopentylamine
cyclohexylamine
ε-aminocaproic acid benzyl ester
ε-aminocaproamide
3-amino-2-methyl-propionic acid ethyl ester
2-amino-propionic acid ethyl ester
glycine-t-butyl ester
valine-benzyl ester
p-OH-aniline
p-OH-m-iodo-aniline
p-carboxy-thienyl ester of aniline
m-F-benzylamine
4-OH-3,3′-Br-benzylamine
4-Cl-benzylamine
3,4-dichloro-benzylamine
3-$NO_2$-benzylamine
3-phenylpropylamine
2-indolylethylamine
2-amino-pyridine
adenine
Cytidine
pyrroline
4-phenylbutylamine
α-methyl-alanine ethyl ester
3-hydroxy-propylamine
3-Boc-amino-propylamine
1-amino-3-hydroxy-butane
1-adamantanamine
2-adamantanamine
1-adamantanemethyl amine

TABLE I: AMINE AND IMINE COMPOUNDS (continued)

$N^{\varepsilon}$-Boc-lysine-ethyl ester
$N^{\alpha}$-Boc-lystine-t-butyl ester
$N^{\delta}$ hydroxy-arginine-ethyl ester
$N^{\delta}$ methyl-homoarginine-t-butyl ester
$N^{im}$-benzyl-histidine-t-butyl ester
leucine-t-butyl ester
isoleucine-t-butyl ester
norvaline-ethyl ester
norleucine-methyl ester
glycine-p-methyl benzyl ester
α-methyl-alanine-diphenylmethyl ester
glycyl-benzylamide
α-methyl-alanyl-4-OH-benzylamide
$N^{im}$-benzyl-histidinyl-3-iodo-anilide
glycyl-pyrrolide
glycyl-1-adamantanamide
glutamine-ethyl ester
asparagine-t-butyl ester
α-methyl-valine-t-butyl ester
α-methyl-phenylalanine-t-butyl ester
tyrosine-t-butyl ester
O-benzyl-tyrosine-t-butyl ester
4-iodo-phenylalanine ethyl ester
3,5-dibromotyrosine-ethyl ester
thyronine-ethyl ester
vinyl glycine ethyl ester
β-fluoro-alanine ethyl ester
serine ethyl ester
threonine t-butyl ester
O-t-butyl-threonine-t-butyl ester
O-t-butyl-serine-ethyl ester
O-benzyl-serine-ethyl ester
O-methyl-serine-methyl ester
O-ethyl-serine-ethyl ester
S-ethyl-cysteine-ethyl ester
S-t-butyl-cysteine-t-butyl ester
S-benzyl-cysteine-t-butyl ester
S-benzyl-homocysteine-t-butyl ester
S-methyl-homocysteine ethyl ester
S-ethyl-homocysteine-ethyl ester
S-t-butyl-homocysteine-t-butyl ester
O-t-butyl-homoserine-t-butyl ester
O-benzyl-homoserine-benzyl ester
O-methyl-homoserine-methyl ester
O-ethyl-homoserine-ethyl ester
O-phenyl-homoserine-ethyl ester
O-phenyl-serine-ethyl ester
S-phenyl-cysteine-ethyl ester
β-fluoro-phenylalanine ethyl ester
β-OH-phenylalanine-methyl ester
β-Br-alanine-methyl ester
β-thienylserine-t-butyl ester
3,5-dimethyl-tyrosine-t-butyl ester
β-hydroxynorvaline ethyl ester
β-benzyloxynorvaline-ethyl ester
$N^{\varepsilon}$-Boc-hydroxylysine t-butyl ester
3-Boc-amino-tyrosine-ethyl ester
α-methyl-phenylalanine-ethyl ester
t-leucine methyl ester
α-methyl glutamine methyl ester
$N^{\varepsilon}$-hydroxylysine t-butyl ester
β-N-methyl-lysine-methyl ester

TABLE I: AMINE AND IMINE COMPOUNDS (continued)

| |
|---|
| 5,5'-dihydroxy-leucine-ethyl ester |
| β-fluoro-asparagine-ethyl ester |
| β-methyl-asparagine-ethyl ester |
| γ-N-methyl-lysine-methyl ester |
| β-methyl-β-benzylamido-aspartic acid-β-ethyl ester |
| 2-ethoxy-5-$NO_2$-phenylalanine ethyl ester |
| β-ethoxy-phenylalanine-t-butyl ester |
| α-methyl-serine-t-butyl ester |
| O-benzyl-α-methyl-serine-t-butyl ester |
| O-benzyl-α-methyl-serine-t-butyl ester |

C. Any of the α-amides or α-imides of S-benzyl-D-cysteine of Synthesis B of this example are then used to alkylate any of the α-keto carboxylic acids in Table II, immediately below. A quantity of 5 mmoles of any of the α-amides or α-imides of D-cysteine-α-benzyl ester of Synthesis B is dissolved in 1 ml of ethanol and added with stirring to 5 mmoles of $NaHCO_3$ in 0.2 ml $H_2O$. To the resulting solution is added 25 mmoles of any α-keto carboxylic acid of Table II in 2 ml of ethanol plus 1.6 g of molecular sieves. The mixture is stirred for 1 hour at room temperature, then 5 mmoles of sodium cyanoborohydride, in 1.5 ml of ethanol, is added drop-wise over a period of 4 hrs. The reaction mixture is left at room temperature overnight. After filtration, solvent is removed from the filtrate, and the product purified by column chromatography. The products are compounds of the formula:

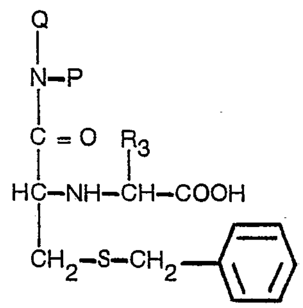

TABLE II: α KETO CARBOXYLIC ACIDS

| |
|---|
| pyruvic acid |
| phenylpyruvic acid |
| 3-cyclohexyl-2-oxopropionic acid (cyclohexylpyruvic acid) |
| 6-methyl-2-oxoheptanoic acid |
| 4-methyl-2-oxopentanoic acid |
| 2-oxobutyric acid |
| 3-methyl-2-oxobutyric acid |
| 2-oxoglutanic acid |
| 2-oxoadipic acid |
| 2-oxo-4-phenylbutyric acid (and its t-butyl ester) |
| 4-(3-indolyl)-2-oxobutyric acid |
| N-acetylaminoethyl-2-oxo-4-phenylbutyrate |
| dimethylaminoethyl-2-oxo-4-phenylbutyrate |
| 2-oxo-5-methylhexanoic acid |
| phenoxypyruvic acid |
| phenylthiopyruvic acid |
| 4-p-chlorophenyl-2-oxobutyric acid |
| indole-3-pyruvic acid |
| 2-oxo-3-p-cyanophenylpropionic acid |
| 4-α-naphthyl-2-oxobutyric acid |
| 4-(3,4-dichlorophenyl)-2-oxo-butyric acid |
| 2-oxo-4-p-phenoxyphenylbutyric acid |

D. Any of the products of synthesis C of this example are reacted with L-proline ethyl ester, or L-proline-tert butyl ester or an α-ethyl ester of any of the L-proline analogs listed in Table III, immediately below. The reaction is carried out according to the coupling procedures of Synthesis B in Example 2.

TABLE III

3,4-dehydroproline
2,3-dehydroproline, 4,5-dehydroproline
2-OH-proline
3,4-di-OH-proline
3-methoxyproline
2-methoxyproline
3,4-dimethoxy proline
4-fluoro-proline
3-fluoro-proline
2-fluoro-proline3,4-di-OH-proline
3-methoxyproline
2-methoxyproline
3,4-dimethoxyproline
4-fluoro-proline
3-fluoro-proline
2-fluoro-proline
3,4-fluoroproline
2,3-difluoro-proline
3,4-difluoro-proline
4-Cl-proline
3-Cl-proline
2-Cl-proline
3,4-dichloro-proline
2,3-dichloro-proline
3-Br-proline
2-Br-proline
3,4-dibromo-proline
2,3-dibromo-proline
4-iodo-proline
3-iodo-proline
2-iodo-proline
3,4-diiodo-proline
5-phenyl-thioproline
5-hydroxyphenyl-thioproline (o-, m- or p-)
4-mercapto-proline
3-mercapto-proline
4-methylthio-proline
3-methylthio-proline
4-aminomethyl-proline
3-aminomethyl-proline
β-thioproline
α-methyl-proline
3-OH-5-methyl-proline
4-methylene proline
4-hydroxymethyl-proline
4-propyl-proline
3-propyl-proline
L-proline
L-pyroglutamic acid
4-keto-L-proline
3-keto-L-proline
4-hydroxy-L-proline
3-hydroxy-L-proline
L-pipecolic acid
4-methoxy-L-proline
4-bromo-L-proline
L-thiazolidine-4-carboxylic acid
L-2-azetidine carboxylic acid

Products of Synthesis D of this example are saponified to remove the ethyl ester. They are treated with anhydrous HF in the presence of anisole to remove the S-benzyl protecting group. If the ethyl ester or t-butyl ester groups are removed, the final product of Synthesis D of this example has the formula:

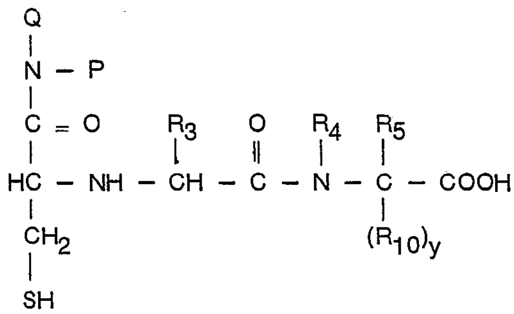

Example 58

By substituting the $N^{\alpha}$-Boc-α-methyl-S-benzyl-D-cysteine-α-$NO_2$-phenyl ester for $N^{\alpha}$-Boc-S-benzyl-D-cysteine-α-$NO_2$-phenyl ester of Synthesis B of Example 57, and following the procedures of Example 57, compounds of the formula:

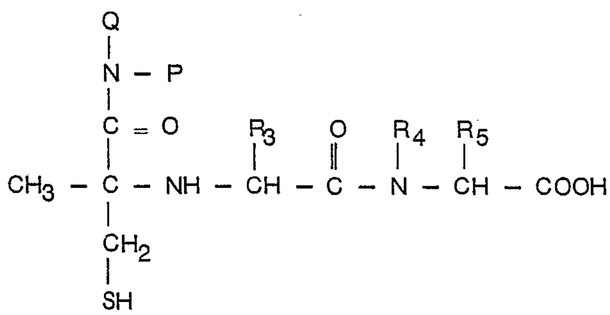

are isolated.

Example 59

By substituting $N^{\alpha}$-Boc-S-benzyl-D-homocysteine-α-$NO_2$-phenyl ester for $N^{\alpha}$-Boc-S-benzyl-D-cysteine-α-$NO_2$-phenyl ester of Synthesis B of Example 57, and following the procedures of Example 57, compounds of the formula

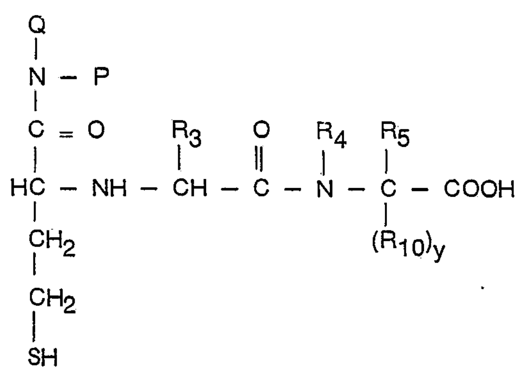

are obtained.

Example 60

By substituting $N^{\alpha}$-Boc-S-benzyl-α-methyl-D-homocysteine-α-$NO_2$phenyl ester for $N^{\alpha}$-Boc-S-benzyl-D-cysteine-α-$NO_2$-phenyl ester of Synthesis B in Example 57, and following the procedures of Example 57, compounds of the formula:

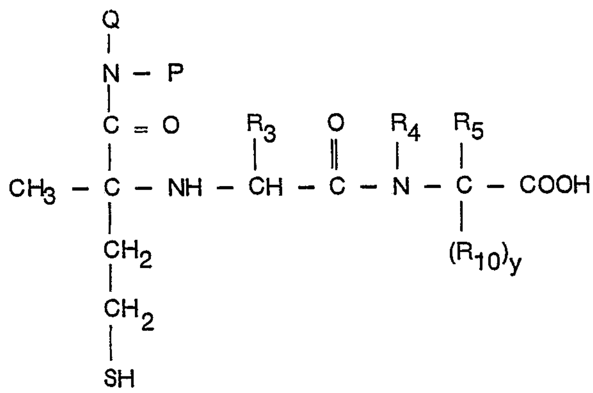

are obtained.

Example 61

By substituting $N^{\alpha}$-Boc-D-aspartic acid-α-$NO_2$-phenyl ester-β-ethyl ester for $N^{\alpha}$-Boc-S-benzyl-D-cysteine-α-$NO_2$-phenyl ester of Synthesis B in Example 57, and following the procedure of Example 57, with selected deprotection steps, compounds of the formula:

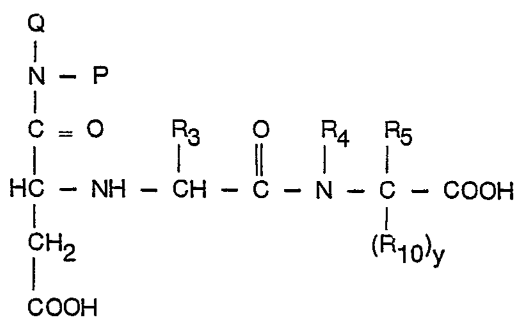

are obtained.

Example 62

By substituting $N^{\alpha}$-Boc-α-methyl-D-aspartic acid-α-$NO_2$-phenyl ester-β-ethyl ester for $N^{\alpha}$-Boc-S-benzyl-D-cysteine-α-$NO_2$-phenyl ester of Synthesis B in Example 57, and following the procedures of Example 57, with selected deprotection steps, compounds of the formula

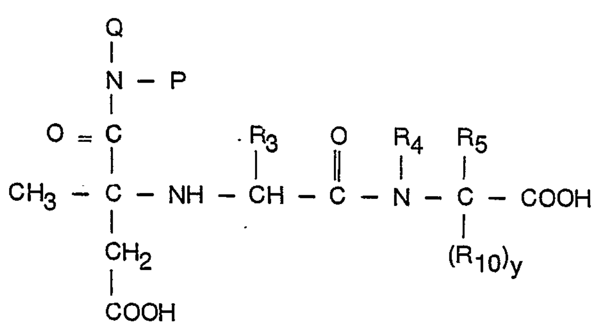

are obtained.

Example 63

By substituting $N^{\alpha}$-Boc-α-methyl-D-aspartic-α-p-$NO_2$-phenyl ester-β-ethyl ester for $N^{\alpha}$-Boc-S-benzyl-D cysteine-α-$NO_2$-phenyl ester of Synthesis B in Example 57, and by adding N-pyruvoyl-α-methyl-L-proline-t-butyl ester as the 2-keto-acyl-proline ester analog in Example 57, and following the procedures of Example 57, with selected deprotection steps, compounds of the formula:

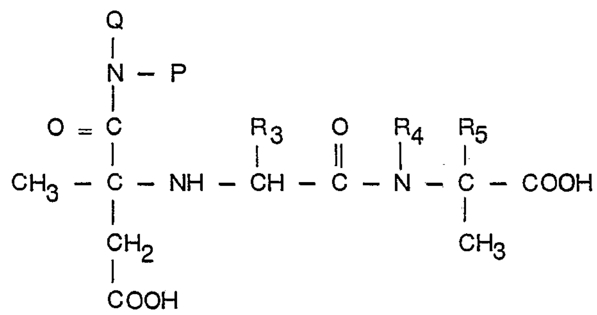

are obtained.

Example 64

By substituting $N^{\alpha}$-Boc-α-methyl-D-aspartic acid β-thiophenol ester for $N^{\alpha}$-Boc-S-benzyl-D-cysteine-α-$NO_2$-phenyl ester of Synthesis B in Example 57, and following the procedures of Example 57 with selected deprotection steps, including treatment with NaSH, compounds of the formula:

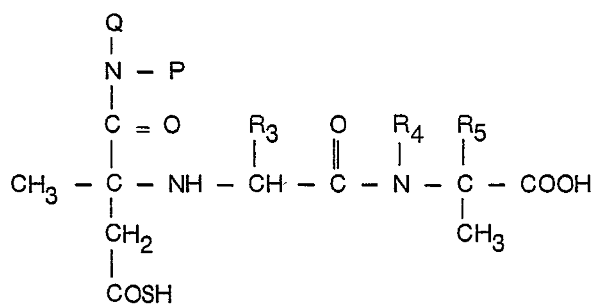

are obtained.

The foregoing examples are intended to be illustrative, not limiting. Many other variations of the present invention will readily occur to those of ordinary skill in the art, and it is intended that such variations are within the scope of the invention and the appended claims.

**Claims**

1. Novel compounds of the general formula:

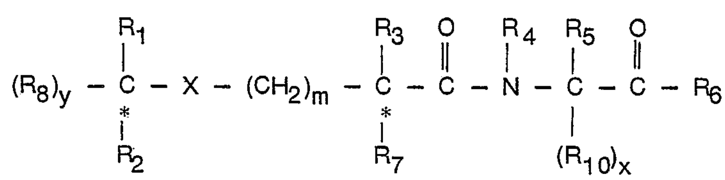

wherein

x and y are 0 or 1, X may be S, O or N—$R_9$ and $R_9$ may be —H or —$CH_3$;

$R_{10}$ is H, $CH_3$, F, Cl or Br;

m is 0 or 1,

$R_2$ is COOH, $CH_2COOH$, COSH, $CH_2COSH$, $CH_2SH$, $CH_2CH_2SH$, a physiologically acceptable nontoxic salt of any of them; COOY, $CH_2COOY$, COSY, $CH_2SY$, or $CH_2CH_2SY$ wherein Y is phenyl, benzyl or a 1—5 carbon alkyl group, or

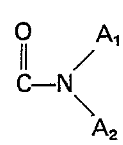

wherein either of $A_1$ and $A_2$ may be H, phenyl, benzyl or a 1—5 carbon alkyl group;

$R_4$ and $R_5$ together form a ring with the nitrogen and carbon atoms to which they are respectively attached, which ring is one of the structures:

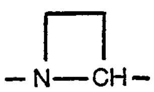 , 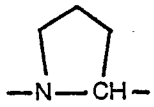 , 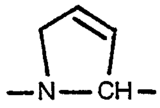 , 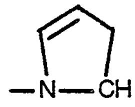 , 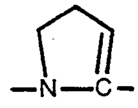 ,

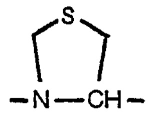 , 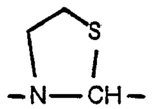 , 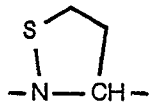 , 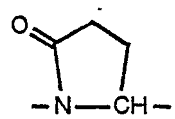 , 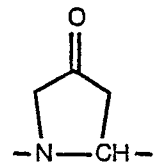 ,

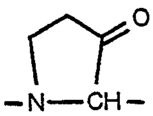 , 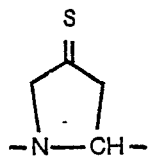 , 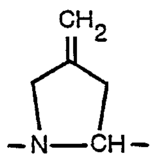 , 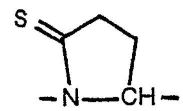 , 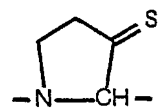 ,

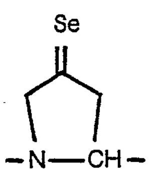 , 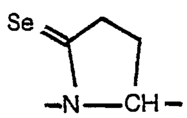 , 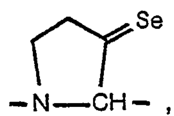 , 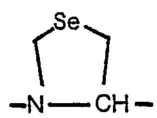 , 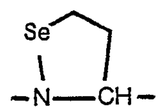 ,

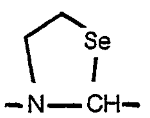 , 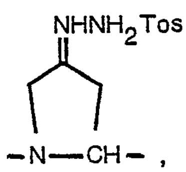 , 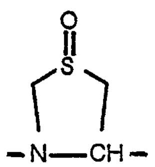 , 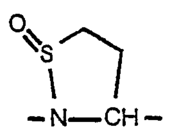 , 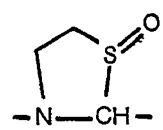 ,

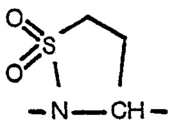 , 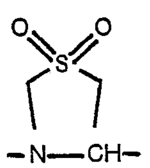 , 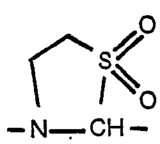 , 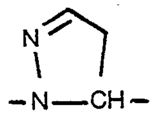 , 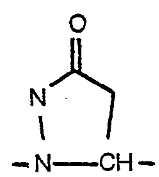 ,

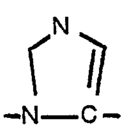 , 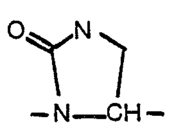 , 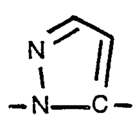 , 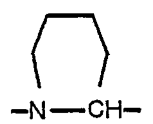 , 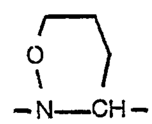 ,

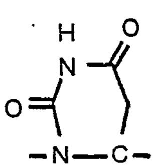 , 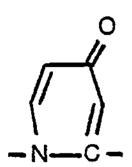 , 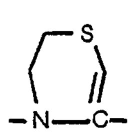 , 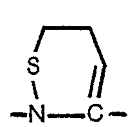 , or 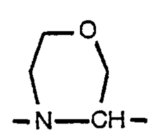 ,

it being understood that any of these structures may be monosubstituted with

$-OH$, $-OCH_3$, F, $-O-$ 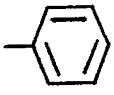 , $OCH_2-$ 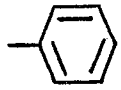 ,

Cl, Br, I, phenyl, hydroxyphenyl, $-SH$, $-SCH_3$, $-S-$ 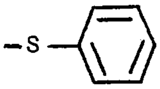 ,

$-SCH_2-$ 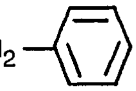 , $-NHCH_3$, $-CH_2NH_2$, $-CH_3$, $-CH_2OH$, propyl,

guanidino, nitro guanidino or thioguanidino and that any of the 5- or 6-membered rings may be disubstituted with —OH, F, Cl, Br, I, $OCH_3$ or any combination of two of this group of substituents;

$R_6$ is —OM or —SM, wherein M may be H, an alkyl group of 1—3 carbon atoms or any other ester moiety hydrolizable under mammalian *in vivo* conditions to —OH, or an ionically bonded anion of a physiologically acceptable nontoxic salt;

$R_7$ is H—, $CH_3$—, halomethyl, hydroxymethyl, aminomethyl or mercaptomethyl;

$R_8$ is H—, $CH_3$—, amino, halomethyl, hydroxymethyl, aminomethyl, dihalomethyl, trihalomethyl, mercaptomethyl, methoxymethyl, methylthiomethyl, methoxycarbonylmethyl, cyanomethyl, benzyl, acetoxymethyl, $CH_2=CH-CH_2-$, isobutyl, mercaptoalkyl of 2—3 carbon atoms, hydroxyalkyl of 2—3 carbon atoms, acetylthioethyl, benzamido, acetamido, phthaloylaminoalkylene wherein the alkylene group has 1—4 carbon atoms, α-alkoxycarbonyl isoalkylene wherein the alkyl group contains 1—5 carbons and the isoalkylene group contains 3—5 carbons, benzoylamine, alkanoylamine of 1—5 carbons, alkylamide of 1—5 carbons, phenylamine, alkylamine of 1—5 carbons, or ethyl; and

A. $R_1$ and $R_3$ may each be of the general formula

$$Q-\underset{\underset{P}{|}}{N}-\overset{\overset{O}{\|}}{C}-A_3-$$

(with P attached to N and =O on C)

wherein $A_3$ is

I. alkylene of 1—6 carbons, branched chain alkyl of 1—6 carbons, cycloalkyl alkylene, alkylcycloalkylalkylene, or alkylcycloalkylene;

II. aralkylene wherein the alkyl group is 1—6 carbons or alkylaryl;

III. phenyl;

IV. alkylaralkylene wherein the alkyl groups may be the same or different and are 1—6 carbons in length;

V. substituted alkylene, substituted branched chain alkyl, substituted cycloalkylalkylene, substituted alkylcycloalkylalkylene, substituted alkylcycloalkylene, substituted alkylaryl, substituted aralkylene, substituted phenyl or substituted alkylaralkylene wherein the substituent or substituents may be the same or different, may be included in an alkylene chain or pendant thereto, and are selected from amino, halo, hydroxy, mercapto, $NO_2$, carboxy, $CONH_2$, lower alkyl, halomethyl, hydroxymethyl, aminomethyl, dihalomethyl, trihalomethyl, cyano, mercaptomethyl, methoxymethyl, methylthiomethyl, methoxycarbonylmethyl, cyanomethyl, benzyl, acetoxymethyl, $CH_2=CH-CH_2-$, isobutyl, mercaptoalkyl of 2—3 carbon atoms, hydroxyalkyl of 2—3 carbon atoms, acetylthioethyl, benzamido, acetamido, phthaloylaminoalkylene wherein the alkylene group has 1—4 carbon atoms, α-alkoxycarbonyl isoalkylene wherein the alkyl group contains 1—5 carbons and the isoalkylene group contains 3—5 carbons, benzoylamino, alkanoylamino of 1—5 carbons, alkylamide of 1—5 carbons, phenylamine, alkylamine of 1—5 carbons, lower alkoxy, aryloxy, lower alkylamino, diloweralkylamino, acylamino, arylamino, guanidino, imidazolyl, indolyl, lower alkylthio, arylthio, carboxy amido and carbolower alkoxy;

VI. alkylenethio- or alkylenethioalkylene of 1—6 carbons, alkylthioalkylene of 1—6 carbons;

VII. alkyleneoxy or alkyleneoxyalkylene wherein the alkyl groups may be the same of different and are 1—6 carbons;

VIII. alkoxyphenyl or alkoxybenzoyl in which the alkoxy group has 1—3 carbons, phenoxyphenyl, phenoxybenzyl, benzyloxybenzyl or benzyloxyphenyl or a thioether analog of any of them;

IX. $$-(CH_2)_n-\underset{\underset{OB}{|}}{CH}-(CH_2)_m-$$

wherein n = 0—4, m = 0—4 and B = H or a 1—5 carbon alkyl group; or an —SB analog thereof;

X. 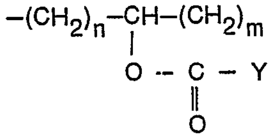 or 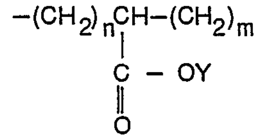

or 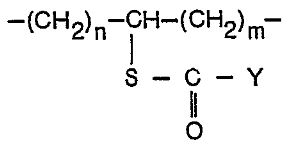 or 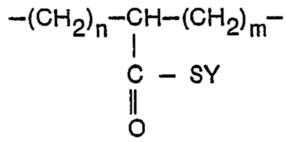

wherein n and m have the same significance as above, Y is phenyl, benzyl or a 1—5 carbon alkyl group;

XI. 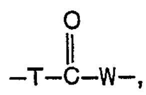, 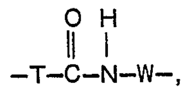, 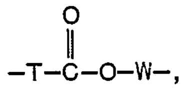, 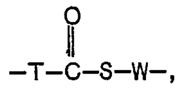,

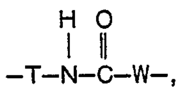, 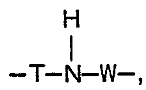, 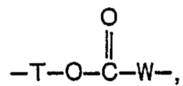, 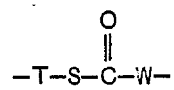

wherein T and W may be the same or different and are alkylene, aryl, benzyl or cycloalkyl; and P and Q may be the same, or one of them may be H or they may combine to form a ring with the nitrogen to which they are attached.

Either or both of P and Q may be selected from any of the following:

(a) $C_1$—$C_6$ straight or branched chain alkyl groups or $C_1$—$C_6$ straight or branched chain alkenyl groups, any one of which may be substituted with any of halo, hydroxy, alkoxy, aryloxy, amino, alkylamino, dialkylamino, alkylacylamino, arylamino, guanidino, thioguanidino, nitroguanidino, hydrazino, ureido, nitro, mercaptocarbonyl, hydroxyamino, histidinyl, cyano, imidazolyl, indolyl, mercapto, alkylthio, arylthio, carboxy amido or carboalkoxy, wherein the alkyl groups contain 1—6 carbon atoms;

(b) cycloalkyl or cycloalkyl alkylene wherein cycloalkyl has 4—12 carbons, and alkylene 1—5 carbons, which may be substituted with any of —OH, —SH, halo, COOH, COSH, $CONH_2$, $NO_2NH_2$, $NO_2$, $CH_3$, $—OCH_3$,

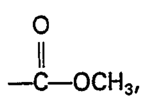

hydrazine, ureido, $—SCH_3$, hydroxyamino, cyano, guanidino, thioguanidino or nitroguanidino groups;

(c) aralkyl or alkaryl groups which may be ring substituted with one or more of the follows: SH, halo, $CH_2COOH$, $CH_2CONH_2$, $CH_2CONH$-alkyl, COSH, COOH, $CONH_2$, CONH-alkyl, $CH_2COSH$, $CH_2OH$, OH, $NO_2$, amino, alkyl, alkoxy, aralkyloxy, alkylthio and aralkylthio groups, wherein the alkyl groups contain 1—6 carbon atoms and may also alternatively be chain substituted with $—CH_3$, —OH, $—OCH_3$, halo,

$—SCH_3$, 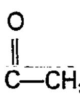, $—NH_2$, $—NO_2$, —CN, —SH, $—NHNH_2$, 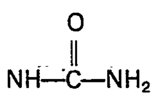

—NHOH or a thio or nitro derivative thereof, —COOH or COSH;

(d) an aryl, heterocyclic or adamantanyl group which may be ring-substituted with at least one group selected from halo, —OH, —O-alkyl, —O-aryl, $NH_2$, NH-alkyl, N-(alkyl)$_2$, 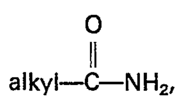, aryl-$NH_2$, guanidino, thioguanidino, nitroguanidino, hydrazino, ureido, nitro, mercaptocarbonyl, hydroxyamino, cyano imidazolyl, indanyl, histidinyl,

—S—alkyl, S—aryl, 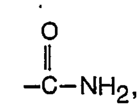, 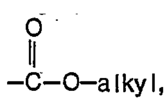, 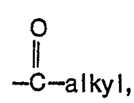, 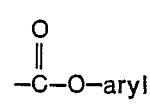,

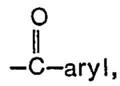, 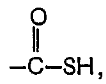, 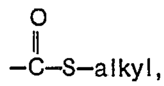, 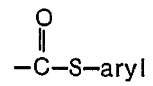 and $-NO_2$,

when P and Q join with N to form a ring, the ring may be any 4—10 membered hererocyclic ring which contains a nitrogen with only two of its valences attached to other ring members.

B. Alternatively $R_1$ may be 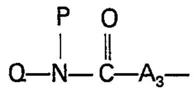

and $R_3$ may be

(i) mono-N substituted alkylene of 2—4 carbons wherein the N substituent is benzoyl, Boc, CbO, Tos, formyl or acetyl;

(ii) hydroxyphenyl or hydroxyphenyl-(1—6C)-alkylene or a thiol analog of either,

(iii) mercaptoalkylene of 1—6 carbons;

(iv) phenylalkylene wherein the alkylene group has 1—6 carbons

(v) phenylthioalkylene or benzylthioalkylene wherein the alkylene group has 1—6 carbons;

(vi) alkylthioalkylene wherein the alkyl and alkylene groups have 1—3 carbons;

(vii) alkoxyphenyl or alkoxybenzyl in which the alkoxy group has 1—3 carbons, phenoxyphenyl, phenoxybenzyl, benzyloxybenzyl or benzyloxyphenyl or a thioether analog of any of them;

(viii) 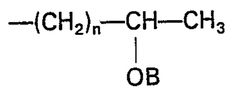

wherein n = 0—4 and B = H or a 1—6 carbon alkyl group; or an —SB analog thereof;

(ix) $(CH_2)_pCOOZ$ or $(CH_2)_p$, COSZ wherein p = 0—3 and Z is H, phenyl, benzyl, a 1—5 carbon alkyl group, or an anion of a physiologically acceptable salt;

(x) 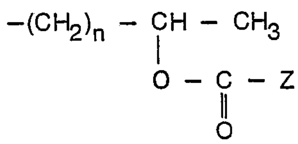 or 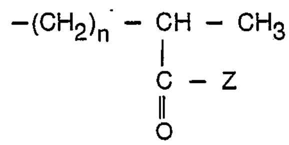

or 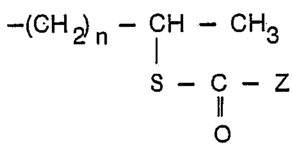 or 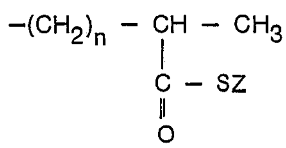

wherein n is 0 to 4 and Z each have the same significance as above;

(xi) 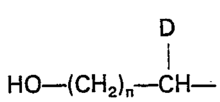 or 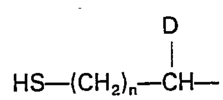

wherein n = 0—4, D is phenyl, thienyl or a 1—3 carbon alkyl group;

(xii) $HO—(CH_2)_n—C(CH_3)_2—$, $HS—(CH_2)_n—C(CH_3)_2—$, p-hydroxyphenyl — $(CH_2)_n—C(CH_3)_2—$ or p-mercaptophenyl-$(CH_2)_n—C(CH_3)_2$ — wherein n has the same significance as above;

(xiii) p-mercaptophenyl-$(CH_2)_n—CH_2—$ or p-hydroxyphenyl $—(CH_2)_n—CH_2—$ wherein the phenyl ring has one or two nitro or amino substituents and n has the same significance as above;

(xiv) 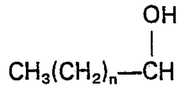 or 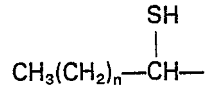

wherein n has the same significance as above;

(xv) $NH_2$—alkylene or $NO_2$—alkylene containing one hydroxy or mercapto substituent and having 1—6 carbon atoms;

(xvi) hydroxy- or mercapto-phenoxybenzyl;

$$\text{(xvii)}\ ZO(CH_2)_q - \overset{\overset{O}{\|}}{C} - (CH_2)_n -,\quad ZS - (CH_2)_q - \overset{\overset{O}{\|}}{C} - (CH_2)_n -,\quad NH_2-(CH_2)_q - \overset{\overset{O}{\|}}{C} - (CH_2)_n -,$$

$$NO_2(CH_2)_q - \overset{\overset{O}{\|}}{C} - (CH_2)_n -,\quad HONH - (CH_2)_q - \overset{\overset{O}{\|}}{C} - (CH_2)_n -,$$

$$NH_2NH - (CH_2)_q - \overset{\overset{O}{\|}}{C} - (CH_2)_n -,\quad ZO - \overset{\overset{O}{\|}}{C} - (CH_2)_q - \overset{\overset{O}{\|}}{C} - (CH_2)_n -,$$

$$ZS - \overset{\overset{O}{\|}}{C} - (CH_2)_q - \overset{\overset{O}{\|}}{C} - (CH_2)_n -,\ \text{or}\ NH_2\overset{\overset{O}{\|}}{C}NH - (CH_2)_q - \overset{\overset{O}{\|}}{C} - (CH_2)_n -$$

wherein $q = 1 - 5$ and n is from 0 to 4 and Z has the same significance as above;

$$\text{(xviii)}\ ZO-(CH_2)_q - \overset{\overset{OH}{|}}{CH} - (CH_2)_n -,\ ZS-(CH_2)_q - \overset{\overset{OH}{|}}{CH} - (CH_2)_n -,$$

$$NH_2-(CH_2)_q - \overset{\overset{O}{\|}}{C} - (CH_2)_n -,\quad NO_2 - (CH_2)_q - \overset{\overset{OH}{|}}{CH} - (CH_2)_n -,$$

$$NH_2 - \overset{\overset{O}{\|}}{C} - NH - (CH_2)_q - \overset{\overset{OH}{|}}{CH} - (CH_2)_n -,\ ZO-(CH_2)_q - \overset{\overset{OH}{|}}{CH} - (CH_2)_n -,$$

$$ZS - (CH_2)_q - \overset{\overset{OH}{|}}{CH} - (CH_2)_n -,\ HONH - (CH_2)_q - \overset{\overset{OH}{|}}{CH} - (CH_2)_n -,\ \text{or}$$

$$NH_2NH - (CH_2)_q - \overset{\overset{OH}{|}}{CH} - (CH_2)_n -,$$

wherein q and n all have the same significance as above;

$$\text{(xix)}\ G - NH - (CH_2)_q - \overset{\overset{O}{\|}}{C} - (CH_2)_n -,\ G - NH(CH_2)_q -$$

$$\overset{\overset{OH}{|}}{CH} - (CH_2)_n -,\ G - (CH_2)_q - \overset{\overset{O}{\|}}{C} - (CH_2)_n -,\ G - (CH_2)_q - \overset{\overset{OH}{|}}{CH} - (CH_2)_n -,$$

$$NH_2 - \underset{\underset{O}{\|}}{C} - (CH_2)_q - \underset{\underset{O}{\|}}{C} - (CH_2)_n -,\ \text{or}\ NH_2 - \overset{\overset{O}{\|}}{C} - (CH_2)_q - \overset{\overset{OH}{|}}{CH} - (CH_2)_n -,$$

wherein G is an alkacyl or alkacyloxy group of 1—6 carbons, a benzoyl or benzoyloxy group, or a phenylalkacyl or phenylalkacyloxy group wherein the alkacyl or alkacyloxy group contains 2—6 carbons and q and n have the same significance as set forth above;

(xx) $K-(CH_2)_n-\overset{O}{\overset{\|}{C}}-(CH_2)_n-$ or $K-(CH_2)_n-\overset{OH}{\overset{|}{C}H}-(CH_2)_n-$

wherein n has the significance stated above and K is selected from carboxyphenyl, aminophenyl, nitrophenyl, halophenyl, hydroxyphenyl, alkylthiophenyl, alkylphenyl, mercaptophenyl, cyanophenyl, mercapto-carbonylphenyl, alkylcarbonylphenyl, alkylcarbonyloxyphenyl, hydrazinophenyl, ureidophenyl, alkylcarbonylaminophenyl, alkylcarbonylthiophenyl, alkoxyphenyl and hydroxyaminophenyl, wherein all alkyl groups contain 1—6 carbon atoms;

(xxi) $L-(CH_2)_n-\overset{O}{\overset{\|}{C}}-(CH_2)_n-$ or $L-(CH_2)_n-\overset{OH}{\overset{|}{C}H}-(CH_2)_n-$

wherein n has the significance stated above and L is selected from cycloalkyl groups of 3—7 carbons which may be unsubstituted or substituted with up to two groups selected from among carboxy, amino, nitro, halo, hydroxy, mercapto, mercaptocarbonyl, hydroxyamino, alkyl, alkylcarbonyl, alkylcarbonyloxy, alkylthio, alkylcarbonylamino, alkylcarbonylthio, cyanohydrazino, ureido and alkyloxy, wherein all alkyl groups contain 1—6 carbon atoms;

(xxii) guanidino alkylene, thioguanidinoalkylene or nitroquanidino alkylene in which the alkylene groups contain 1—6 carbon atoms;

(xxiii) ring substituted aryl groups in which the ring substituents may be the same or different and may comprise up to five per ring of the following: $—NH_2$, —OZ, —SZ, halogen, —CN, $—NO_2$, —COOZ, —COSZ, $CONH_2$, $—NHNH_2$, alkyl alkylcarbonyl, alkylcarbonyloxy, alkylcarbonylamino, haloalkyl, dihaloalkyl, trihalomethyl, hydroxyamino, alkylcarbonylthio, phenoxy, and benzyloxy wherein the alkyl groups contain 1—6 carbon atoms and Z has the same significance as above;

(xxiv) amidoalkylene or alkylcarbonyl-aminoalkylene wherein the alkyl and alkylene groups contain 1—6 carbon atoms;

(xxv) hydroxyaminoalkylene of 1—6 carbons;

(xxvi) vinyl and substituted vinyl groups in which the substituents may be alkyl, aryl, cycloalkyl or heterocyclic groups;

(xxvii) unsubstituted heretocyclic groups from among phenothiazinyl, pyrrolidinyl, pyrrolyl, quinolinyl, imidazolyl, pyridyl, thyminyl, benzothiazinyl, indolyl, thienyl, purinyl, piperidinyl, morpholinyl, azaindolyl, pyrazinyl, pyrimidyl, piperonyl, piperazinyl, furanyl, thiazolyl and thiazolidinyl, cytosinyl;

(xxviii) alkylene or alkenyl groups of 1—6 carbons substituted with one of the heterocyclic rings from (xxvii) above;

(xxix) groups from (xxvii) or (xxviii) above containing up to four ring substituents on the heterocyclic ring selected from among —OZ, —SZ, —COOZ, $—NO_2$, $—NH_2$, —COSZ, halogen, haloalkyl, dihaloalkyl, trihalomethyl, cyano, $CONH_2$, alkyl, alkylcarbonyl, alkylcarbonyloxy, alkylcarbonylthio, phenoxy, benzyloxy, $-NH-\overset{O}{\overset{\|}{C}}-NH_2$, $—NHNH_2$ and HONH—, wherein Z has the same significance as above;

(xxx) groups from (xxvii), (xxviii) or (xxix) attached to one valence of an etheric —O— or —S—;

(xxxi) mono-, di or tri-alkyl, alkenyl- or phenyl-silyl or -selenyl wherein the alkyl or alkenyl groups contain 1—6 carbons;

(xxxii) any of H, 1—5 carbon straight or branched chain alkyl, phenyl, —OH, alkoxy of 1—6 carbons, benzyloxy, benzyloxyalkylene or phenoxyalkylene wherein the alkylene has 1—5 carbons, alkoxyalkylene having 1—5 carbons in the alkoxy and alkelene groups, aminoalkylene of 1—6 carbons, alkenyl of 1—6 carbons, benzyl, hydroxyalkyl of 1—6 carbons, mercaptoalkyl of 1—6 carbons, histidinyl, haloalkyl of 1—6 carbons, 4-aminomethyl-benzyl, acetamidoalkyl of 1—5 carbons, benzylthiomethylene, or dimethylaminoalkyl of 1—5 carbons.

C. Alternatively, $R_3$ may be $Q-\overset{P}{\overset{|}{N}}-\overset{Q}{\overset{\|}{C}}-A_3-$ and

$R_1$ may be any of groups (i) — (xxxii) above or any of H, $C_1$—$C_8$ straight or branched chain alkyl, phenyl, benzyl, unsubstituted aminoalkylene of 2—6 carbons, hydroxyalkylene of 1—6 carbons, hydroxyphenyl, phenoxyalkylene or benzyloxyalkylene wherein the alkylene group has 1—6 carbons, cycloalkyl of 3—6 carbons, cycloalkyl methyl, 3 indolyl, phenylethyl, methylthioethyl, 3 indolyl alkyl wherein the alkyl group

contains 1—5 carbons, imidazolyl, imidazolylalkyl wherein the alkyl group contains 1—5 carbons, phenoxymethyl, phenylthiomethyl, 4-aminomethyl benzyl, 2-aminophenethyl, naphthylethyl, 4-halophenethyl, 3,4-dihalophenethyl or phenoxyphenethyl, or $R_1$ and $R_2$ together may form with —CH a lactone ring of the formula:

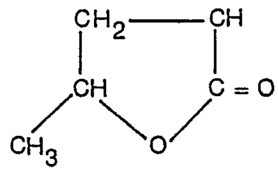

or

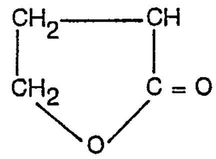

or an analogous six-membered ring.

2. A compound according to claim 1 wherein X is —NH—, —S— or —O—.

3. A compound according to claim 1 or 2 wherein $R_1$ and $R_3$ are each of the general formula

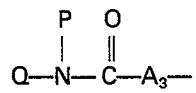

and P and Q may be the same, or one of them may be H or they may combine to form a ring with the nitrogen to which they are attached, wherein P and Q may be selected from any of the radicals of the groupa (a)—(d).

4. A compound according to claim 1 or 2 wherein $R_1$ is of the general formula 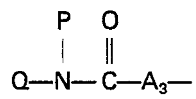

$R_3$ is a radical of groups (i)—(xxxii);
and P and Q may be the same, or one of them may be H or they may combine to form a ring with the nitrogen to which they are attached, wherein P and Q may be selected from any of the radicals of groups (a)—(d).

5. A compound according to any of claims 1 to 4 wherein
m is O;
X is N—$R_9$ and $R_9$ is H;
$R_4$ and $R_5$ together form a ring with the nitrogen and carbon atoms to which they are respectively attached, which ring is one of the structures:

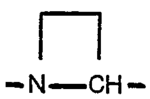 , 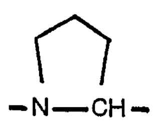 , 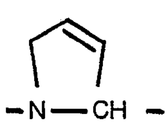 , 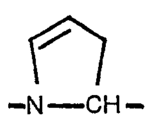 , 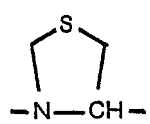 ,

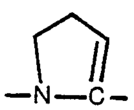 , 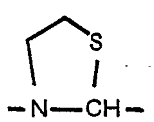 , 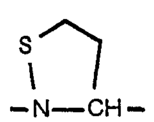 , 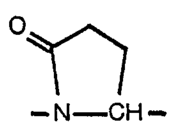 , 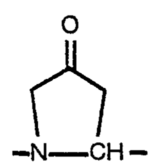 ,

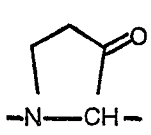 , 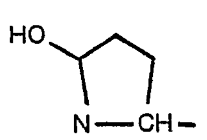 , 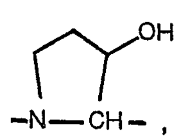 , 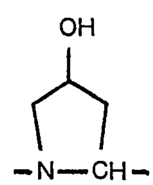 ;

$R_7$ is H— or $CH_3$—;
$R_8$ is H— or $CH_3$—; and
$R_{10}$ is H— or $CH_3$—.

6. A compound according to claim 5 wherein
$A_3$ is a radical of groups I—V; and
P and Q are the same or different and are selected from H and any radical of groups (a)—(d).

7. A compound according to claim 5 wherein
$A_3$ is a radical of groups I—V;
P and Q are the same or different and are selected from radicals of the groups (b)—(d),

when P and Q join to form a ring, the ring is any 4—10 membered heterocyclic ring which contains a nitrogen with only two of its valences attached to other ring numbers.

8. A compound according to any of claims 1—7 wherein $R_4$ and $R_5$ form one of the structures

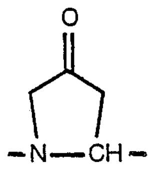 , 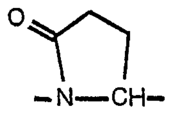 , 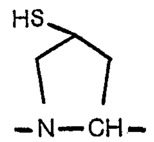 , 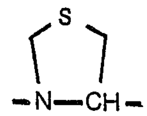 , 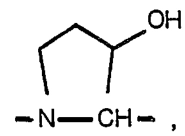 ,

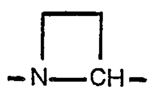 , 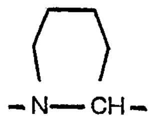 , 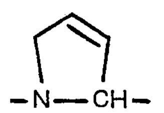 , 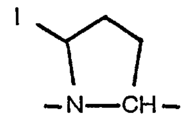 , 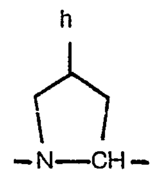 ,

(where h is Cl, F, Br or I), and $R_6$ is —OH, or a lower alkyl ester or physiologically acceptable salt thereof.

9. A compound according to any of claims 1—7 wherein $R_4$ and $R_5$ and $R_6$ together form substituted proline, or a lower alkyl ester thereof and physiologically acceptable salts thereof, wherein the substituent is selected from the group consisting of Cl, Br, F or I.

10. A compound according to any of claims 1—9 wherein P—N—Q form the structure

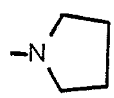

and x = 1, y = 1, X = N—$R_9$, m = O, $R_8$ = H, $R_3$ = $CH_3$, $R_4$ and $R_5$ together form the structure

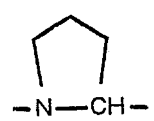 ,

$R_6$ = OH, $R_7$ = H and $R_2$ = COOH, $R_9$ = H, $R_{10}$ = H or physiologically acceptable salts thereof.

11. A compound according to any of claims 1—9 wherein P = H, Q = phenyl or iodo-phenyl, x = 1, y = 1, X = $NR_9$, m = O, $R_8$ = H, $R_3$ = $CH_3$, $R_4$ and $R_5$ together form the structure

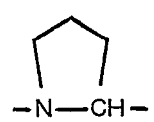 ,

$R_6$ = OH, $R_7$ = H and $R_2$ = COOH, $R_9$ = H, $R_{10}$ = H or physiologically acceptable salts thereof.

12. A compound according to claim 1 wherein $R_2$ is COOH, COOEt, COOMe, $CONH_2$, COSH, $CH_2SH$; or wherein P—N—Q forms structures selected from the group consisting of anilino, benzylamino, 2-amino pyridyl amino, 3-amino pyridyl amino, 4-amino pyridyl amino, 3-indolyl amino, and histamino; or wherein $R_3$ is $CH_3$.

13. A compound according to any of claims 1—12 wherein

P of $R_1$ is H;

Q of $R_1$ is aminoalkylene;

$A_3$ of $R_1$ is alkylene; and

P—N—Q of $R_3$ forms structures selected from the group consisting of anilino, benzylamino, 2-amino pyridyl amino, 3-amino pyridyl amino, 4-amino pyridyl amino, 3-indolyl amino, and histamino; or wherein

P of $R_3$ is H,

Q of $R_3$ is aminoalkylene;

$A_3$ of $R_3$ is alkylene;

P—N—Q of $R_1$ forms structures selected from the group consisting of anilino, benzylamino, 2-amino pyridyl amino, 3-amino pyridyl amino, 4-amino pyridyl amino, 3-indolyl amino, and histamino; or wherein

$R_1$ is 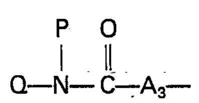

$R_3$ is phenyloxyalkylene, benzyloxyalkylene, benzylalkyleneoxyalkylene, wherein the alkylene group has 1—5 carbons; or wherein

$R_1$ is phenyloxyalkylene, benzyloxyalkylene, benzylalkylene oxyalkylene, wherein the alkylene group has 1—5 carbons;

$R_3$ is 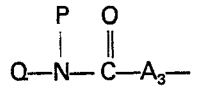

14. A composition of matter effective to inhibit angiotensin converting enzyme *in vivo* or to reduce the blood pressure *in vivo* of a mammal in a hypertensive state which contains as its essential active ingredient a therapeutically effective amount of a compound of any of claims 1—13.

15. A compound of any of claims 1—13 for use in treating hypertension or abnormal serum levels of angiotensin II in mammals.

**Patentansprüche**

1. Neue Verbindungen der allgemeinen Formel

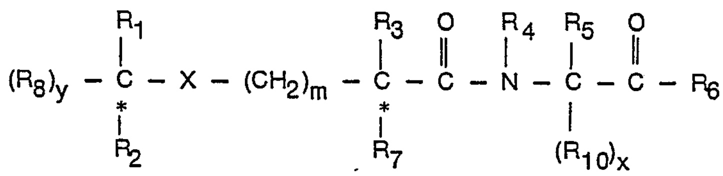

worin

x und y 0 oder 1 sind, S, O oder N—$R_9$ und $R_9$—H oder —$CH_3$ sein kann, $R_{10}$H, $CH_3$, F, Cl oder Br ist; m 0 oder 1 ist;

$R_2$ COOH, $CH_2COOH$, COSH, $CH_2COSH$, $CH_2SH$, $CH_2CH_2SH$, ein pharmazeutisch annehmbares Salz jeder dieser Gruppen, COOY, $CH_2COOY$, COSY, $CH_2SY$ oder $CH_2CH_2SY$, worin Y Phenyl, Benzyl oder eine Alkylgruppe mit 1 bis 5 Kohlenstoffatom ist, oder

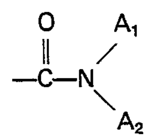

worin $A_1$ und $A_2$ H, Phenyl, Benzyl oder eine Alkylgruppe mit 1 bis 5 Kohlenstoffatom sein können, ist;

$R_4$ und $R_5$ zusammen einen Ring mit dem Stickstoffatom und den Kohlenstoffatomen bilden, an welchen sie jeweils hängen, welcher Ring eine der folgenden Strukturen aufweist:

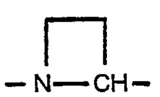 , 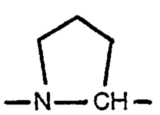 , 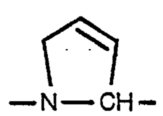 , 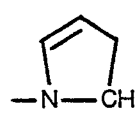 , 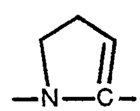 ,

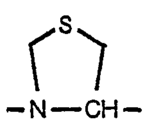 , 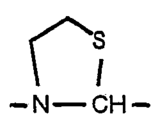 , 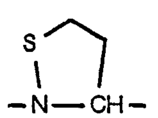 , 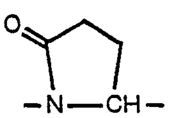 , 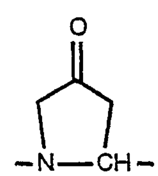 ,

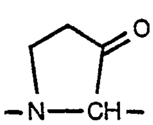 , 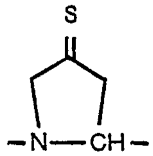 , 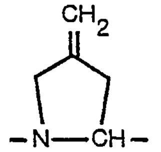 , 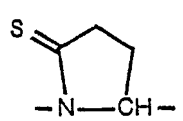 , 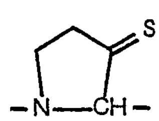 ,

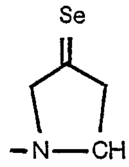 , 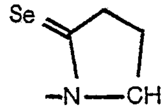 , 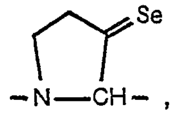 , 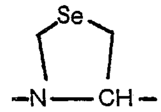 , 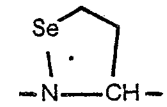 ,

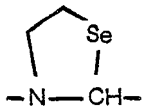 , 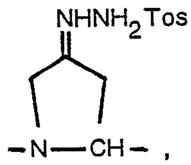 , 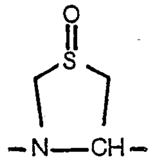 , 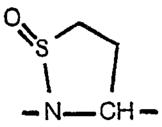 , 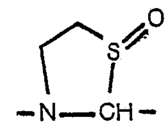 ,

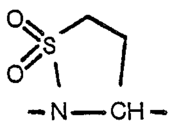 , 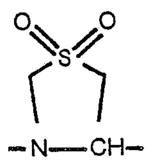 , 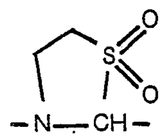 , 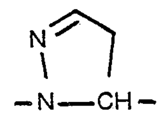 , 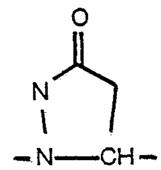 ,

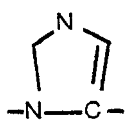 , 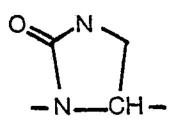 , 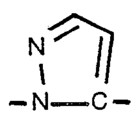 , 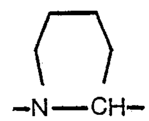 , 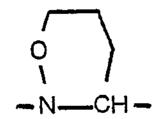 ,

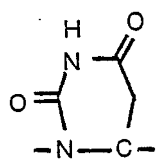 , 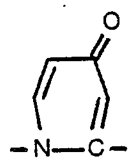 , 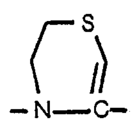 , 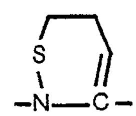 , oder 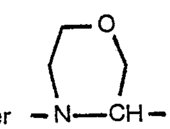 ,

wobei jede dieser Strukturen mit $-OH$, $-OCH_3$, F, $-O$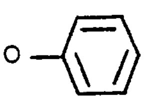, $-OCH_2$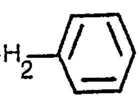, Cl, Br, I, Phenyl, Hydroxyphenyl, $-SH$, $-SCH_3$, $-S$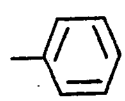, $-SCH_2$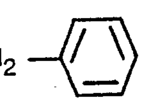, $-NHCH_3$, $-CH_2NH_2$, $-CH_3$, $-CH_2OH$, Propyl, Guanidino, Nitroguanidino oder Thioguanidino monosubstituiert sein kann und jeder der 5- oder 6-gliedrigen Ringe mit —OH, F, Cl, Br, I, $OCH_3$ oder irgendeiner Kombination von zweien dieser Substituentengruppe disubstituiert sein kann;

$R_6$—OM oder —SM ist, wobei M H, ein Alkylgruppe mit 1 bis 3 Kohlenstoffatom oder jede andere, unter in-vivo-Bedingungen beim Säugetier zu —OH hydrolysierbare Estergruppe oder ein ionisch gebundenes Anion eines physiologisch annehmbaren nicht toxischen Salzes sein kann,

$R_7$ H—, $CH_3$—, Halomethyl, Hydroxymethyl, Aminomethyl oder Mercaptomethyl ist;

$R_8$ H, $CH_3$—, Amino, Halomethyl, Hydroxymethyl, Aminomethyl, Dihalomethyl, Trihalomethyl, Mercaptomethyl, Methoxymethyl, Methylthiomethyl, Methoxycarbonylmethyl, Cyanomethyl, Benzyl, Acetoxymethyl, $CH_2{=}CH{-}CH_2$—, Isobutyl, Mercaptoalkyl mit 2 bis 3 Kohlenstoffatomen, Hydroxyalkyl mit 2 bis 3 Kohlenstoffatomen, Acetylthioethyl, Benzamido, Acetamido, Phthaloylaminoalkylen, worin die Alkenylgruppe 1 bis 4 Kohlenstoffatome hat, α-Alkoxycarbonylisoalkylen, worin die Alkylgruppe 1 bis 5 Kohlenstoffatome hat und die Isoalkylengruppe 3 bis 5 Kohlenstoffatome hat, Benzoylamin, Alkanoylamin

mit 1 bis 5 Kohlenstoffatomen, Alkylamid mit 1 bis 5 Kohlenstoffatomen, Phenylamin, Alkylamin mit 1 bis 5 Kohlenstoffatomen oder Ethyl ist; und

A. $R_1$ und $R_3$ jeweils die allgemeine Formel 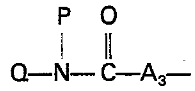

aufweisen können, worin $A_3$

I. Alkylen mit 1 bis 6 Kohlenstoffatomen, ein verzweigtkettiges Alkyl mit 1 bis 6 Kohlenstoffatomen, Cycloalkylalkylen, Alkylcycloalkylalkylen oder Alkylcycloalkylen;

II. Aralkylen, worin die Alkylgruppe 1 bis 6 Kohlenstoffatom hat, oder Alkylaryl;

III. Phenyl;

IV. Alkylaralkylen, worin die Alkylgruppen gleich oder verschieden sein können und 1 bis 6 Kohlenstoffatomen enthalten;

V. substituiertes Alkylen, substituiertes verzweigtkettiges Alkyl, substituiertes Cycloalkylalkylen, substituiertes Alkylcycloalkylalkylen, substituiertes Alkylcycloalkylen, substituiertes Alkylaryl, substituiertes Aralkylen, substituiertes Phenyl oder substituiertes Alkylaralkylen, worin der Substituent oder die Substituenten gleich oder verschieden, in einer Alkylenkette eingeschlossen sein oder daran hängen können und aus Amino, Halo, Hydroxy, Mercapto, $NO_2$, Carboxy, $CONH_2$, niederem Alkyl, Halomethyl, Hydroxymethyl, Aminomethyl, Dihalomethyl, Trihalomethyl, Cyano, Mercaptomethyl, Methoxymethyl, Methylthiomethyl, Methoxycarbonylmethyl, Cyanomethyl, Benzyl, Acetoxymethyl, $CH_2{=}CH{-}CH_2{-}$, Isobutyl, Mercaptoalkyl mit 2 bis 3 Kohlenstoffatomen, Hydroxyalkyl mit 2 bis 3 Kohlenstoffatomen, Acetylthioethyl, Benzamido, Acetamido, Phthaloylaminoalkylen, worin die Alkylengruppe 1 bis 4 Kohlenstoffatome hat, α-Alkoxycarbonylisoalkylen, worin die Alkylgruppe 1 bis 5 Kohlenstoffatome und die Isoalkylengruppe 3 bis 5 Kohlenstoffatome hat, Benzoylamino, Alkanoylamino mit 1 bis 5 Kohlenstoffatomen, Phenylamin, Alkylamin mit 1 bis 5 Kohlenstoffatomen, niederem Alkoxy, Aryloxy, niederem Dialkyl-amino, Acylamino, Arylamino, Guanidino, Imidazolyl, Indolyl, niederem Alkylthio, Arylthio, Carboxyamino und niederem Alkoxy-carbonyl ausgewählt sind;

VI. Alkylenthio oder Alkylenthioalkylen mit 1 bis 6 Kohlenstoffatomen, Alkylthioalkylen mit 1 bis 6 Kohlenstoffatomen;

VII. Alkylenoxy oder Alkylenoxyalkylen, worin die Alkylgruppen gleich oder verschieden sein können und 1 bis 6 Kohlenstoffatome haben;

VIII. Alkoxyphenyl oder Alkoxybenzyl, worin die Alkoxygruppe 1 bis 3 Kohlenstoffatome hat, Phenoxyphenyl, Phenoxybenzyl, Benzyloxybenzyl oder Benzyloxyphenyl, oder ein Thioether-Analoges davon;

(IX). 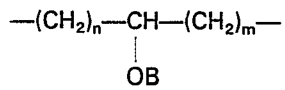

worin n 0 bis 4, m 0 bis 4 und B H oder eine Alkylgruppe mit 1 bis 5 Kohlenstoffatomen ist, oder ein —SB—Analoges davon;

X. 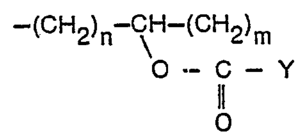 oder 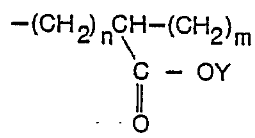

oder 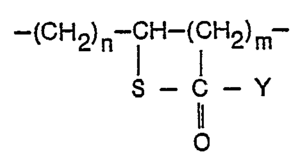 oder 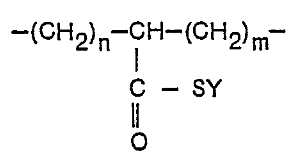

worin n und m die oben angegebene Bedeutung haben und Y Phenyl, Benzyl oder eine Alkylgruppe mit 1 bis 5 Kohlenstoffatomen ist;

XI. 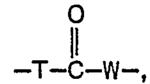, 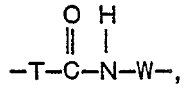, 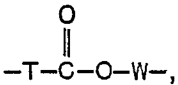, 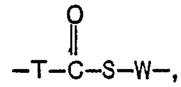, 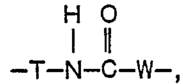, 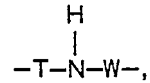,

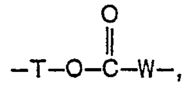, 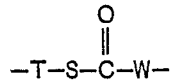

worin T und W gleich oder verschieden sein können und Alkylen, Aryl, Benzyl oder Cycloalkyl sind, und P und Q gleich sein können oder eines davon H sein kann oder beide zusammen einen Ring mit dem Stickstoff bilden können, an welchem sie hängen, ist.

P und Q zusammen oder jedes für sich kann aus einer der folgenden Gruppen ausgewählt sein:

(a)Gerad- oder verzweigtkettige Alkylengruppen mit 1 bis 6 Kohlenstoffatome oder gerad- oder verzweigtkettige Alkenylgruppen mit 1 bis 6 Kohlenstoffatomen, deren jede mit Halo, Hydroxy, Alkoxy, Aryloxy, Amino, Alkylamino, Dialkylamino, Alkylacylamino, Arylamino, Guanidino, Thioguanidino, Nitroguanidino, Hydrazino, Ureido, Nitro, Mercaptocarbonyl, Hydroxyamino, Histidinyl, Cyano, Imidazolyl, Indolyl, Mercapto, Alkylthio, Arylthio, Carboxyamido oder Alkoxycarbonyl substituiert sein kann, wobei die Alkylgruppen 1 bis 6 Kohlenstoffatome enthalten;

(b) Cycloalkyl oder Cycloalkylalkylen, worin Cycloalkyl 4 bis 12 Kohlenstoffatome und Alkylen 1 bis 5 Kohlenstoffatome hat, welche mit —OH, —SH, Halo, COOH, COSH, $CONH_2$, $NO_2NH_2$, $NO_2$, $CH_3$, —$OCH_3$,

$$-\overset{\overset{O}{\|}}{C}-OCH_3,$$

Hydrazin-, Ureido-, —$SCH_3$, Hydroxyamino-, Cyano-, Guanidino-, Thioguanidino- oder Nitroguanidinogruppen substituiert sein können;

(c) Aralkyl oder Alkarylgruppen, welche mit einer oder mehreren der folgenden Gruppen ringsubstituiert sein können:

SH, Halo, $CH_2COOH$, $CH_2CONH_2$, $CH_2CONH$-Alkyl, COSH, COOH, $CONH_2$, CONH-Alkyl, $CH_2COSH$, $CH_2SH$, $CH_2OH$, OH, $NO_2$, Amino-, Alkyl-, Alkoxy-, Aralkyloxy-, Alkylthio- und Aralkylthiogruppen, worin die Alkylgruppen 1 bis 6 Kohlenstoffatome enthalten und wahlweise auch in der Kette mito —$CH_3$, —OH,

$$-OCH_3,\ Halo,\ -SCH_3,\ \overset{\overset{O}{\|}}{C}-CH_3,\ -NH_2,\ NO_2,\ -CN,\ -SH,\ -NHNH_2,\ NH-\overset{\overset{O}{\|}}{C}-NH_2,$$

—NHOH oder einem Thio- oder Nitroderivat davon, COOH oder COSH substituiert sein können;

(d) eine Aryl, heterocyclische oder Adamantanylgruppe, welche mit wenigstens einer der aus den folgenden Gruppen ausgewählten Gruppen ringsubstituiert sein kann: Halo, OH, —O—Alkyl, —O—Aryl,

$$NH_2,\ NH\text{-}Alkyl,\ N\text{-}(Alkyl)_2,\ Alkyl-\overset{\overset{O}{\|}}{C}-NH_2,$$

Aryl-$NH_2$, Guanidino, Thioguanidino, Nitroguanidino, Hydrazino, Ureido, Nitro, Mercaptocarbonyl, Hydroxyamino, Cyano, Imidazolyl, Indanyl, Histidinyl,

$$-SH,\quad -S-Alkyl,\quad S-Aryl,\quad -\overset{\overset{O}{\|}}{C}-NH_2,\quad -\overset{\overset{O}{\|}}{C}-O-Alkyl,\quad -\overset{\overset{O}{\|}}{C}-Alkyl,\quad -\overset{\overset{O}{\|}}{C}-O-Aryl,$$

$$-\overset{\overset{O}{\|}}{C}-Aryl,\quad -\overset{\overset{O}{\|}}{C}-SH,\quad -\overset{\overset{O}{\|}}{C}-S-Alkyl,\quad -\overset{\overset{O}{\|}}{C}-S-Aryl\ \text{und}\ -NO_2,$$

wenn P und Q zusammen mit N einen Ring bilden, kann der Ring jeder 4- bis 10-gliedrige Ring sein, der ein Stickstoffatom enthält, das mit nur zwei seiner Valenzen an andere Ringglieder gebunden ist.

B. Wechselweise kann $R_1$

$$Q-\overset{\overset{P}{|}}{N}-\overset{\overset{O}{\|}}{C}-A_3$$

sein und $R_3$ kann

(i) mono-N-substituiertes Alkylen mit 2 bis 4 Kohlenstoffatomen, worin der N-Substituent Benzoyl, Boc, CbO, Tos, Formyl oder Acetyl ist;

(ii) Hydroxyphenyl oder Hydroxyphenyl-alkylen (mit 1 bis 6 Kohlenstoffatomen) oder ein Thiolanaloges von beiden;

(iii) Mercaptoalkylen mit 1 bis 6 Kohlenstoffatomen;

(iv) Phenylalkylen, worin die Alkylengruppe 1 bis 6 Kohlenstoffatome hat;

(v) Phenylthioalkylen oder Benzylthioalkylen, worin die Alkylengruppe 1 bis 6 Kohlenstoffatome hat;

(vi) Alkylthioalkylen, worin die Alkyl- und Alkylengruppe 1 bis 3 Kohlenstoffatome hat;

(vii) Alkoxyphenyl oder Alkoxybenzyl, worin die Alkoxygruppe 1 bis 3 Kohlenstoffatome hat, Phenoxyphenyl, Phenoxybenzyl, Benzyloxybenzyl oder Benzyloxyphenyl oder ein Thioetheranaloges jeder dieser Gruppen;

(vii) 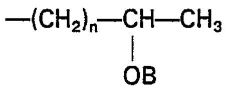

worin n 0 bis 4 und B H oder eine Alkylgruppe mit 1 bis 6 Kohlenstoffatomen ist, oder ein SB—Analoges davon;

(ix) $(CH_2)_pCOOZ$ oder $(CH_2)_pCOSZ$, worin p 0 bis 3 und Z H, Phenyl, Benzyl, eine Alkylgruppe mit 1 bis 5 Kohlenstoffatomen oder ein Anion eines physiologisch annehmbaren Salzes ist;

(x) 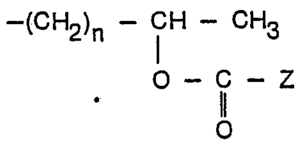 oder 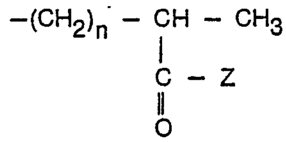

oder 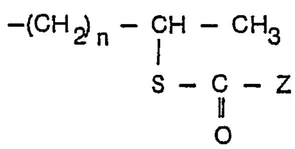 oder 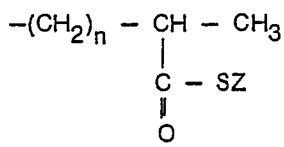

worin n 0 bis 4 ist und Z jeweils die oben angegebene Bedeutung hat;

(xi) 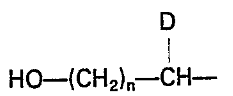 oder 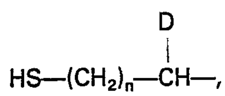

worin n 0 bis 4 und D Phenyl, Thienyl oder eine Alkylgruppe mit 1 bis 3 Kohlenstoffatomen ist;

(xii) $HO—(CH_2)_n—C(CH_3)_2—$, $HS—(CH_2)_n—C(CH_3)_2—$, p-Hydroxyphenyl-$(CH_2)_n—C(CH_3)_2—$ oder p-Mercaptophenyl-$(CH_2)_n—C(CH_3)_2—$, worin n die oben angegebene Bedeutung hat;

(xiii) p-Mercaptophenyl-$(CH_2)_n—CH_2—$ oder p-Hydroxyphenyl-$(CH_2)_n—CH_2—$, worin der Phenylring einen oder zwei Nitro- oder Aminosubstituenten hat und n die oben angegebene Bedeutung hat;

(xiv) 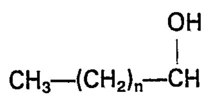 oder 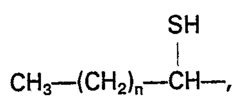

worin n die oben angegebene Bedeutung hat;

(xv) $NH_2$—Alkylen oder $NO_2$—Alkylen mit einem Hydroxy- oder Mercaptosubstituenten und 1 bis 6 Kohlenstoffatomen;

(xvi) Hydroxy- oder Mercapto-phenoxybenzyl;

(xvii) 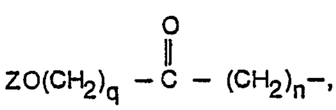 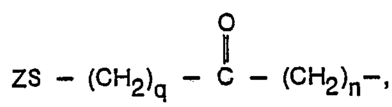 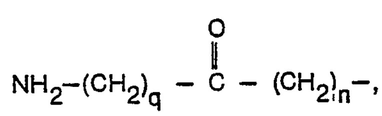

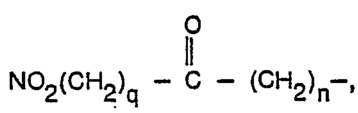 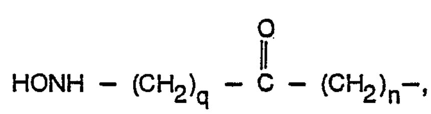

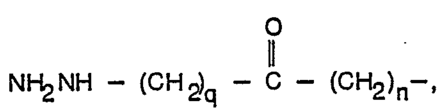 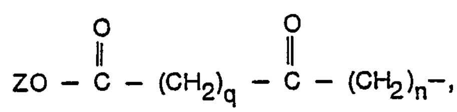

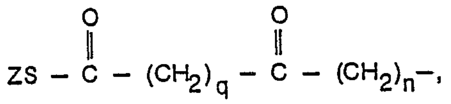 od. 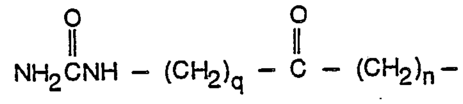

worin q = 1 – 5 und n 0 bis 4 ist und Z die oben angegebene Bedeutung hat;

(xviii) 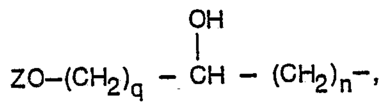 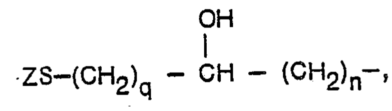

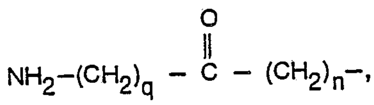 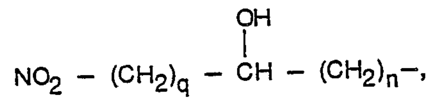

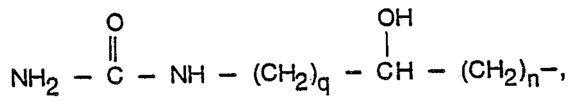 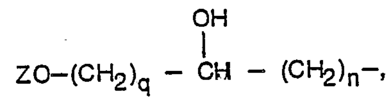

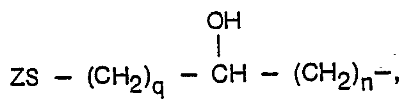 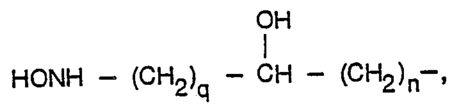 oder

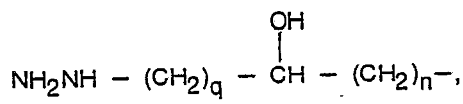

worin q und n die oben angegebenen Bedeutungen haben;

(xix) 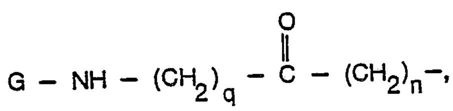 G – NH$(CH_2)_q$ –

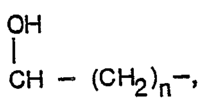 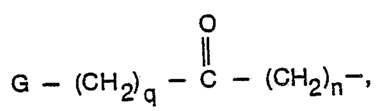 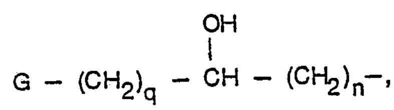

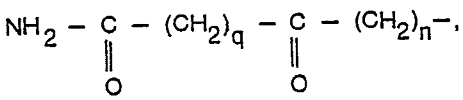 oder 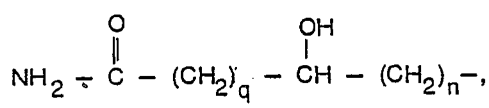

worin G eine Alkacyl- oder Alkacyloxygruppe mit 1 bis 6 Kohlenstoffatomen, eine Bonzoyl- oder Benzoylgruppe oder eine Phenylalkacyloxygruppe ist, worin die Alkacyl- oder Alkacyloxygruppe 2 bis 6 Kohlenstoffatome enthält und q und n die oben angegebene Bedeutung haben;

(xx) 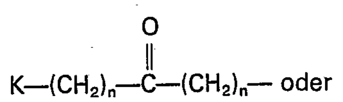 oder 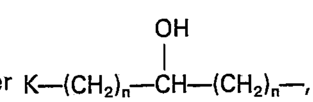

worin n die oben angegebene Bedeutung hat und K aus Carboxyphenyl, Aminophenyl, Nitrophenyl, Halophenyl, Hydroxyphenyl, Alkylthiophenyl, Alkylphenyl, Mercaptophenyl, Cyanophenyl, Mercaptocarbonylphenyl, Alkylcarbonylphenyl, Alkylcarbonyloxyphenyl, Hydrazinophenyl, Ureidophenyl, Alkylcarbonylaminophenyl, Alkylcarbonylthiophenyl, Alkyloxyphenyl und Hydroxyaminophenyl ausgewählt ist, worin alle Alkylgruppen 1 bis 6 Kohlenstoffatome enthalten;

(xxi) $L-(CH_2)_n-C(=O)-(CH_2)_n-$ oder $L-(CH_2)_n-CH(OH)-(CH_2)_n-$,

worin n die oben angegebene Bedeutung hat und L aus Cycloalkylgruppen mit 3 bis 7 Kohlenstoffatomen ausgewählt ist, welche unsubstituiert oder mit bis zu zwei aus Carboxy, Amino, Nitro, Halo, Hydroxy, Mercapto, Mercaptocarbonyl, Hydroxyamino, Alkyl, Alkylcarbonyl, Alkylcarbonyloxy, Alkylthio, Alkylcarbonylamino, Alkylcarbonylthio, Cyanohydrazino, Ureido und Alkyloxy ausgewählten Gruppen substituiert sein kann, worin alle Alkylgruppe 1 bis 6 Kohlenstoffatome enthalten;

(xxii) Guanidinoalkylen, Thioguanidinoalkylen oder Nitroguanidinoalkylen, wobei die Alkylengruppen 1 bis 6 Kohlenstoffatome enthalten;

(xxiii) ringsubstituierte Arylgruppen, worin die Ringsubstituenten gleich oder verschieden sein können und bis zu fünf der folgenden Gruppen pro Ring enthalten können: $-NH_2$, —OZ, —SZ, Halogen, —CN, $-NO_2$, —COOZ, —COSZ, $-CONH_2$, $-NHNH_2$, Alkyl, Alkylcarbonyl, Alkylcarbonyloxy, Alkylcarbonylamino, Haloalkyl, Dihaloalkyl, Trihalomethyl, Hydroxyamino, Alkylcarbonylthio, Phenoxy und Benzyloxy, worin die Alkylgruppen 1 bis 6 Kohlenstoffatome enthalten und Z die oben angegebene Bedeutung hat;

(xxiv) Amidoalkylen oder Alkylcarbonyl-aminoalkylen, worin die Alkyl- und Alkylengruppen 1 bis 6 Kohlenstoffatomen enthalten;

(xxv) Hydroxyaminoalkylen mit 1 bis 6 Kohlenstoffatomen;

(xxvi) Vinyl- und substituierte Vinylgruppen, wobei die Substituenten Alkyl, Aryl, Cycloalkyl oder heterocyclische Gruppen sein können;

(xxxvii) unsubstituierte heterocyclische Gruppen aus der von Phenothiazinyl, Pyrrolidinyl, Pyrrolyl, Chinolinyl, Imidazolyl, Pyridyl, Thyminyl, Benzothiazinyl, Indolyl, Thienyl, Purinyl, Piperidinyl, Morpholinyl, Azaindolyl, Pyrazinyl, Pyrimidyl, Piperonyl, Piperazinyl, Furanyl, Thiazolyl und Thiazolidinyl, Cytosinyl gebildeten Gruppe;

(xxviii) mit einem der unter (xxvii) angeführten heterocyclischen Ringe substituierte Alkylen- oder Alkenylgruppen mit 1 bis 6 Kohlenstoffatomen;

(xxix) unter (xxvii) oder (xxviii) angeführte Gruppen mit bis zu vier aus —OZ, —SZ, —COOZ, $-NO_2$, $-NH_2$, —COSZ, Halogen, Haloalkyl, Dihaloalkyl, Trihalomethyl, Cyano, $CONH_2$, Alkyl, Alkylcarbonyl, Alkylcarbonyloxy, Alkylcarbonylthio, Phenoxy, Benzyloxy, $-NH-C(=O)-NH_2$, $-NHNH_2$ und HONH— ausgewählten Ringsubstituenten am heterocyclischen Ring, wobei Z die oben angegebene Bedeutung hat;

(xxx) unter (xxvii), (xxviii) oder (xxix) angeführte, an eine Valenz eines etherischen —O— oder —S— gebundene Gruppen;

(xxxi) Mono-, Di- oder Trialkyl, Alkenyl oder Phenylsilyl oder -selenyl, worin die Alkyl- oder Alkenylgruppen 1 bis 6 Kohlenstoffatome enthalten;

(xxxii) H, gerad- oder verzweigtkettiges Alkyl mit 1 bis 5 Kohlenstoffatomen, Phenyl, —OH, Alkoxy mit 1 bis 6 Kohlenstoffatomen, Benzyloxy, Benzyloxyalkylen oder Phenoxyalkylen, worin das Alkylen 1 bis 5 Kohlenstoffatome hat, Alkoxyalkylen mit 1 bis 5 Kohlenstoffatomen in der Alkoxy- und Alkenylgruppe, Aminoalkylen mit 1 bis 6 Kohlenstoffatomen, Alkenyl mit 1 bis 6 Kohlenstoffatomen, Benzyl, Hydroxyalkyl mit 1 bis 6 Kohlenstoffatomen, Mercaptoalkyl mit 1 bis 6 Kohlenstoffatomen, Histidinyl, Haloalkyl mit 1 bis 6 Kohlenstoffatomen, 4-Aminomethylbenzyl, Acetamidoalkyl mit 1 bis 5 Kohlenstoffatomen, Benzylthiomethylen oder Dimethylaminoalkyl mit 1 bis 5 Kohlenstoffatomen sein.

C. Wechselweise kann $R_3$ $Q-N(P)-C(=Q)-A_3-$

sein und $R_1$ kann jede der unter (i) bis (xxxii) angeführten Gruppen oder H, gerad- oder verzweigtkettiges Alkyl mit 1 bis 8 Kohlenstoffatomen, Phenyl, Benzyl, unsubstituiertes Aminoalkylen mit 2 bis 6 Kohlenstoffatomen, Hydroxyalkylen mit 1 bis 6 Kohlenstoffatomen, Hydroxyphenyl, Phenoxyalkylen oder Benzyloxyalkylen, worin die Alkylengruppe 1 bis 6 Kohlenstoffatome hat, Cycloalkyl mit 3 bis 6 Kohlenstoffatomen, Cycloalkylmethyl, 3-Indolyl, Phenylethyl, Methylthioethyl, 3-Indolylalkyl, worin die Alkylgruppe 1 bis 5 Kohlenstoffatome hat, Imidazolyl, Imidazolylalkyl, worin die Alkylgruppe 1 bis 5 Kohlenstoffatome hat, Phenoxymethyl, Phenylthiomethyl, 4-Aminomethylbenzyl, 2-Aminophenethyl, Naphthylethyl, 4-Halophenethyl, 3,4-Dihalophenethyl oder Phenoxyphenethyl sein, oder $R_1$ und $R_2$ bilden zusammen mit —CH einen Lactonring der Formel

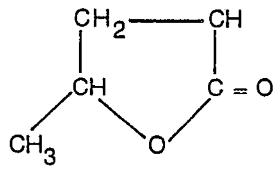 oder 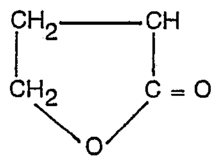

oder einen analogen 6-gliedrigen Ring.

2. Verbindung nach Anspruch 1, wobei X—NH—, —S— oder —O— ist.

3. Verbindung nach Anspruch 1 oder 2, worin $R_1$ und $R_3$ jeweils die allgemeine Formel

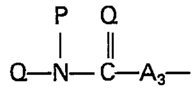

aufweisen und P und Q gleich sein können oder eines davon H sein kann oder beide zusammen mit dem Stickstoffatom, an welches sie gebunden sind, eine Ring bilden können, wobei P und Q aus jedem der Reste der Gruppen aus (a) bis (d) ausgewählt sein können.

4. Verbindung nach Anspruch 1 oder 2, worin $R_1$ die allgemeine Formel 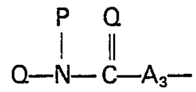 hat, $R_3$ ein Rest der Gruppen aus (i) bis (xxxii) ist und P und Q gleich sein können oder eines davon H sein kann oder beide zusammen mit dem Stickstoffatom, an welches sie gebunden sind, einen Ring bilden können, wobei P und Q aus jedem der Reste der Gruppen aus (a) bis (d) ausgewählt sein können.

5. Verbindung nach einem der Ansprüche 1 bis 4, worin m 0 ist, X N—$R_9$ ist und $R_9$ H ist, $R_4$ und $R_5$ zusammen mit dem Stickstoff- und dem Kohlenstoffatom, an welches sie jeweils gebunden sind, einen Ring bilden, welcher eine der folgenden Strukturen aufweist kann;

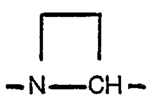 , 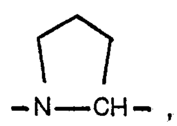 , 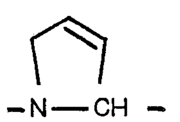 , 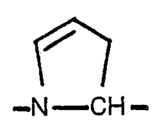 , 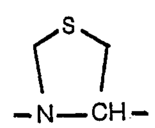 ,

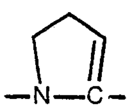 , 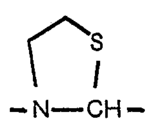 , 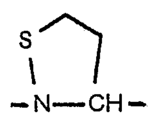 , 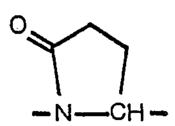 , 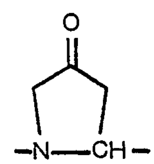 ,

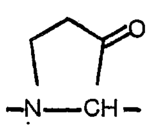 , 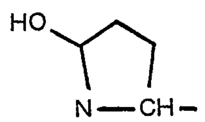 , 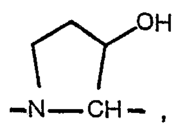 , 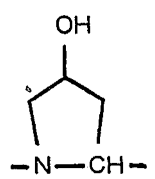 ;

$R_7$ H oder $CH_3$ ist, $R_8$ H oder $CH_3$ ist, und $R_{10}$ H oder $CH_3$ ist.

6. Verbindung nach Anspruch 5, wobei $A_3$ ein Rest der Gruppen aus I bis V ist und P und Q gleich oder verschieden sind und aus H und allen Resten der Gruppen aus (a) bis (d) ausgewählt sind.

7. Verbindung nach Anspruch 5, wobei $A_3$ ein Rest von Gruppen aus I bis V ist, P und Q gleich oder verschieden sind und aus Resten der Gruppen aus (b) bis (d) ausgewählt sind; wenn P und Q zusammen einen Ring bilden, ist der Ring jeder 4- bis 10-gliedrige Ring, welcher ein Stickstoffatom enthält, das nur mit zwei seiner Valenzen an andere Ringglieder gebunden ist.

8. Verbindung nach einem der Ansprüche 1 bis 7, wobei $R_4$ und $R_5$ eine der folgenden Strukturen aufweisen können:

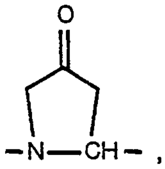
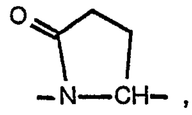
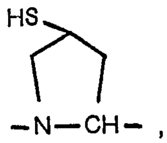
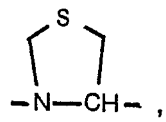
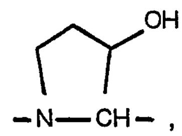

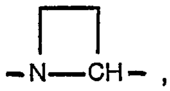
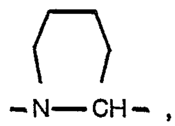
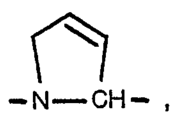
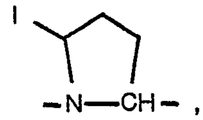
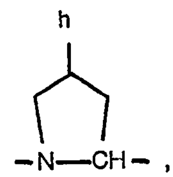

(worin h, Cl, F, Br oder I ist), und $R_6$ —OH ist, oder ein niederer Alkylester oder ein physiologisch annehmbares Salz hiervon.

9. Verbindung nach einem der Ansprüche 1 bis 7, wobei $R_4$ und $R_5$ und $R_6$ zusammen ein substituiertes Prolin bilden, oder ein niederer Alkyl-ester oder physiologisch annehmbare Salze hiervon, worin der Substituent aus der aus Cl, Br, F oder I bestehenden Gruppe gewählt ist.

10. Verbindung nach einem der Ansprüche 1 bis 9, wobei P—N—Q die Struktur

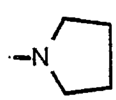

bilden und x = 1, y = 1, X = N—$R_9$, m = O, $R_8$ = H, $R_3$ = $CH_3$ ist, $R_4$ und $R_5$ zusammen die Struktur

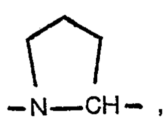

bilden, $R_6$ = OH, $R_7$ = H und $R_2$ = COOH, $R_9$ = H, $R_{10}$ = H ist, oder ein physiologisch annehmbares Salz hiervon.

11. Verbindung nach einem der Ansprüche 1 bis 9, wobei P = H, Q = Phenyl oder Jodo-phenyl, x = 1, y = 1, X = N—$R_9$, m = O, $R_8$ = H, $R_3$ = $CH_3$ ist, $R_4$ und $R_5$ zusammen die Struktur

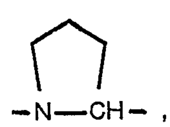

bilden, $R_6$ = OH, $R_7$ = H und $R_2$ = COOH, $R_9$ = H, $R_{10}$ = H ist, oder ein physiologisch annehmbares Salz hiervon.

12. Verbindung nach Anspruch 1, wobei $R_2$ = COOH, COOEt, COOMe, $CONH_2$, COSH, $CH_2SH$ ist, oder wobei P—N—Q aus der aus Anilino, Benzylamino, 2-Aminopyridylamino, 3-Aminopyridylamino, 4-Aminopyridylamino, 3-Indolylamino und Histamino bestehenden Gruppe ausgewählte Strukturen bilden, oder wobei $R_3$ = $CH_3$ ist.

13. Verbindun nach einem der Anspruch 1 bis 12, wobei P von $R_1$ H ist, Q von $R_1$ Aminoalkylen ist, $A_3$ von $R_1$ Alkylen ist und P—N—Q von $R_3$ aus der aus Anilino, Benzylamino, 2-Aminopyridylamino, 3-Aminopyridylamino, 4-Aminopyridylamino, 3-Indolylamino und Histamino bestehenden Gruppe ausgewählte Strukturen bilden, oder wobei P von $R_3$ H ist, Q von $R_3$ Aminoalkylen ist, $A_3$ von $R_3$ Alkylen ist, P—N—Q von $R_1$ aus der aus Aninilo, Benzylamino, 2-Aminopyridylamino, 3-Aminopyridylamino, 4-Aminopyridylamino, 3-Indolylamino und Histamino bestehenden Gruppe ausgewählte Strukturen bilden, oder wobei $R_1$

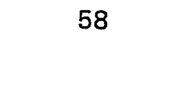

ist, $R_3$ Phenyloxyalkylen, Benzyloxyalkylen, Benzylalkylenoxyalkylen ist, wobei die Alkylengruppe 1 bis 5

Kohlenstoffatome hat, oder wobei $R_1$ Phenoxyalkylen, Benzyloxyalkylen, Benzylalkylenoxyalkylen, wobei die Alkylengruppe 1 bis 5 Kohlenstoffatome hat, und $R_3$

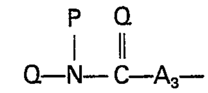

ist.

14. Zusammensetzung aus für die Hemmung des Angiotensin umwandelnden Enzyms in vivo oder für die Senkung des Blutdruckes in vivo eines Säugetieres in hypertonischem Zustand wirksamen Substanzen, welche als wesentlichen aktiven Bestandteil eine therapeutisch wirksame Menge einer Verbindung nach einem der Ansprüche 1 bis 13 enthält.

15. Anwendung einer Verbindung nach einem der Ansprüche 1 bis 13 zur Behandlung von Bluthochdruck oder anomalen Serumwerten von Angiotensin II bei Säugetieren.

**Revendications**

1. Nouveaux composés de formule générale:

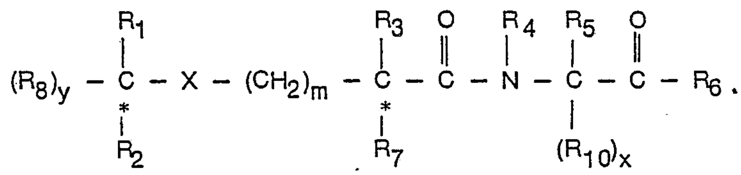

dans laquelle

x et y sont 0 ou 1, X peut être S, O ou N—$R_9$ et $R_9$ peut être —H ou —$CH_3$, $R_{10}$ est H, $CH_3$, F, Cl ou Br;

m est 0 ou 1;

$R_2$ est COOH, $CH_2COOH$, COSH, $CH_2COSH$, $CH_2SH$, $CH_2CH_2SH$, un sel non toxique physiologiquement acceptable de l'un quelconque d'entre eux; COOY, $CH_2COOY$, COSY, $CH_2SY$ ou $CH_2CH_2SY$ où Y est phényle, benzyle ou un radical alkyle ayant de à 5 atomes de carbone; ou

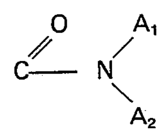

où chacun de $A_1$ et $A_2$ peut être H, phényle, benzyle ou un radical alkyle ayant 1 à 5 atomes de carbone;

$R_4$ et $R_5$ forment ensemble un cycle avec les atomes d'azote et de carbone auxquels ils sont respectivement fixés, lequel cycle est une des structures:

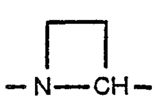 , 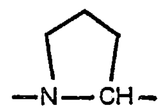 , 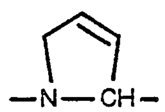 , 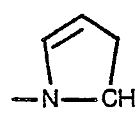 , 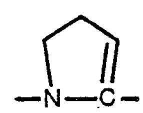 ,

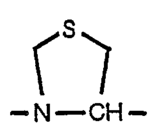 , 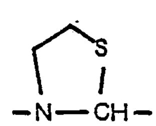 , 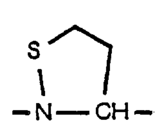 , 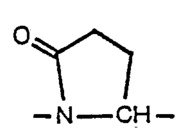 , 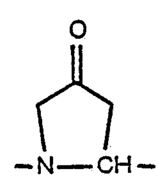 ,

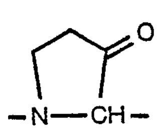 , 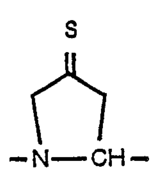 ; 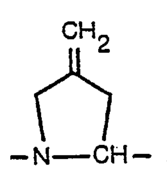 , 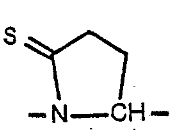 , 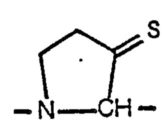 ,

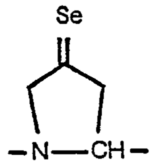, 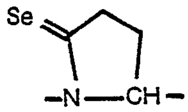, 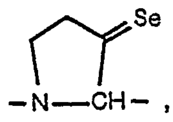, 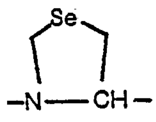, 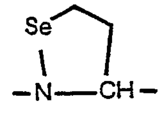,

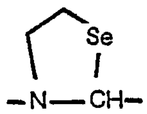, 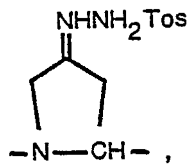, 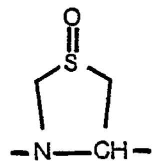, 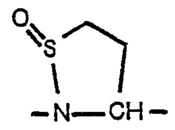, 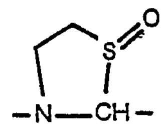,

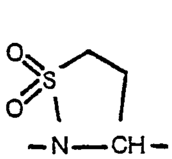, 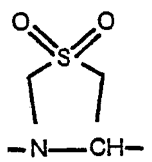, 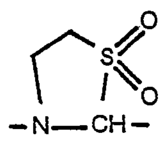, 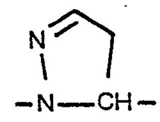, 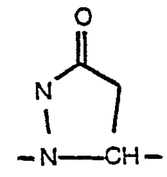,

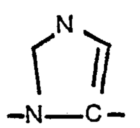, 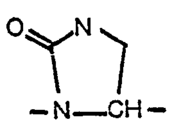, 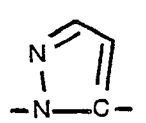, 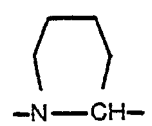, 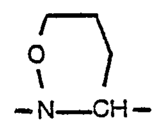,

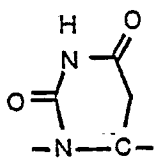, 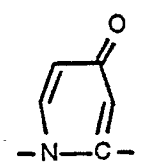, 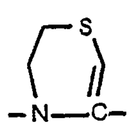, 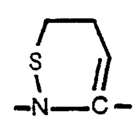, ou 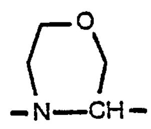,

étant entendu que l'une quelconque de ces structures peut être monosubstituée par

$-OH$, $-OCH_3$, F, 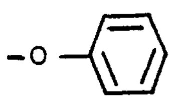, 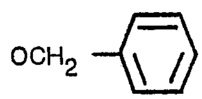,

Cl, Br, I, phenyle, hydroxyphenyle, $-SH$, $-SCH_3$,

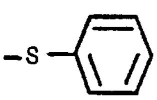, 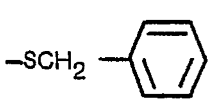, $-NHCH_3$, $-CH_2NH_2$, $-CH_3$, $-CH_2OH$, propyle,

guanidino, nitroguanidino ou thioguanidino et que l'un quelconque des cycles à 5 ou 6 chaînons peut être disubstitué par —OH, F, Cl, Br, I, $OCH_3$ ou une combinaison quelconque de deux substituants de ce groupe;

$R_6$ est —OM ou —SM, où M peut être H, un radical alkyle ayant de 1 à 3 atomes de carbone ou tout autre fragment ester hydrolysable en —OH dans les conditions existant in vivo chez les mammifères, ou un anion à liaison ionique d'un sel non toxique physiologiquement acceptable;

$R_7$ est H—, $CH_3$—, halogénométhyle, hydroxymethyle, aminométhyle, ou mercaptométhyle;

$R_8$ est H—, $CH_3$—, amino, halogénométhyle, hydroxyméthyle, aminométhyle, dihalogénéométhyle, trihalogénométhyle, mercaptométhyle, méthoxyméthyle, méthylthiométhyle, méthoxycarbonylméthyle, cyanométhyle, benzyle, acétoxyméthyle, $CH_2{=}CH{-}CH_2$—, isobutyle, mercaptoalkyle ayant 2 ou 3 atomes de carbone, hydroxyalkyle ayant 2 ou 3 atomes de carbone, acétylthioéthyle, benzamido, acétamido, phtaloylaminoalkylène dont le radical alkylène a de 1 à 4 atomes de carbone, α-alcoxycarbonylisoalkylène dont le radical alkyle contient de 1 à 5 atomes de carbone et le radical isoalkylène contient de 3 à 5 atomes de carbone, benzoylamine, alcanoylamine ayant de 1 á 5 atomes de carbone, alkylamide ayant de 1 à 5 atomes de carbone, phénylamine, alkylamine ayant de 1 à 5 atomes de carbone ou éthyle;

A. $R_1$ et $R_3$ peuvent chacun répondre à la formule générale:

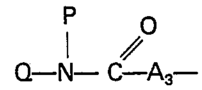

dans laquelle $A_3$ est:

I. alkylène de 1 à 6 atomes de carbone, alkyle à chaîne ramifiée de 1 à 6 atomes de carbone, cycloalkylalkylène, alkylcycloalkylalkylène ou alkylcycloalkylène;

II. aralkylène dont le radical alkyle a de 1 à 6 atomes de carbone ou alkylaryle;

III. phényle;

IV. alkylaralkylène dont les radicaux alkyle peuvent être semblables ou différents et sont longs de 1 à 6 atomes de carbone;

V. alkylène substitué, alkyle à chaîne ramifié substitué, cycloalkylalkylène substitué, alkylcycloalkylalkylène substitué, alkylcycloalkylène substitué, alkylaryle substitué, aralkylène substitué, phényle substitué ou alkylaralkylène substitué dont le ou les substituants peuvent être semblables ou différents, peuvent être incorporés à la chaîne alkylène ou être latéraux et sont choisis parmi amino, halogéno, hydroxy, mercapto, $NO_2$, carboxy, $CONH_2$, alkyle inférieur, halogénométhyle, hydroxyméthyle, aminométhyle, dihalogénométhyle, trihalogénométhyle, cyano, mercaptométhyle, méthoxyméthyle, methylthiométhyle, méthoxycarbonylméthyle, cyanométhyle, benzyle, acétoxyméthyle, $CH_2{=}CH{-}CH_2{-}$, isobutyle, mercaptoalkyle ayant 2 ou 3 atomes de carbone, hydroxyalkyle ayant 2 ou 3 atomes de carbone, acétylthioéthyle, benzamido, acétamido, phtaloylaminoalkylène dont le radical alkylène a de 1 à 4 atomes de carbone, α-alcoxycarbonylisoalkylène dont le radical alkyle contient de 1 à 5 atomes de carbone et le radical isoalkylène contient de 3 à 5 atomes de carbone, benzoylamino, alcanoylamino ayant de 1 à 5 atomes de carbone, alkylamide ayant de 1 à 5 atomes de carbone, phénylamine, alkylamine ayant de 1 à 5 atomes de carbone, alcoxy inférieur, aryloxy, alkylamino inférieur, dialkylamino inférieur, acylamino, arylamino, guanidino, imidazolyle, indolyle, alkylthio inférieur, arylthio, carboxamido et carbalcoxy inférieur;

VI. alkylènethio ou alkylénethioalkylène ayant de 1 à 6 atomes de carbone, alkylthioalkylène ayant de 1 à 6 atomes de carbone;

VII. alkylèneoxy ou alkylèneoxyalkylène dont les radicaux alkyle peuvent être semblables ou différents et ont 1 à 6 atomes de carbone;

VIII. alcoxyphényle ou alcoxybenzyle dont le radical alcoxy a de 1 à 3 atomes de carbone, phénoxyphényle, phénoxybenzyle, benzyloxybenzyle ou benzyloxyphényle ou un analogue de type thioéther de l'un quelconque d'entre eux;

IX. 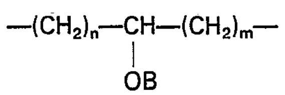

où n = 0—4, m = 0—4 et B = H ou un radical alkyle ayant 1 à 5 atomes de carbone; ou un analogue de type —SB;

X. 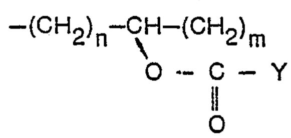 ou 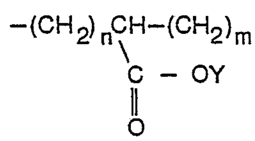

ou 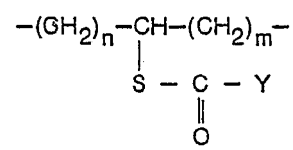 ou 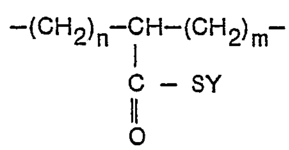

où n et m ont les mêmes significations que ci-dessus, Y est phényle, benzyle ou un radical alkyle ayant 1 à 5 atomes de carbone;

XI. 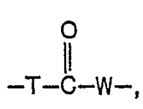 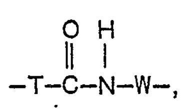 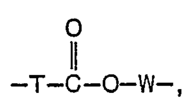 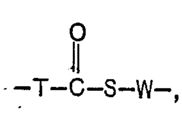 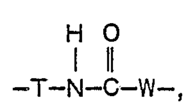 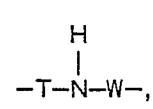

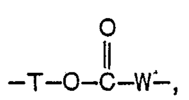 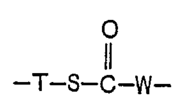

où T et W peuvent être semblables ou différents et sont alkylène, aryle, benzyle ou cycloalkyle; et P et Q

peuvent être semblables, ou l'un d'entre eux peut être H ou bien ils peuvent être combines pour former un cycle avec l'azote auquel ils sont fixés;

l'un ou l'autre de P et Q ou les deux peuvent être choisis, parmi l'un quelconque des suivants:

(a) radicaux alkyle en $C_1$—$C_6$ à chaîne droite ou ramifiée ou radicaux alcényle en $C_1$—$C_6$ à chaîne droite ou ramifiée dont l'un quelconque peut être substitué par l'un quelconque d'halogéno, hydroxy, alcoxy, aryloxy, amino, alkylamino, dialkylamino, alkylacylamino, arylamino, guanidino, thioguanidino, nitroguanidino, hydrazino, uréido, nitro, mercaptocarbonyle, hydroxyamino, histidinyle, cyano, imidazolyle, indolyle, mercapto, alkylthio, arylthio, carboxamido ou carbalcoxy, où les radicaux alkyle contiennent de 1 à 6 atomes de carbone;

(b) cycloalkyle ou cycloakylalkylène dont le cycloalkyle a de 4 à 12 atomes de carbone et l'alkylène a de 1 à 5 atomes de carbone, pouvant être substitués par l'un quelconque des radicaux —OH, —SH, halogéno,

$$COOH,\ COSH,\ CONH_2,\ NO_2NH_2,\ NO_2,\ CH_3,\ —OCH_3,\ —\overset{\overset{O}{\|}}{C}—OCH_3,$$

hydrazino, uréido, —$SCH_3$, hydroxyamino, cyano, guanidino, thioguanidino ou nitroguanidino;

(c) radicaux aralkyle ou alkaryle pouvant être substitués sur le cycle par un ou plusieurs des radicaux suivants: SH, halogéno, $CH_2COOH$, $CH_2CONH_2$, $CH_2CONH$-alkyle, COSH, COOH, $CONH_2$, CONH-alkyle, $CH_2COSH$, $CH_2SH$, $CH_2OH$, OH, $NO_2$, amino, alkyle, alcoxy, aralcoxy, alkylthio et aralkylthio, où les radicaux alkyle contiennent de 1 à 6 atomes de carbone et peuvent sinon aussi être substitués sur la chaîne par

$$—CH_3,\ —OH,\ —OCH_3,\ \text{halogéno},\ —SCH_3,\ \overset{\overset{O}{\|}}{C}—CH_3,\quad —NH_2,\ NO_2,\ —CN,\ —SH,\ —NHNH_2,\quad NH—\overset{\overset{O}{\|}}{C}—NH_2,$$

—NHOH, ou un dérivé de type thio ou nitro correspondant, —COOH ou COSH;

(d) un radical aryle, hétérocyclique ou adamantanyle pouvant être substitué sur le cycle par au moins un radical choisi parmi halogéno, —OH, —O-alkyle, —O-aryle, $NH_2$, NH-alkyle, N-$(alkyle)_2$,

$$\text{alkyle}—\overset{\overset{O}{\|}}{C}—NH_2,$$

aryl-$NH_2$, guanidino, thioguanidino, nitroguanidino, hydrazino, uréido, nitro, mercaptocarbonyle, hydroxyamino, cyano-imidazolyle, indanyle, histidinyle,

$$—SH,\quad —S—\text{alkyle},\quad S—\text{aryle},\quad —\overset{\overset{O}{\|}}{C}—NH_2,\quad —\overset{\overset{O}{\|}}{C}—O—\text{alkyle},\quad —\overset{\overset{O}{\|}}{C}—\text{alkyle},\quad —\overset{\overset{O}{\|}}{C}—O—\text{aryle},$$

$$—\overset{\overset{O}{\|}}{C}—\text{aryle},\quad —\overset{\overset{O}{\|}}{C}—SH,\quad —\overset{\overset{O}{\|}}{C}—S—\text{alkyle},\quad —\overset{\overset{O}{\|}}{C}—S—\text{aryle et } —NO_2,$$

lorsque P et Q sont unis à N pour former un cycle, le cycle peut être un hétérocycle à 4—10 chaînons quelconque qui contient un azote avec deux seulement de ses valences fixées aux autres éléments du cycle.

B. Ou bien $R_1$ peut être $\quad Q—\overset{\overset{P}{|}}{N}—\overset{\overset{O}{\|}}{C}—A_3—\quad$ et

$R_3$ peut être

(i) alkylène mono-N-substitué ayant de 2 à 4 atomes de carbone dont le substituant sur N est benzoyle, Boc, CbO, Tos, formyle ou acétyle;

(ii) hydroxyphényle ou hydroxyphényl-alkylène-($C_{1-6}$) ou un analogue de type thiol de l'un ou l'autre;

(iii) mercaptoalkylène ayant de 1 à 6 atomes de carbone;

(iv) phénylalkylène dont le radical alkylène a de 1 à 6 atomes de carbone;

(v) phénylthioalkylène ou benzylthioalkylène dont le radical alkylène a de 1 à 6 atomes de carbone;

(vi) alkylthioalkylène dont les radicaux alkyle et alkylène ont de 1 à 3 atomes de carbone;

(vii) alcoxyphényle ou alcoxybenzyle dont le radical alcoxy a de 1 à 3 atomes de carbone, phénoxy-

phényle, phénoxybenzyle, benzyloxybenzyle ou benzyloxyphényle ou un analogue de type thioéther de l'un quelconque d'entre eux;

(viii) 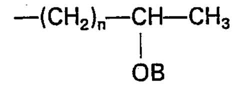

où n = 0—4 et B = H ou un radical alkyle ayant de 1 à 6 atomes de carbone, ou un analogue de type —SB;

(ix) $(CH_2)_pCOOZ$ ou $(CH_2)_pCOSZ$ où p = 0—3 et Z est H, phényle, benzyle, un radical alkyle ayant de 1 à 5 atomes de carbone, ou un anion d'un sel physiologiquement acceptable;

(x) 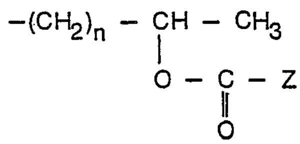 ou 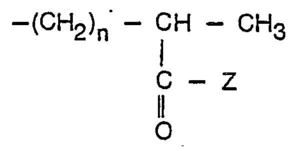

ou 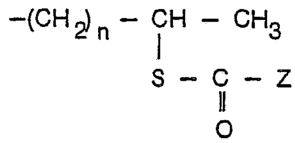 ou 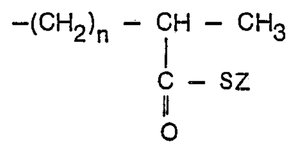

où n est 0 à 4 et chaque Z a la même signification que ci-dessus;

(xi) 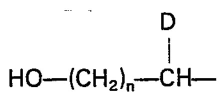 ou 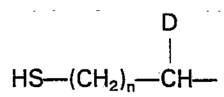

où n = 0—4, D est phényle, thiényle ou un radical alkyle ayant de 1 à 3 atomes de carbone;

(xii) $HO—(CH_2)_n—C(CH_3)_2—$, $HS—(CH_2)_n—C(CH_3)_2—$, p-hydroxyphenyl $—(CH_2)_n—C(CH_3)_2$ ou p-mercaptophényl $—(CH_2)_n—C(CH_3)_2—$ ou n a la même signification que ci-dessus;

(xiii) p-mercaptophényl $—(CH_2)_n—CH_2—$ ou p-hydroxyphényl-$(CH_2)_n—CH_2—$ où le cycle phényle a un ou deux substituants nitro ou amino et n a la même signification que ci-dessus;

(xiv) 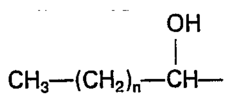 ou 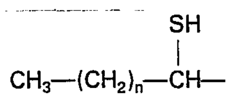

où n a la même signification que ci-dessus;

(xv) $NH_2$-alkylène ou $NO_2$-alkylène contenant un substituant hydroxy ou mercapto et ayant de 1 à 6 atomes de carbone;

(xvi) hydroxy- ou mercapto-phénoxybenzyle;

(xvii) 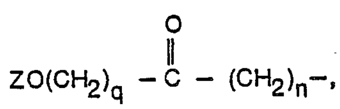, 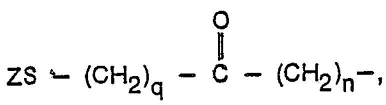, 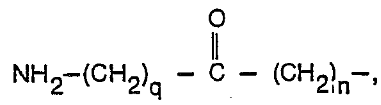,

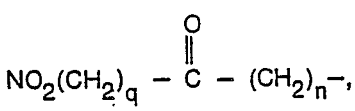, 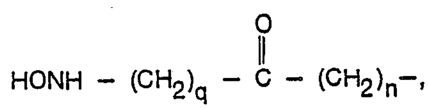,

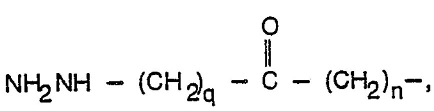, 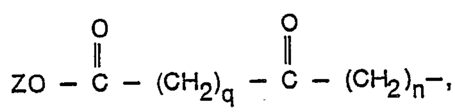,

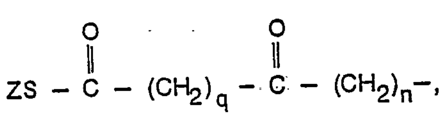, ou 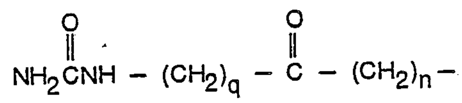,

où q = 1 − 5 et n a une valeur de 0 a 4 et Z a la même signification que ci-dessus;

$$\text{(xviii)}\ ZO-(CH_2)_q-\overset{OH}{\overset{|}{C}}H-(CH_2)_n-,\quad ZS-(CH_2)_q-\overset{OH}{\overset{|}{C}}H-(CH_2)_n-,$$

$$NH_2-(CH_2)_q-\overset{O}{\overset{\|}{C}}-(CH_2)_n-,\quad NO_2-(CH_2)_q-\overset{OH}{\overset{|}{C}}H-(CH_2)_n-,$$

$$NH_2-\overset{O}{\overset{\|}{C}}-NH-(CH_2)_q-\overset{OH}{\overset{|}{C}}H-(CH_2)_n-,\quad ZO-(CH_2)_q-\overset{OH}{\overset{|}{C}}H-(CH_2)_n-,$$

$$ZS-(CH_2)_q-\overset{OH}{\overset{|}{C}}H-(CH_2)_n-,\quad HONH-(CH_2)_q-\overset{OH}{\overset{|}{C}}H-(CH_2)_n-,\ \text{ou}$$

$$NH_2NH-(CH_2)_q-\overset{OH}{\overset{|}{C}}H-(CH_2)_n-,$$

où q et n ont tous la même signification que ci-dessus;

$$\text{(xix)}\ G-NH-(CH_2)_q-\overset{O}{\overset{\|}{C}}-(CH_2)_n-,\quad G-NH-(CH_2)_q\overset{OH}{\overset{|}{C}}H-(CH_2)_n-,$$

$$G-(CH_2)_q-\overset{O}{\overset{\|}{C}}-(CH_2)_n-,\quad G-(CH_2)_q-\overset{OH}{\overset{|}{C}}H-(CH_2)_n-,$$

$$NH_2-\overset{O}{\overset{\|}{C}}-(CH_2)_q-\overset{O}{\overset{\|}{C}}-(CH_2)_n-,\ \text{ou}\ NH_2-\overset{O}{\overset{\|}{C}}-(CH_2)_q-\overset{OH}{\overset{|}{C}}H-(CH_2)_n-,$$

où G est un radical alkacyle ou alkacyloxy de 1 à 6 atomes de carbone, un radical benzoyle ou benzoyloxy, ou un radical phénylalkacyle ou phénylalkacyloxy où le radical alkacyle ou alkacyloxy contient de 2 à 6 atomes de carbone et q et n ont la même signification que celle indiquée ci-dessus;

$$\text{(xx)}\quad K-(CH_2)_n-\overset{O}{\overset{\|}{C}}-(CH_2)_n-\ \text{ou}\ K-(CH_2)_n\overset{OH}{\overset{|}{C}}H-(CH_2)_n-$$

où n a la signification indiquèe ci-dessus et K est choisi parmi carboxyphényle, aminophényle, nitrophényle, halogénophényle, hydroxyphényle, alkylthiophényle, alkylphényle, mercaptophényle, cyanophényle, mercaptocarbonylphényle, alkylcarbonylphényle, alkylcarbonyloxyphényle, hydrazinophényle,uréidophényle, alkylcarbonylaminophényle, alkylcarbonylthiophényle, alcoxyphényle et hydroxyaminophényle, où tous les radicaux alkyle contiennent de 1 à 6 atomes de carbone;

$$\text{(xxi)}\quad L-(CH_2)_n-\overset{O}{\overset{\|}{C}}-(CH_2)_n-\ \text{ou}\ L-(CH_2)_n\overset{OH}{\overset{|}{C}}H-(CH_2)_n-,$$

où n a la signification indiquée ci-dessus et L est choisi parmi les radicaux cycloalkyle ayant de 3 à 7 atomes de carbone, pouvant être non substitués ou substitués par jusqu'à deux radicaux choisis parmi carboxy, amino, nitro, halogéno, hydroxy, mercapto, mercaptocarbonyle, hydroxyamino, alkyle, alkylcarbonyle, alkylcarbonyloxy, alkylthio, alkylcarbonylamino, alkylcarbonylthio, cyanohydrazino, uréido et alcoxy, où tous les radicaux alkyle contiennent de 1 à 6 atomes de carbone;

(xxii) guanidinoalkyléne, thioguanidinoalkylène ou nitroguanidinoalkylène ouì les radicaux alkylène contiennent de 1 à 6 atomes de carbone;

(xxiii) les radicaux aryle substitués sur le cycle dont les substituants du cycle peuvent être semblables ou différents et peuvent comprendre jusqu'à cinq par cycle des suivants: $—NH_2$, —OZ, —SZ, halogéno, —CN, $—NO_2$, —COOZ, —COSZ, $CONH_2$, $—NHNH_2$, alkyle, alkylcarbonyle, alkylcarbonyloxy, alkylcarbonylamino, halogénoalkyle, dihalogénoalkyle, trihalogénométhyle, hydroxyamino, alkylcarbonylthio, phénoxy et benzyloxy où les radicaux alkyle contiennent de 1 à 6 atomes de carbone et Z a la même signification que ci-dessus;

(xxiv) amidoalkylène ou alkylcarbonylaminoalkylène où les radicaux alkyle et alkylène contiennent de 1 à 6 atomes de carbone;

(xxv) hydroxyaminoalkylène de 1 à 6 atomes de carbone;

(xxvi) les radicaux vinyle et vinyle substitué dont les substituants peuvent être des radicaux alkyle, aryle, cycloalkyle ou hétérocycliques;

(xxvii) des radicaux hétérocycliques non substitués choisis parmi phénothiazinyle, pyrrolidinyle, pyrrolyle, quinolinyle, imidazolyle, pyridyle, thyminyle, benzothiazinyle, indolyle, thiényle, purinyle, pipéridinyle, morpholinyle, azaindolyle, pyrazinyle, pyrimidyle, pipéronyle, pipérazinyle, furanyle, thiazolyle et thiazolidinyle, cytosinyle;

(xxviii) des radicaux alkylène ou alcényle ayant de 1 à 6 atomes de carbone substitués par un des hétérocycles de (xxvii) ci-dessus;

(xxix) des radicaux de (xxvii) ou (xxviii) ci-dessus contenant sur l'hétérocycle jusqu'à quatre substituants choisis parmi —OZ, —SZ, —COOZ, $—NO_2$, $—NH_2$, —COSZ, halogéno, halogénoalkyle, dihalogénoalkyle, trihalogénométhyle, cyano, $CONH_2$, alkyle, alkylcarbonyle, alkylcarbonyloxy, alkylcarbonylthio, phénoxy, benzyloxy, 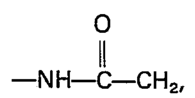

$—NHNH_2$ et HONH—, où Z a la même signification que ci-dessus;

(xxx) des radicaux de (xxvii), (xxviii) ou (xxix) fixés à une valence d'un —O— ou —S— éthérique;

(xxxi) mono-, di- ou tri-alkyle-, alcényl- ou phényl-silyle ou -sélényle où les radicaux alkyle ou alcényle contiennent de 1 à 6 atomes de carbone;

(xxxii) l'un quelconque de H, alkyle à chaîne droite ou ramifiée ayant de 1 à 5 atomes de carbone, phényle, —OH, alcoxy de 1 à 6 atomes de carbone, benzyloxy, benzyloxyalkylène ou phénoxyalkylène ou l'alkylène a de 1 à 5 atomes de carbone, alcoxyalkylène ayant de 1 à 5 atomes de carbone dans les radicaux alcoxy et alkylène, aminoalkylène ayant de 1 à 6 atomes de carbone, alcényle ayant de 1 à 6 atomes de carbone, benzyle, hydroxyalkyle ayant de 1 à 6 atomes de carbone, mercaptoalkyle ayant de 1 à 6 atomes de carbone, histidinyle, halogénoalkyle ayant de 1 à 6 atomes de carbone, 4-amino-méthylbenzyle, acétamido-alkyle ayant de 1 à 5 atomes de carbone, benzylthiométhylène ou diméthylamino-alkyle ayant de 1 à 5 atomes de carbone.

C. Ou bien $R_3$ peut être 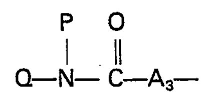 et

$R_1$ peut être l'un quelconque des radicaux (i) à (xxxii) ci-dessus ou l'un quelconque de H, alkyle à chaîne droite ou ramifiée en $C_1—C_8$, phényle, benzyle, aminoalkylène non substitué ayant de 2 à 6 atomes de carbone, hydroxyalkyléne ayant de 1 à 6 atomes de carbone, hydroxyphényle, phénoxyalkylène ou benzyloxyalkylène dont le radical alkylène a de 1 à 6 atomes de carbone, cycloalkyle ayant de 3 à 6 atomes de carbone, cycloalkylméthyle, 3-indolyle, phényléthyle, méthylthioéthyle, 3-indolylalkyle dont le radical alkyle contient de 1 à 5 atomes de carbone, imidazolyle, imidazolylalkyle dont le radical alkyle contient de 1 à 5 atomes de carbone, phénoxyméthyle, phénylthiométhyle, 4-aminométhylebenzyle, 2-aminophénéthyle, naphthyléthyle, 4-halogénophénéthyle, 3,4-dihalogénophénéthyle ou phénoxyphénéthyle, ou $R_1$ et $R_2$ peuvent former ensemble avec —CH un cycle lactone de formule:

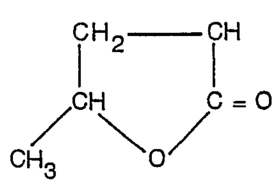

ou

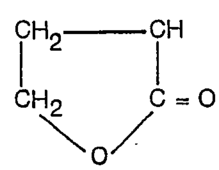

ou un cycle à six chaînons analogue.

2. Un composé selon la revendication 1 dans lequel X est —NH—, —S— ou —O—.

3. Un composé selon la revendication 1 ou 2, dans lequel $R_1$ et $R_3$ répondent chacun à la formule générale:

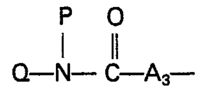 ;

et P et Q peuvent être semblables, ou l'un d'entre eux peut être H ou bien ils peuvent être combinés pour former un cycle avec l'atome d'azote auquel ils sont fixés, dans lequel P et Q peuvent être choisis parmi l'un quelconque des radicaux des groupes (a) à (d).

4. Un composé selon la revendication 1 ou 2 dans lequel $R_1$ répond à la formule générale:

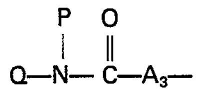 ;

$R_3$ est un radical des groupes (i) à (xxxii);

et P et Q peuvent être semblables, ou l'un d'entre eux peut être H ou bien ils peuvent être combinés pour former un cycle avec l'atome d'azote auquel ils sont fixés, dans lequel P et Q peuvent être choisis parmi l'un quelconque des radicaux des groupes (a) à (d).

5. Un composé selon l'une quelconque des revendications 1 à 4, dans lequel:

m est zéro;

X est N—$R_9$ et $R_9$ est H;

$R_4$ et $R_5$ forment ensemble un cycle avec les atomes d'azote et de carbone auxquels ils sont respectivement fixés, lequel cycle est l'une des structures:

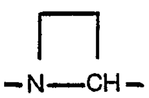 , 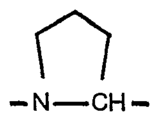 , 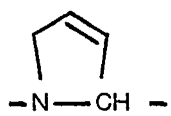 , 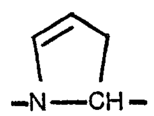 , 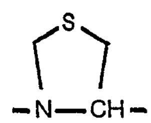 ,

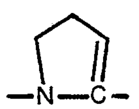 , 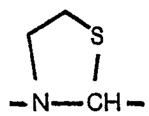 , 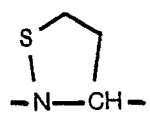 , 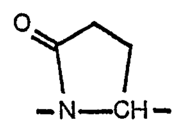 , 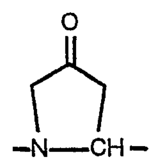 ,

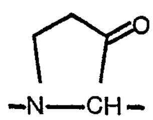 , 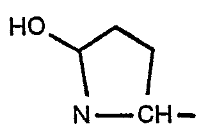 , 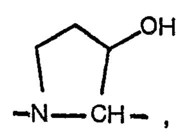 , 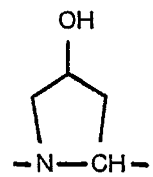 ;

$R_7$ est H— ou $CH_3$—;

$R_8$ est H— ou $CH_3$—; et

$R_{10}$ est H— ou $CH_3$—.

6. Un composé selon la revendication 5, dans lequel:

$A_3$ est un radical des groupes I à V; et

P et Q sont semblables ou différents et sont choisis parmi H et un radical quelconque des groupes (a) à (d).

7. Un composé selon la revendication 5, dans lequel:

$A_3$ est un radical des groupes I à V;

P et Q sont semblables ou différents et sont choisis parmi les radicaux des groupes (b) à (d), lorsque P et Q sont unis pour former un cycle, le cycle est l'un quelconque des hétérocycles de 4 à 10 chaînons qui contiennent un atome d'azote avec seulement deux de ses valences fixées aux autres éléments du cycle.

8. Un composé selon l'une quelconque des revendications 1 à 7, dans lequel $R_4$ et $R_5$ forment une des structures:

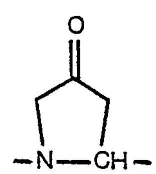 , 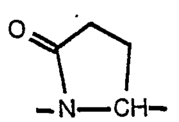 , 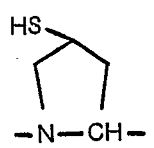 , 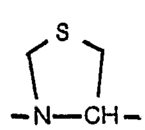 , 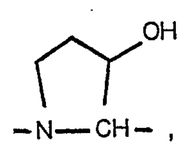 ,

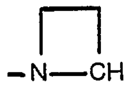, 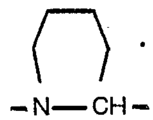, 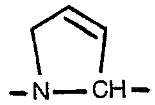, 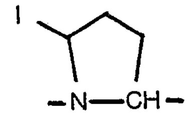, 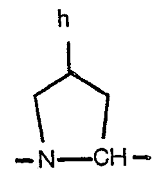,

(dans lesquelles h est Cl, F, Br ou I), et $R_6$ est —OH, ou un ester alkylique inférieur ou un sel physiologiquement acceptable de celui-ci.

9. Un composé selon l'une quelconque des revendications 1 à 7, dans lequel $R_4$ et $R_5$ et $R_6$ forment ensemble une proline substituée, ou un ester alkylique inférieur de celle-ci et les sels physiologiquement acceptables correspondants, où le substituant est choisi dans le groupe constitué par Cl, Br, F ou I.

10. Un composé selon l'une quelconque des revendications 1 à 9, dans lequel P—N—Q forme la structure:

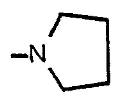

et x = 1, y = 1, X = N—$R_9$, m = O, $R_8$ = H, $R_3$ = $CH_3$, $R_4$ et $R_5$ forment ensemble la structure:

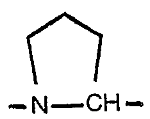,

$R_6$ = OH, $R_7$ = H et $R_2$ = COOH, $R_9$ = H, $R_{10}$ = H ou les sels physiologiquement acceptables de celui-ci.

11. Un composé selon l'une quelconque des revendications 1 à 9, dans lequel P = H, Q = phényle ou iodophényle, x = 1, y = 1, X = $NR_9$, m = O, $R_8$ = H, $R_3$ = $CH_3$, $R_4$ et $R_5$ forment ensemble la atructure:

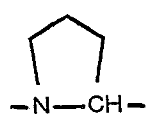,

$R_6$ = OH, $R_7$ = H et $R_2$ = COOH, $R_9$ = H, $R_{10}$ = H ou les sels physiologiquement acceptables de celui-ci.

12. Un composé selon la revendication 1, dans lequel $R_2$ est COOH, COOEt, COOMe, $CONH_2$, COSH, $CH_2SH$; ou dans lequel P—N—Q forme des structures choisies parmi anilino, benzylamino, 2-aminopyridylamino, 3-aminopyridylamino, 4-aminopyridylamino, 3-indolylamino et histamino; ou dans lequel $R_3$ est $CH_3$.

13. Un composé selon l'une quelconque des revendications 1 à 12 dans lequel:

P de $R_1$ est H;

Q de $R_1$ est aminoalkylène;

$A_3$ de $R_1$ est alkylène; et

P—N—Q de $R_3$ forme des structures choisies parmi anilino, benzylamino, 2-aminopyridylamino, 3-aminopyridylamino, 4-aminopyridylamino, 3-indolylamino, et histamino; ou dans lequel:

P de $R_3$ est H,

Q de $R_3$ est aminoalkylène;

$A_3$ de $R_3$ est alkylène;

P—N—Q de $R_1$ forme des structures choisies parmi anilino, benzylamino, 2-aminopyridylamino, 3-aminopyridylamino, 4-aminopyridylamino, 3-indolylamino et histamino; ou dans lequel:

$R_1$ est 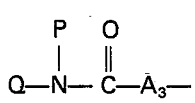

$R_3$ est phényloxyalkylène, benzyloxyalkylène, benzylalkylèneoxyalkylène, où le radical alkylène a de 1 à 5 atomes de carbone; ou dans lequel:

$R_1$ est phényloxyalkylène, benzyloxyalkylène, benzylalkylèneoxyalkylène, où 'le radical alkylène a de 1 à 5 atomes de carbone;

$R_3$ est 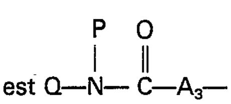

14. Une composition efficace pour inhiber in vivo l'enzyme de conversion de l'angiotensine ou pour

abaisser la pression sanguine in vivo d'un mammifère à l'état hypertendu, qui contient comme ingrédient actif essentiel une quantité thérapeutiquement efficace d'un composé de l'une quelconque des revendications 1 à 13.

15. Un composé suivant l'une quelconque des revendications 1 à 13 pour utilisation dans le traitement de l'hypertension ou des concentrations sériques anormales de l'angiotensine II chez les mammifères.